(19) Europäisches Patentamt
European Patent Office
Office européen des brevets

(11) **EP 4 782 553 A2**

## (12) EUROPEAN PATENT APPLICATION

(43) Date of publication:
**29.07.2026 Bulletin 2026/31**

(21) Application number: **26168777.6**

(22) Date of filing: **05.10.2022**

(51) International Patent Classification (IPC):
***C12Q 1/6883*** (2018.01)

(52) Cooperative Patent Classification (CPC):
**C12Q 1/6883;** C12Q 2600/154

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.10.2021 US 202163252732 P**
**20.12.2021 US 202163291536 P**

(62) Document number(s) of the earlier application(s) in accordance with Art. 76 EPC:
**22797601.6 / 4 413 162**

(71) Applicant: **Inherent Biosciences, Inc.**
**Salt Lake City, Utah 84101 (US)**

(72) Inventors:
• **Miller, Ryan**
**Millcreek, Utah 84106 (US)**
• **Brogaard, Kristin**
**Park City, Utah 84098 (US)**
• **Olson, Andrew**
**Salt Lake City, Utah 84103 (US)**

(74) Representative: **Brand Murray Fuller LLP**
**50 Eastcastle Street**
**London W1W 8EA (GB)**

Remarks:
•This application was filed on 27-03-2026 as a divisional application to the application mentioned under INID code 62.
•Claims filed after the date of filing of the application/ after the date of receipt of the divisional application (Rule 68(4) EPC).

## (54) METHODS AND SYSTEMS FOR PREDICTING SPERM QUALITY

(57) Disclosed herein are methods and systems for predicting sperm quality by determining methylation in different gene promoters. Also disclosed herein, are methods and systems for determining methylation variability of an individual promoter. Additionally, methods of treatment for infertility or diminished fertility are described herein. Also described herein are kits, such as kits comprising arrays.

Number of Dysregulated Promoters across the 1344 Samples

FIG. 5A

EP 4 782 553 A2

**Description**

**CROSS-REFERENCE**

This application claims the benefit of U.S. Provisional Application No. 63/252,732, filed October 06, 2021, and U.S. Provisional Application No. 63/291,536, filed December 20, 2021, the disclosures of which are incorporated herein by reference in their entirety.

**STATEMENT AS TO FEDERALLY SPONSORED RESEARCH**

This invention was made with government support under grant number 2034014 awarded by the National Science Foundation. The government has certain rights in the invention.

**SUMMARY OF THE DISCLOSURE**

Disclosed herein are methods comprising: a) obtaining a biological sample from a male subject, wherein the biological sample comprises seminal fluid; b) extracting DNA from a sperm in the biological sample, extracting cell free DNA from the biological sample, or both; c) detecting in an *in vitro* analytical assay, methylation present in at least 5 regions of an individual promoter comprised in the extracted DNA, the extracted cell free DNA, or both from the biological sample, wherein the promoter is selected from Table 1; d) determining independently a standard deviation for methylation in each of the at least 5 regions of the individual promoter; e) calculating an average standard deviation for methylation of the individual promoter from the standard deviation of methylation in each of the at least 5 regions of the individual promoter; and f) determining if the average standard deviation for methylation of the individual promoter is greater than or equal to three standard deviations from a reference standard deviation of methylation for the individual promoter. In some embodiments, the determining that the average standard deviation of the individual promoter is greater than or equal to three standard deviations can be independently determined in 22 or more different promoters. In some embodiments, the method can be a method of detecting diminished fertility of a male subject. In some embodiments, the determining that the average standard deviation of the individual promoter is greater than or equal to three standard deviations can be independently determined in less than 22 different promoters, and the average standard deviations for methylation of the individual promoters are determined in 1233 different promoters. In some embodiments, the method can be a method of detecting fertility of a male subject. In some embodiments, the calculating the average standard deviation for methylation of the individual promoter from the standard deviation of methylation in each of the at least 5 regions of the individual promoter is calculated by: $\sigma = \sqrt{\dfrac{\sum |x_1 - \mu|^2}{N}}$ , where $\sigma$ = the average standard deviation for methylation, x1 = an m-value of a given methylation array probe in the individual promoter, N = a number corresponding to the number of regions of the individual promoter, and $\mu$ = a mean of probe m-values in the individual promoter. In some embodiments, the reference standard deviation of methylation for the promoter can be derived from a fertile subject. In some embodiments, the method further comprises determining: a) a morphological characteristic, b) a motility characteristic, c) a concentration, or d) any combination thereof of the sperm. In some embodiments, the detecting can employ a computer processor. In some embodiments, the determining independently the standard deviation for methylation in each of the at least 5 regions of the individual promoter can employ a computer processor. In some embodiments, the calculating the average standard deviation for methylation of the individual promoter from the standard deviation of methylation in each of the at least 5 regions of the individual promoter can employ a computer processor. In some embodiments, the determining if the average standard deviation for methylation of the individual promoter is greater than or equal to three standard deviations from a reference standard deviation of methylation for the promoter can employ a computer processor. In some embodiments, the method can further comprise performing a treatment on the subject, wherein the treatment comprises *in vitro* fertilization (IVF) or intrauterine insemination (IUI). In some embodiments, the detecting can comprise a sodium bisulfite conversion, a sequencing, a differential enzymatic cleavage of DNA, an affinity capture of methylated DNA, an array, or any combination thereof.

Also described herein are methods comprising a) obtaining a biological sample from a male subject, wherein the biological sample comprises seminal fluid; b) extracting DNA from a sperm in the biological sample, extracting cell free DNA from the biological sample, or both; c) detecting in an *in vitro* analytical assay, methylation present in at least 5 regions of an individual promoter comprised in the extracted DNA, the extracted cell free DNA, or both from the biological sample, wherein the promoter is selected from Table 1; d) determining, with a computer program executed on a computer, a standard deviation for methylation in each of the at least 5 regions of the individual promoter; e) calculating, with the computer program executed on the computer, an average standard deviation for methylation of the individual promoter from the standard deviation of methylation in each of the at least 5 regions of the individual promoter; and f) determining, with the computer program executed on the computer, if the average standard deviation for methylation of the individual promoter is greater than or equal to three standard deviations from a reference standard deviation of methylation for the individual promoter.

Also described herein are methods comprising: a) obtaining a biological sample from a male subject, wherein the biological sample comprises seminal fluid; b) extracting DNA from a sperm in the biological sample, extracting cell free DNA from the biological sample, or both; c) detecting in an *in vitro* analytical assay, methylation present in at least 5 regions of an individual promoter comprised in the extracted DNA, the extracted cell free DNA, or both from the biological sample, wherein the promoter is selected from Table 1; and d) determining if an average standard deviation of the at least 5 regions of the individual promoter is greater than or equal to three standard deviations from a reference average standard deviation of the at least 5 regions of the individual promoter. In some embodiments, the method can comprise determining independently a standard deviation for methylation in each of the at least 5 regions of the individual promoter. In some embodiments, the method can comprise calculating an average standard deviation for methylation of the individual promoter from the standard deviation of methylation in each of the at least 5 regions of the individual promoter.

Also described herein are computer systems for analyzing a DNA from a sperm cell, a cell free DNA from a seminal sample, or both obtained from a male subject, the computer system comprising: a) a device for receiving sequenced data, wherein the sequenced data comprises methylation of at least 5 regions of an individual promoter comprised in the DNA from the sperm cell, the cell free DNA from the seminal sample, or both, and wherein the individual promoter is a promoter of Table 1; b) a device for determining independently a standard deviation for methylation in each of the at least 5 regions of the individual promoter and calculating an average standard deviation from the standard deviation from methylation in each of the at least 5 regions of the individual promoter; and c) a device for comparing the average standard deviation of the at least 5 regions of the individual promoter to a reference average standard deviation of at least 5 regions of the individual promoter and determining if the average standard deviation is greater than or equal to three standard deviations from the reference standard deviation of the individual promoter.

Also described herein is the use of an array used in detecting DNA methylation in at least 22 promoters selected from Table 1 from DNA obtained from a sperm cell, cell free DNA in a seminal sample, or both, wherein the DNA methylation is determined independently in at least 5 regions of an individual promoter for the manufacture of a diagnostic kit for determining male infertility of a human male subject. In some embodiments, wherein the use can further comprise: a) determining independently a standard deviation for methylation in each of the at least 5 regions of the individual promoter; b) calculating an average standard deviation for methylation of the individual promoter from the standard deviation of methylation in each of the at least 5 regions of the individual promoter; and c) determining if the average standard deviation for methylation of the individual promoter is greater than or equal to three standard deviations from a reference standard deviation of methylation for the individual promoter.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

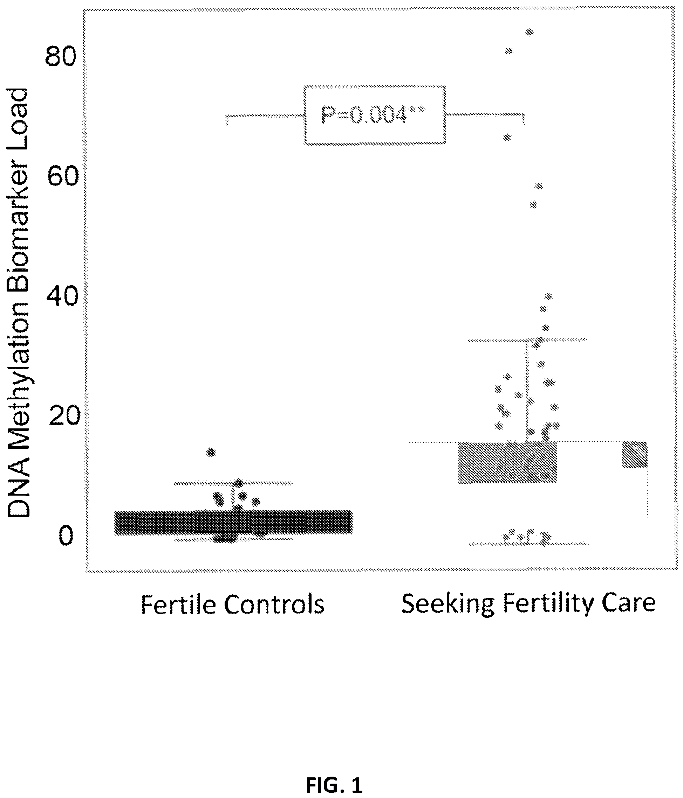

**FIG. 1** shows a bar graph of a DNA methylation analysis of 10,000 promoters from 112 men seeking fertility care and 54 men known to be fertile. The Y axis shows the number of dysregulated promoters methylated in the analysis.

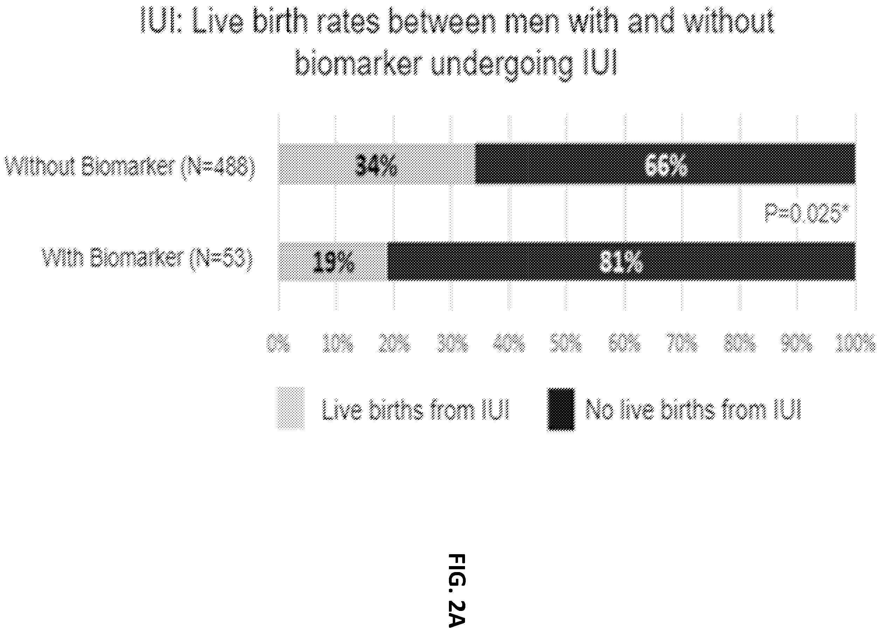

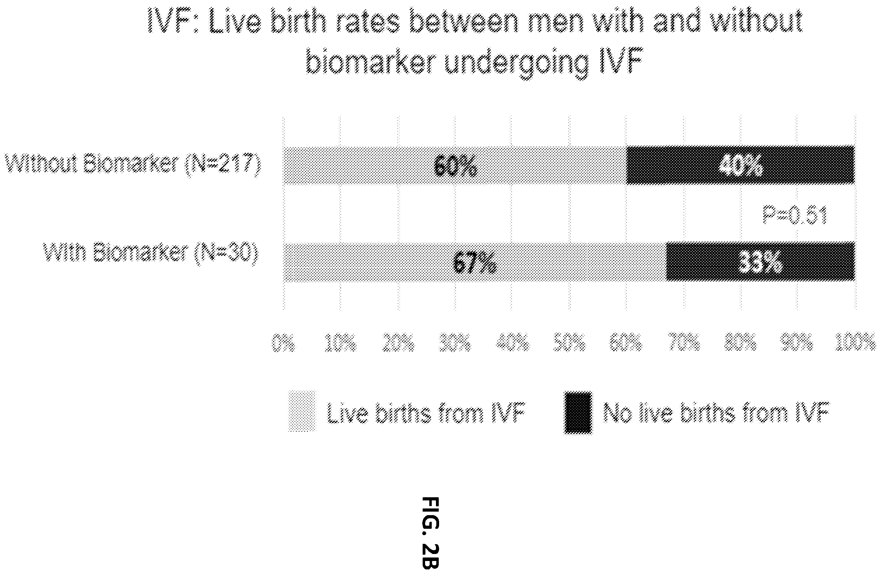

**FIGS. 2A-2B** shows a graph depicting intrauterine insemination (IUI) and *in vitro* fertilization (IVF) birth rates of men seeking fertility care with and without the presence of a methylated promoter biomarker screened from 1336 semen samples from these men. **FIG. 2A** shows the live birth rates of men undergoing IUI with and without the methylated promoter biomarker. **FIG. 2B** shows the live birth rates of men undergoing IVF with and without the methylated promoter biomarker.

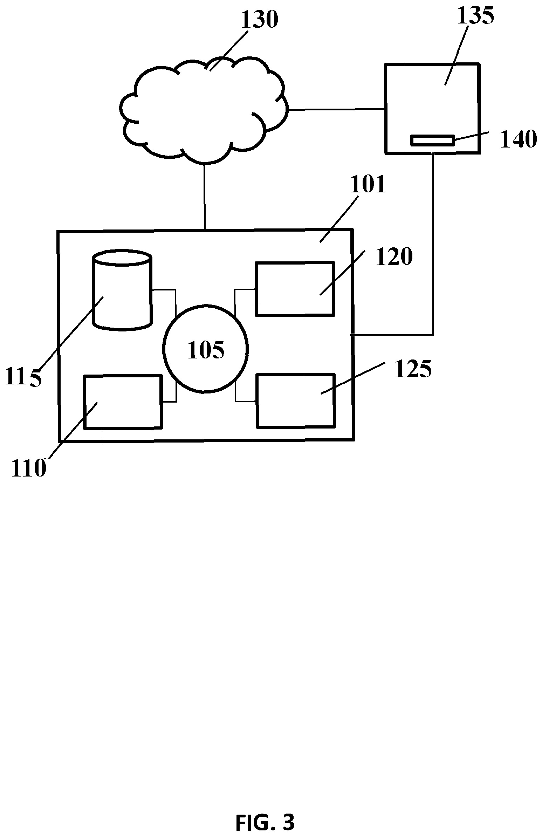

**FIG. 3** shows a computer control system that is programmed or otherwise configured to implement methods provided herein.

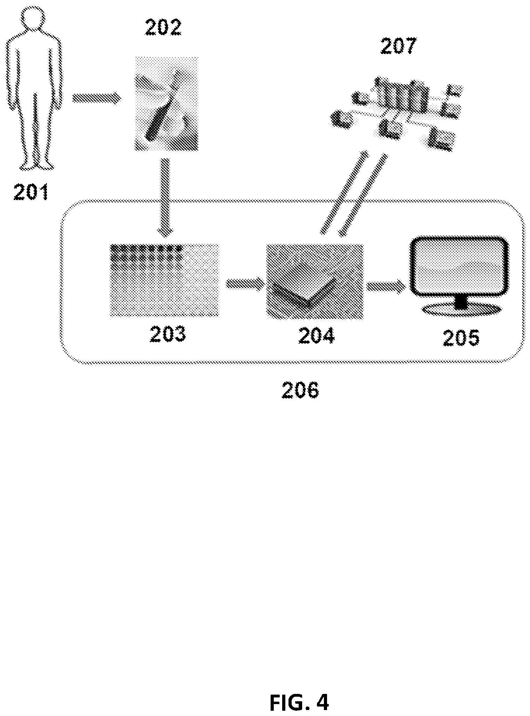

**FIG. 4** shows a method and system as disclosed herein.

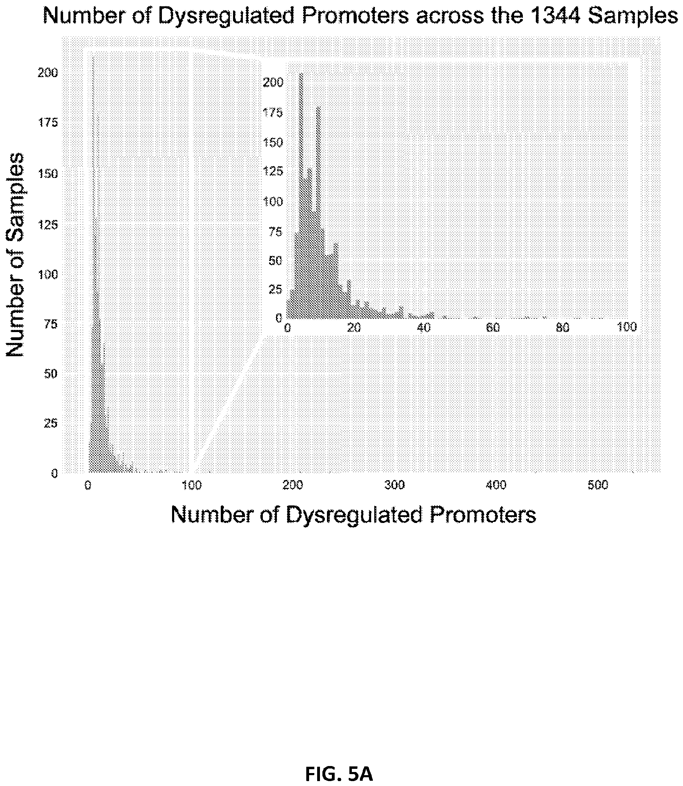

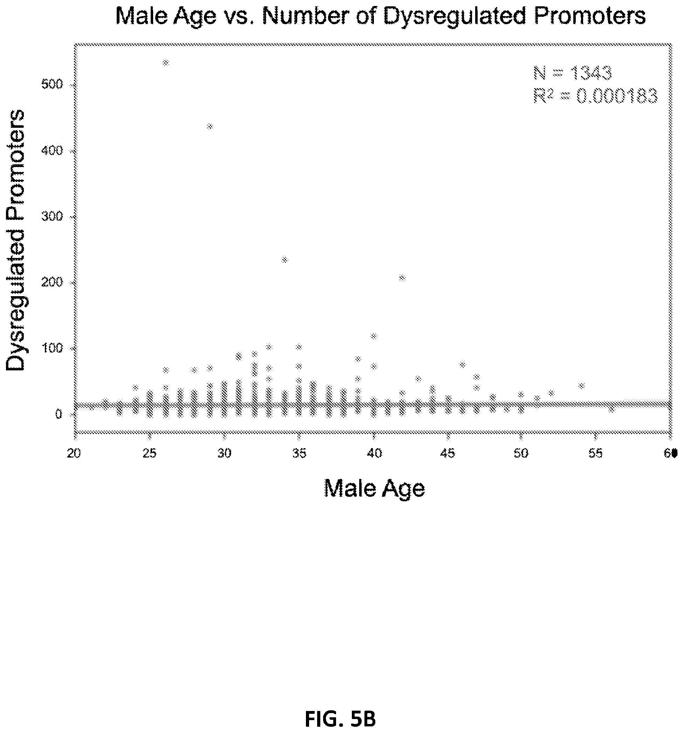

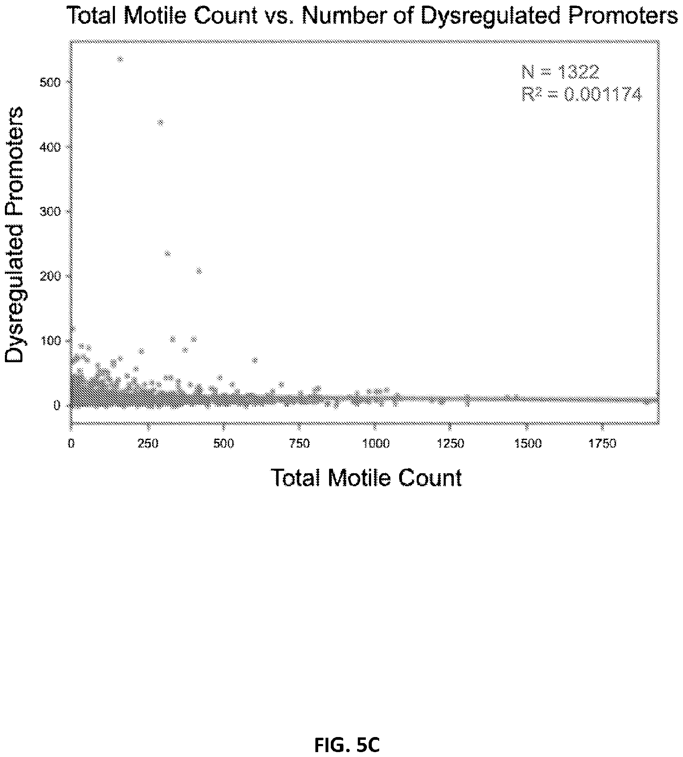

**FIGS. 5A-C** shows graphs analyzing sperm promoters. **FIG. 5A** depicts a graph showing the number of samples (on the Y-axis) from 1344 sperm DNA samples and the number of dysregulated promoters on the X-axis. **FIG. 5B** depicts a graph that shows the number of dysregulated promoters on the Y-axis associated with the age of a male on the X-axis. **FIG. 5C** depicts a graph that shows the number of dysregulated promoters on the Y-axis associated with the total motile sperm count X-axis.

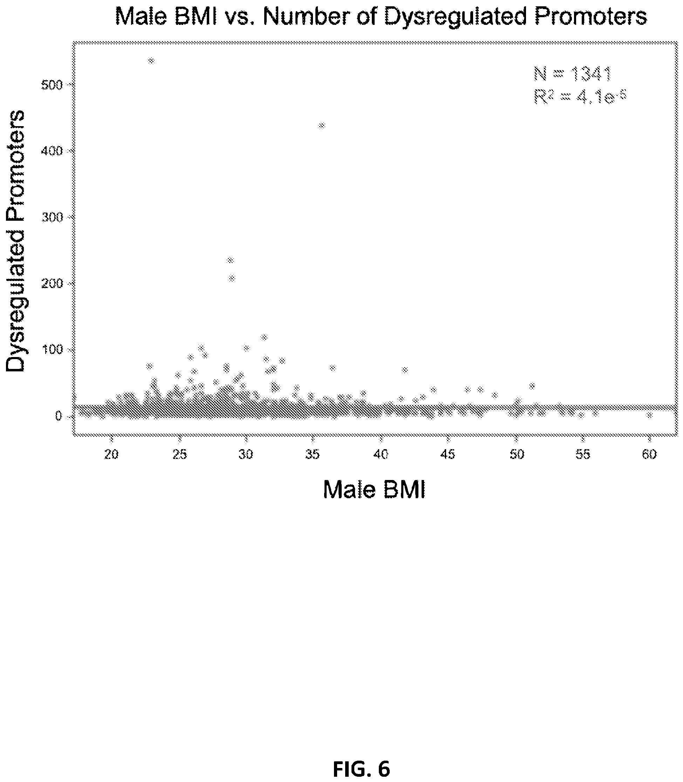

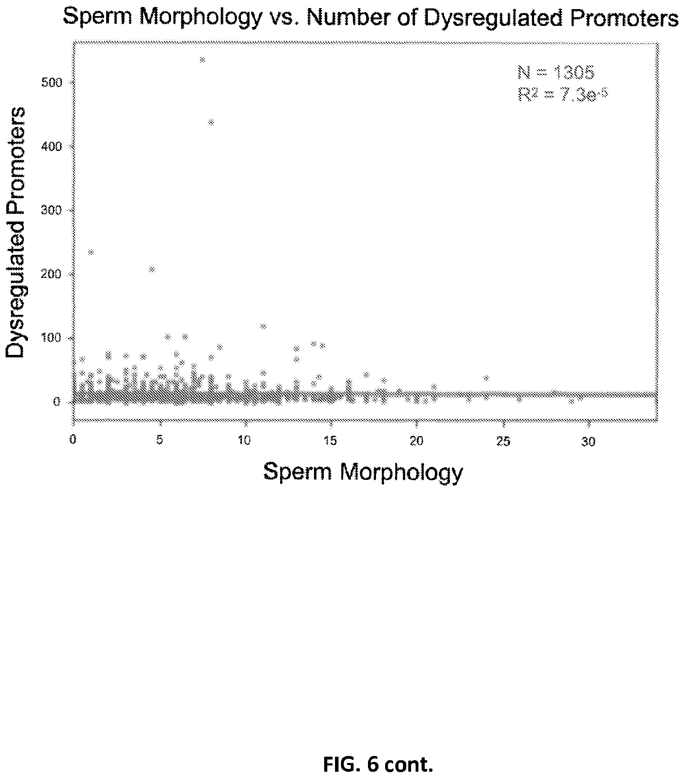

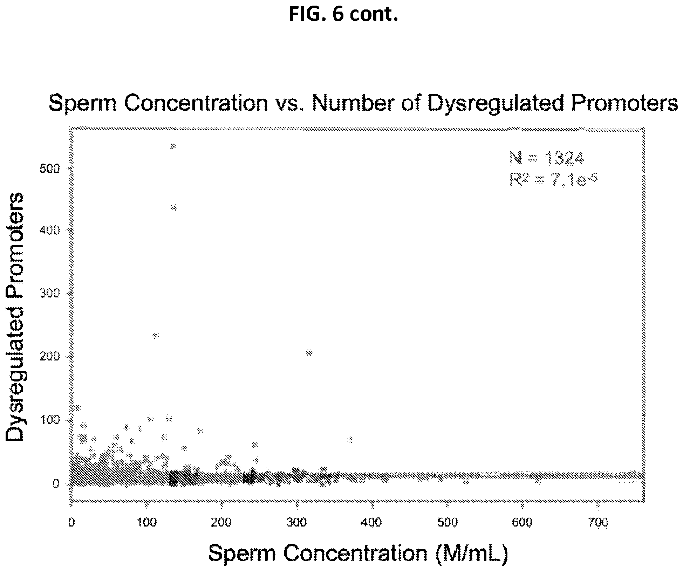

**FIG. 6 s**hows graphs analyzing the male body mass index (BMI) versus number of dysregulated promoters, sperm concentration versus number of dysregulated promoters, and sperm morphology versus number of dysregulated promoters. The Y-axis depicts the number of dysregulated promoters, the X-axis depicts the Male BMI, the morphology of sperm, or the concentration of sperm for each of the 1344 sperm DNA samples.

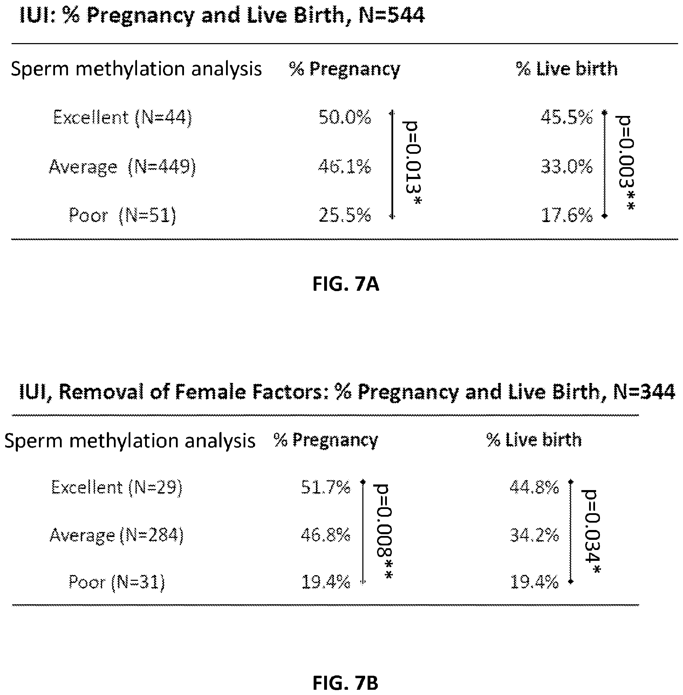

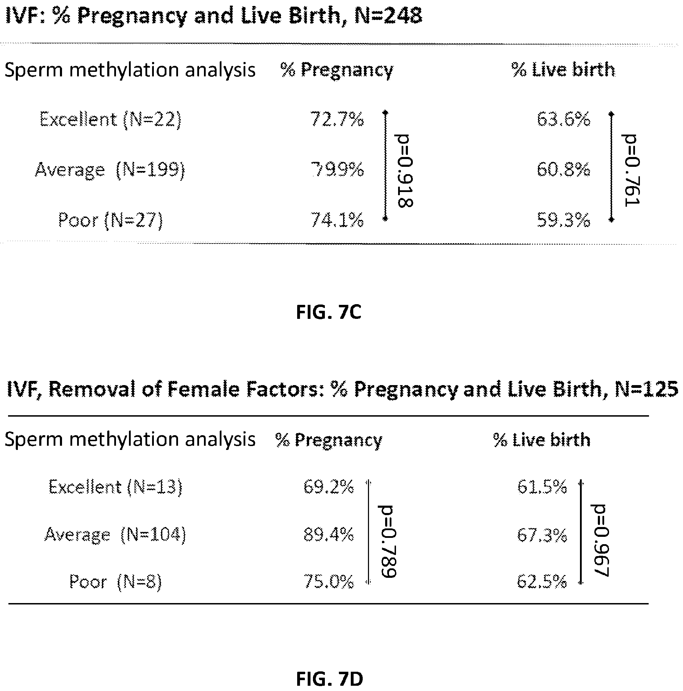

**FIGS. 7A-7D** show analysis of the percent of live births and pregnancies resulting from men undergoing IUI and IVF and the result from the sperm methylation analysis; Excellent" ($\leq 3$ dysregulated promoters), "Average" (between 4 to 21 dysregulated promoters), and "Poor" ($\geq 22$ dysregulated promoters). **FIG. 7A** shows the percent of live births and pregnancies resulting from men undergoing IUI. **FIG. 7B** shows the percent of live births and pregnancies resulting from men undergoing IUI whose female partners had no female infertility factors. **FIG. 7C** shows the percent of live births and pregnancies resulting from men undergoing IVF. **FIG. 7D** shows the percent of live births and pregnancies resulting from men undergoing IVF whose female partners had no female infertility factors.

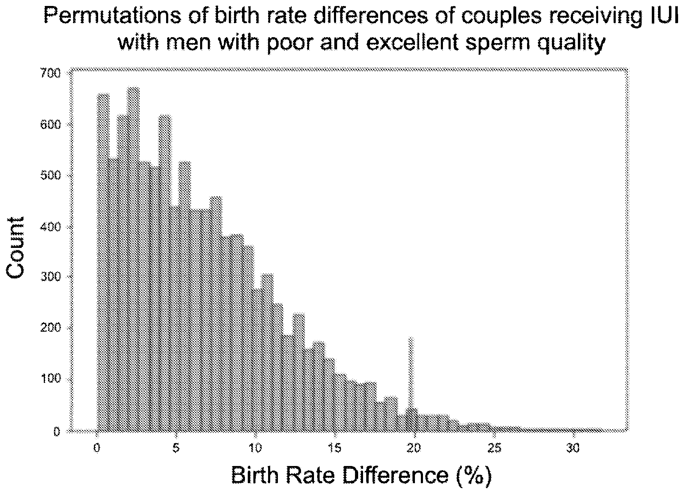

**FIG. 8** shows a histogram depicting live birth rate differences of men with poor and excellent sperm quality after 10,000 permutations of shuffling live birth data of all couples receiving IUI but not IVF. The line at 19.85% represents the birth rate difference between the men with poor and excellent sperm quality in the actual data set (from couples receiving IUI but not IVF) which represents the 99th percentile of permutations.

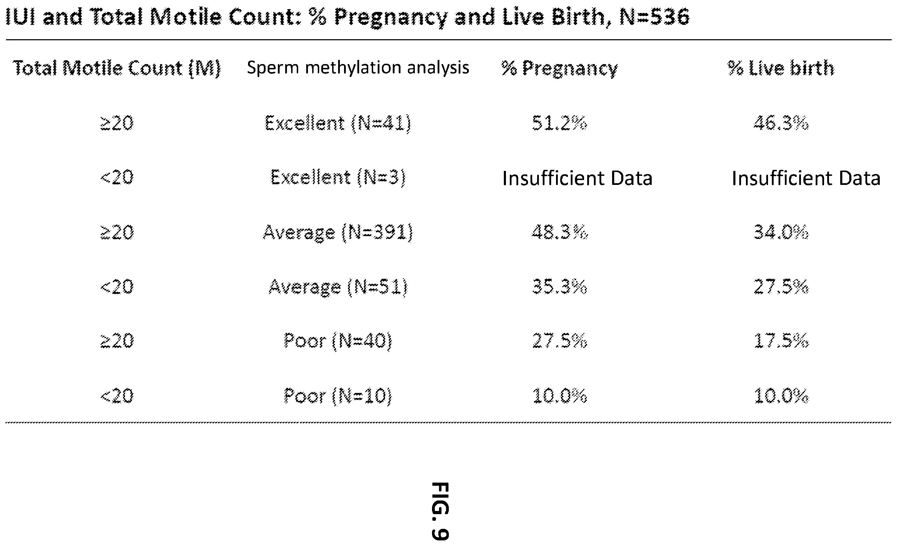

**FIG. 9** shows analysis of the percent of live births and pregnancies resulting from men undergoing IUI. The analysis shows total motile count and the result from the sperm methylation analysis; "Excellent" ($\leq 3$ dysregulated promoters), "Average" (between 4 to 21 dysregulated promoters), and "Poor" ($\geq 22$ dysregulated promoters).

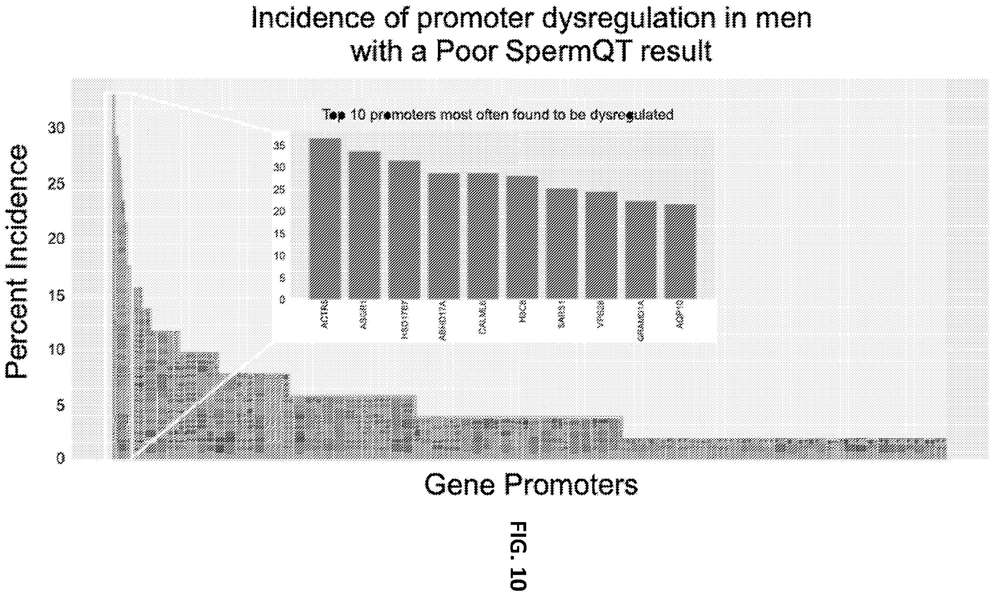

**FIG. 10** shows an analysis of the dysregulated promoters in the 1233 target gene promoters across all samples with a poor score. The Y-axis shows the percent incidence of dysregulation of an individual promoter while the X-axis shows the individual promoters indicated by individual bars. The top ten promoters that are most often found to be dysregulated are shown blown up in the figure. The top ten promoters were ACTR5, ASGR1, HSD17B7, ABHD17A, CALML6, H3C8, SARS1, VPS28, GRAMD1A, AQP10.

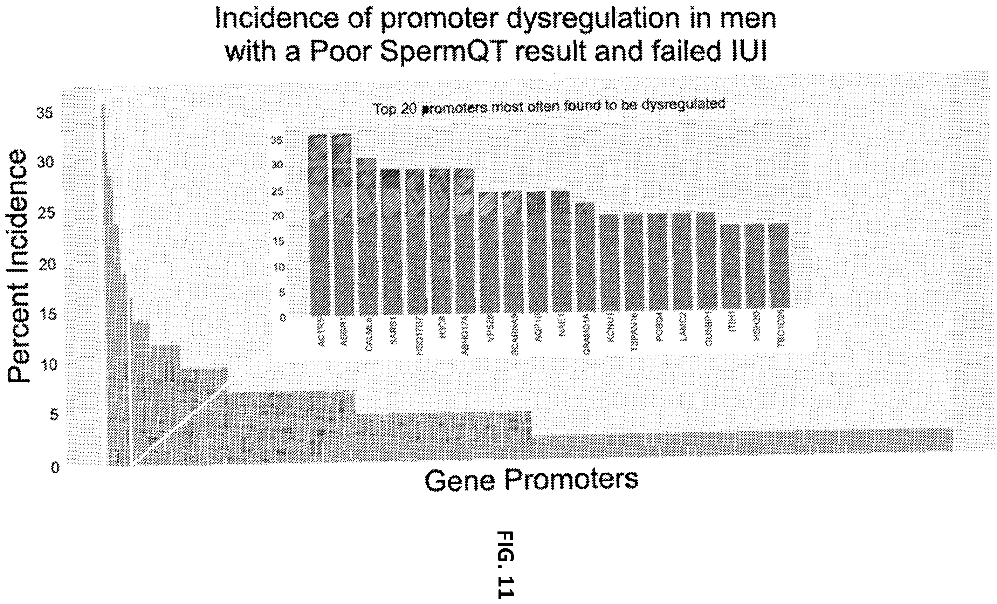

**FIG. 11** shows an analysis of the dysregulated promoters in the 1233 target gene promoters across samples with a Poor score and samples from mem who failed IUI. The Y-axis shows the percent incidence of dysregulation of an individual promoter while the X-axis shows the individual promoters indicated by individual bars. The top twenty promoters that are most often found to be dysregulated are shown blown up in the figure. The top twenty promoters were ACTR5, ASGR1, CALML6, SARS1, HSD17B7, H3C8, ABHD17A, VPS28, SCARNA9, AQP10, NAE1, GRAMD1A, KCNU1, TSPAN16, PGBD4, LAMC2, GUSBP1, ITIH1, HSH2D, TBC1D26.

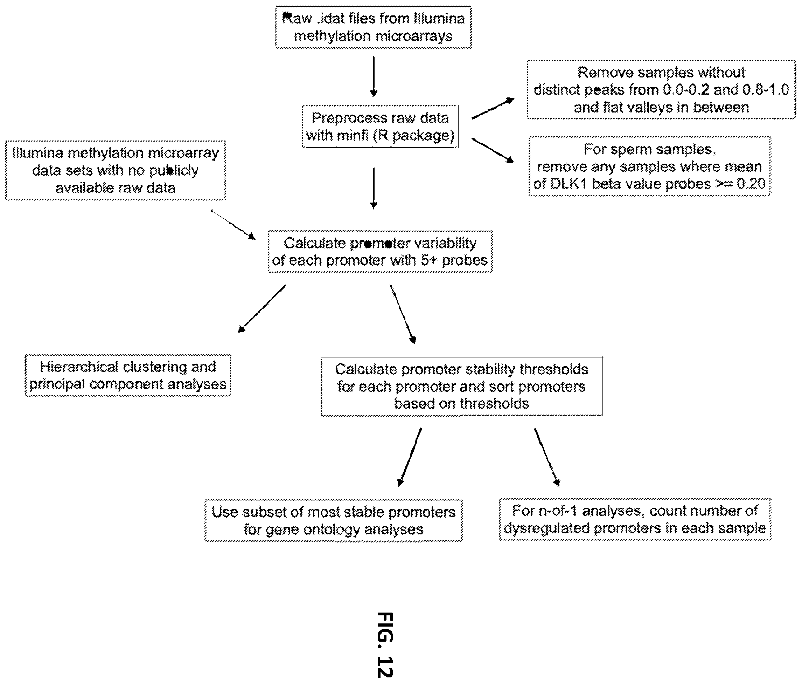

**FIG. 12** shows the outline of the a data processing and statistical analysis workflow. The diagram shows the processing and analysis of array data from multiple tissue types to derive promoter variability and promoter stability thresholds and analyze their relationships among tissue types and between healthy and diseased tissues.

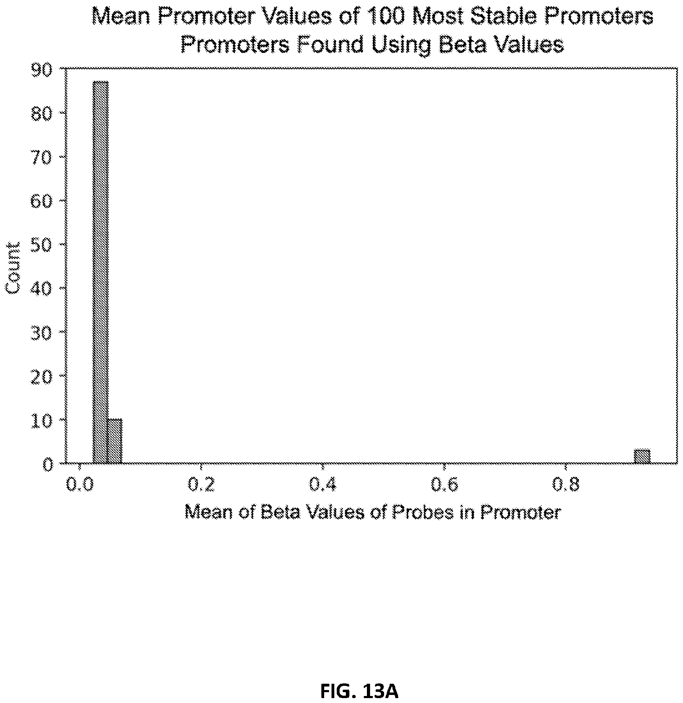

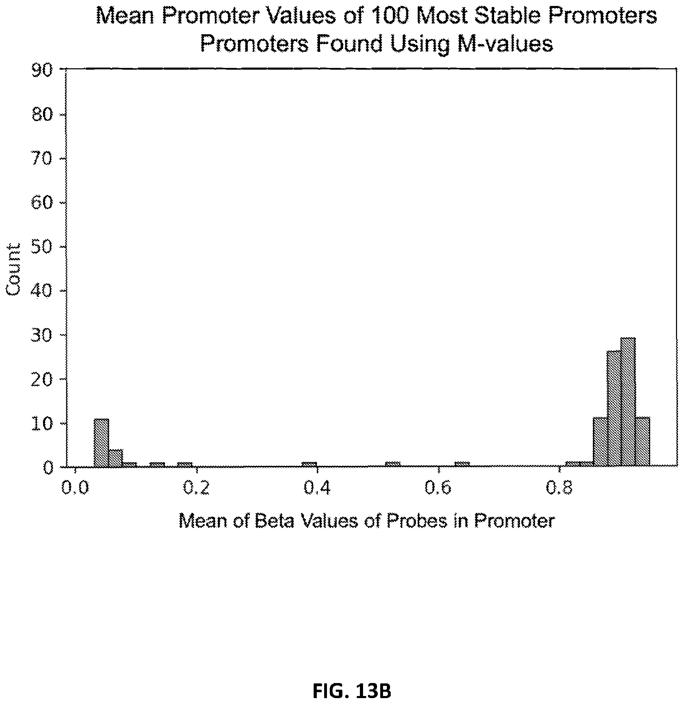

**FIGS. 13A-B** shows heteroscedasticity of beta values in a sperm donor sample. **FIG. 13A** shows the distribution of mean promoter methylation of the 100 most stable promoters in sperm which were found by calculating the variability value of the beta values of all probes in a promoter region. **FIG. 13B** shows the distribution of mean promoter methylation of the 100 most stable promoters in sperm which were found by calculating the variability value of the m-values of all probes in a promoter region.

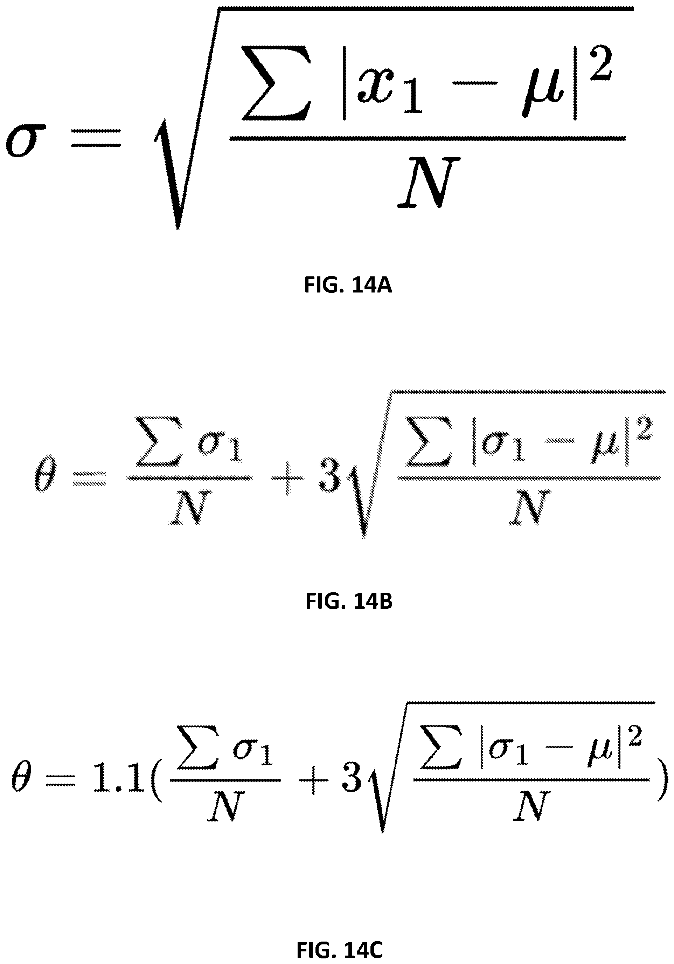

**FIGS. 14A-C** shows the variability equations used in the identification of promoter dysregulation. **FIG. 14A** shows the equation for calculating the variability value (or standard deviation) of a given promoter in a sample; $\sigma$ = gene promoter variability value, x1 = m-value of a given methylation array probe in a given promoter, $\mu$ = mean of probe m-values in a given promoter. **FIG. 14B** and **FIG. 14C** shows equations to calculate the promoter variability threshold (*e.g.*, 3 or greater than 3 standard deviations from a reference promoter average standard deviation) for methylation for a given tissue. $\Theta$ = promoter variability threshold for a given tissue, $\sigma_1$ = promoter variability value of a sample in a given cohort at a given promoter, $\mu$ = mean of the methylation variability values of a given promoter, and N=number of samples.

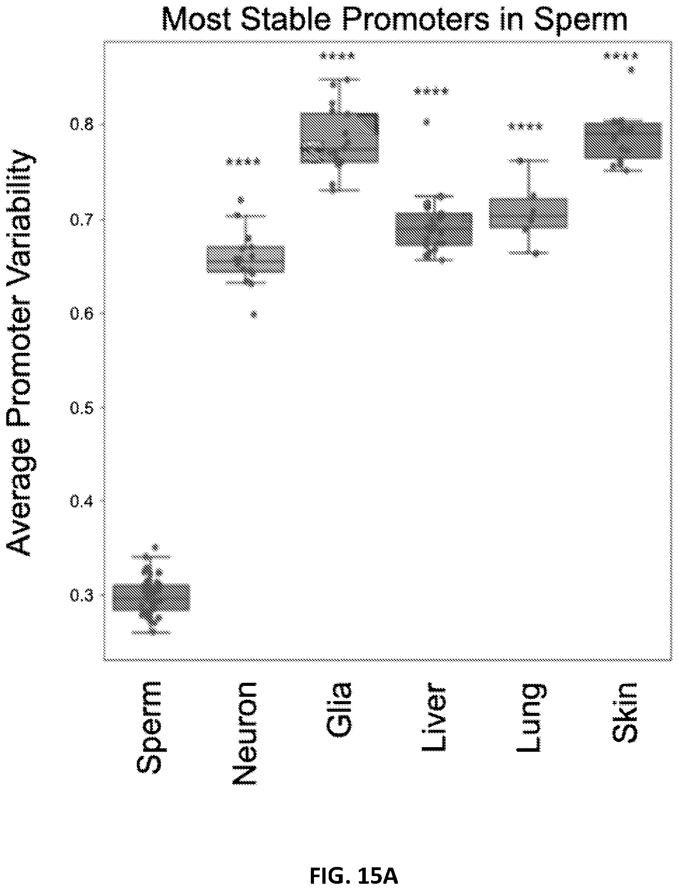

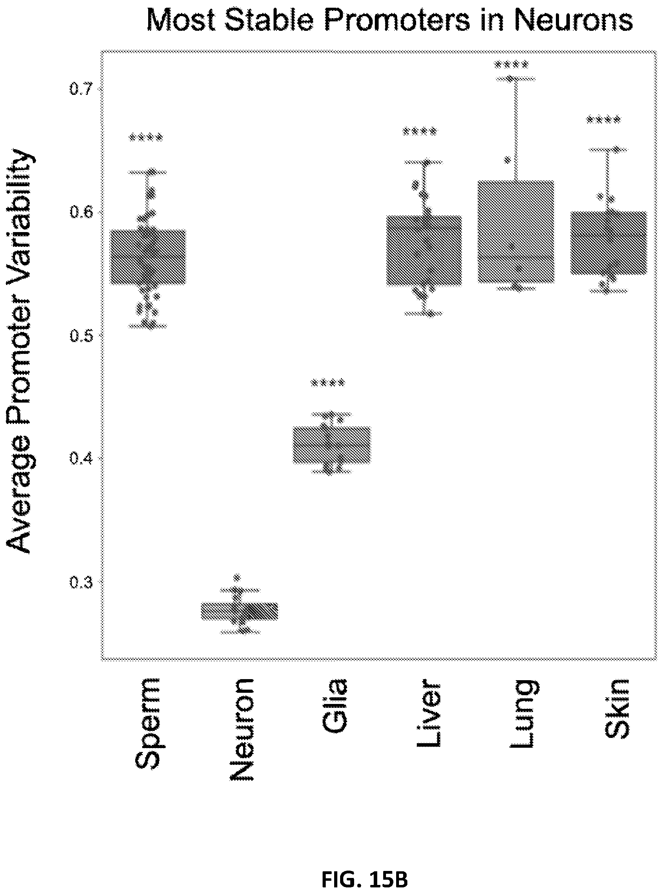

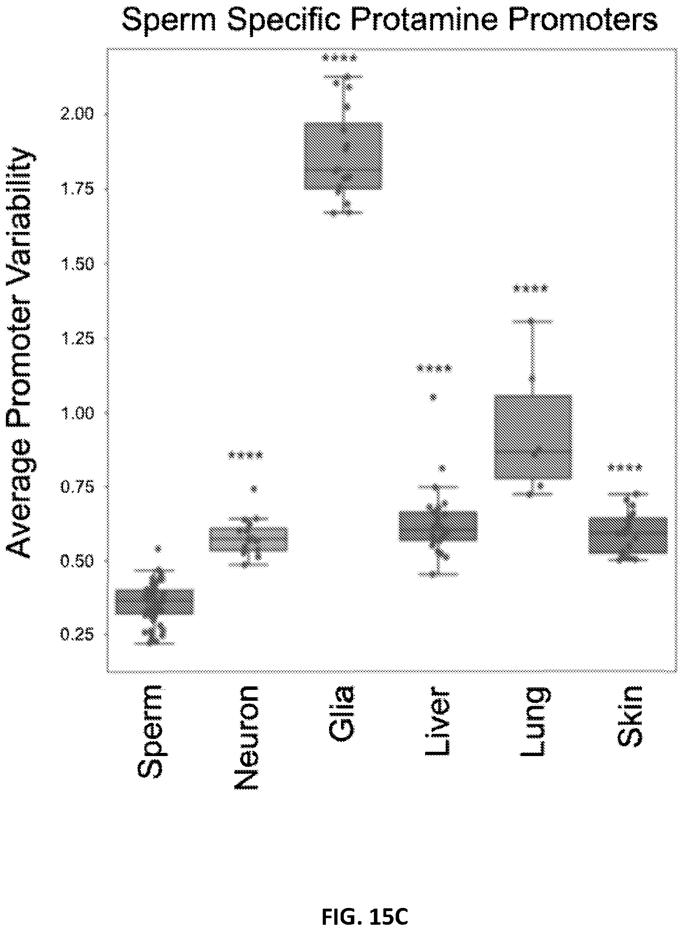

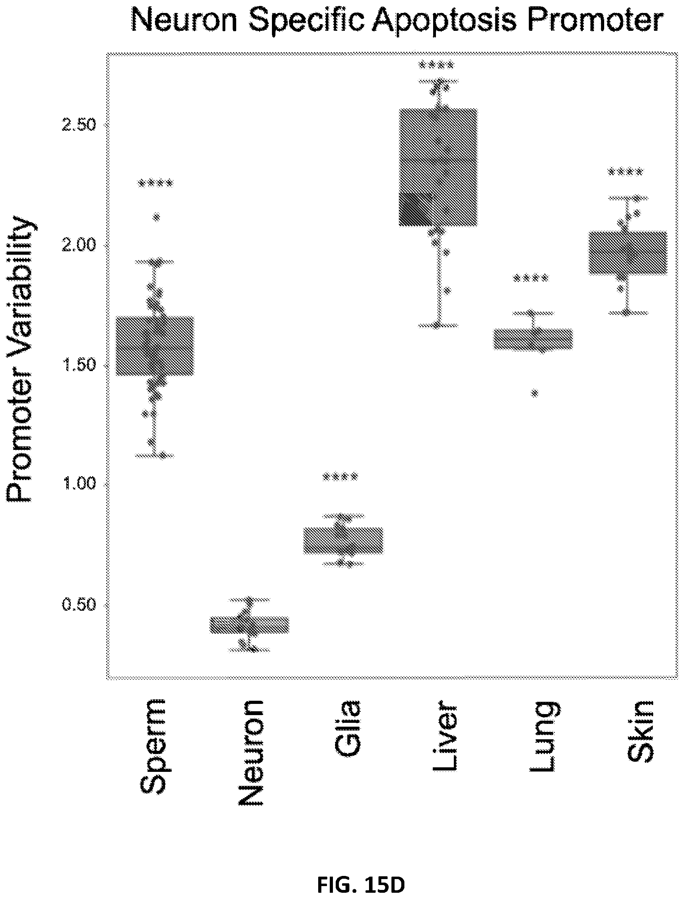

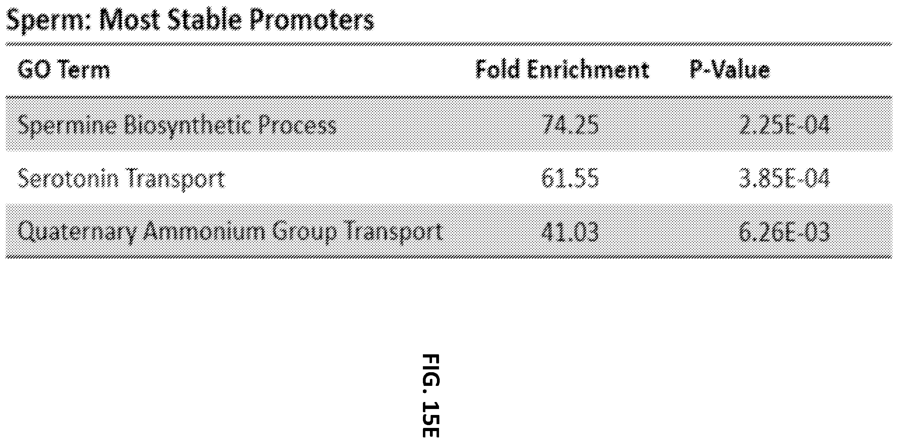

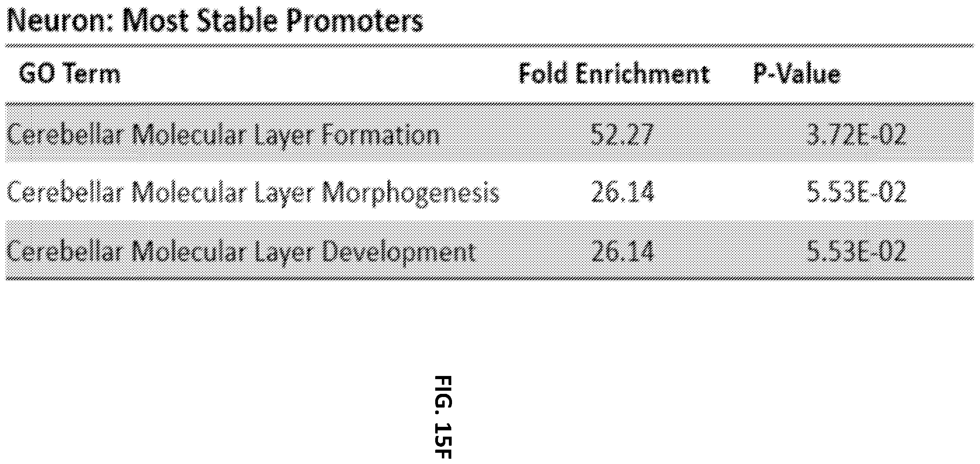

**FIGS. 15A-F** show data indicating tissues have patterns of gene methylation promoter variability. **FIG. 15A** and **FIG. 15B** show the average promoter variability of 6 distinct cell types in the most stable promoters (top 1st percentile) in sperm and neurons, respectively. One dot represents one sample, and boxplots are overlaid to show the distribution of average promoter variability of each tissue. All p-values comparing methylation variance between sperm and neuron to other tissues types were $\leq 5.16E-14$. **FIG. 15C** shows the average promoter variability in the 6 cell types of three sperm-specific protamine promoters. **FIG. 15D** shows the promoter variability from one neuron specific apoptosis promoter in the 6 cell types. **FIG. 15E** and **FIG. 15F** illustrate the gene ontology enrichment of the most stable promoters for sperm and neurons, respectively. All p-values for **FIG. 15E** and **FIG. 15F** when comparing sperm and neurons to other cell types were $\leq 9.99E-17$.

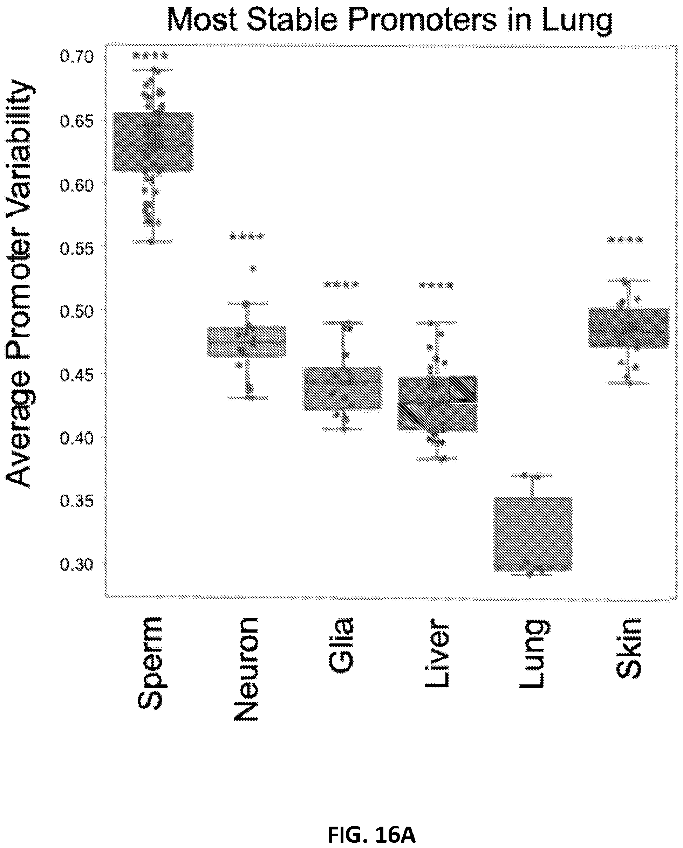

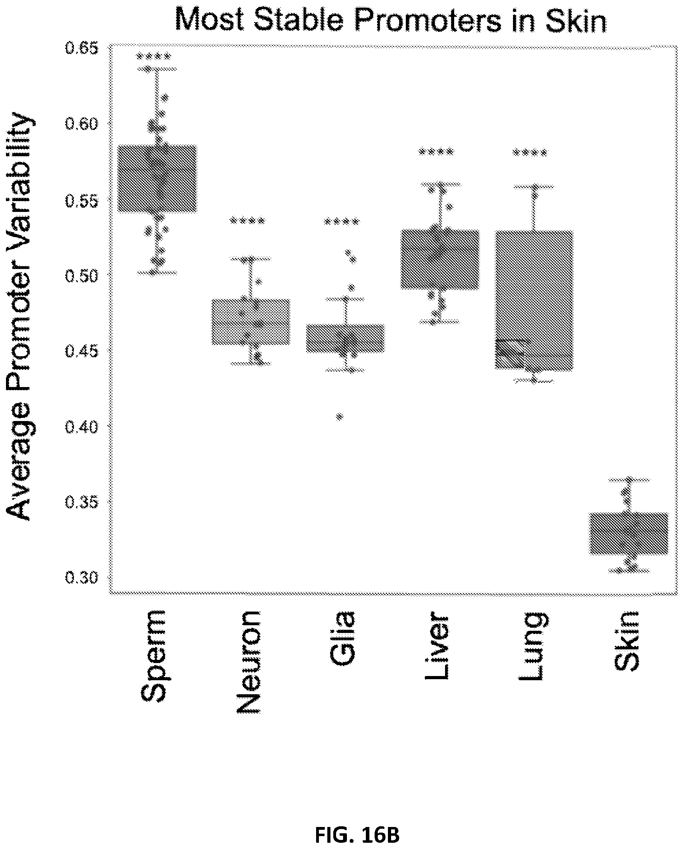

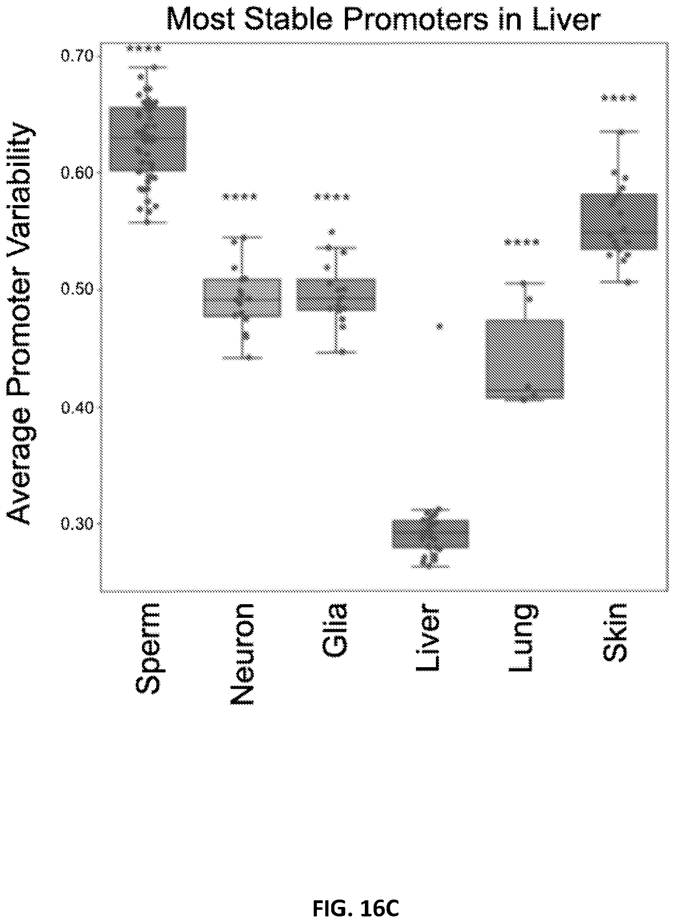

**FIGS. 16A-C** show average promoter variability at tissue-specific promoters. **FIG. 16A** shows the average promoter variability values of numerous samples across several tissues at the most stable promoters in control lung tissue. **FIG. 16B** shows the average promoter variability values of numerous samples across several tissues at the most stable promoters in control skin tissue. **FIG. 16C** shows average promoter variability values of numerous samples across several tissues at the most stable promoters in control liver tissue.

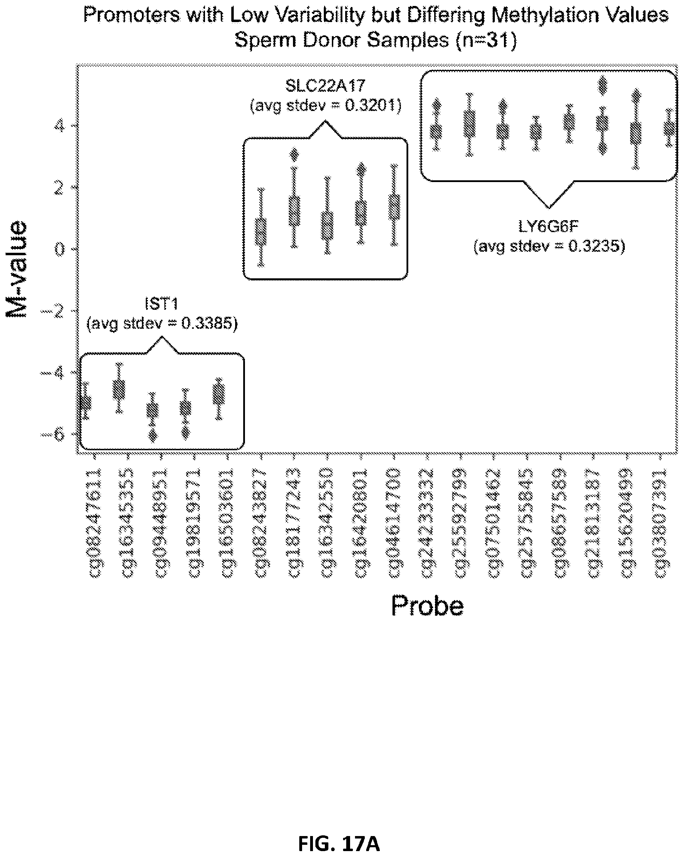

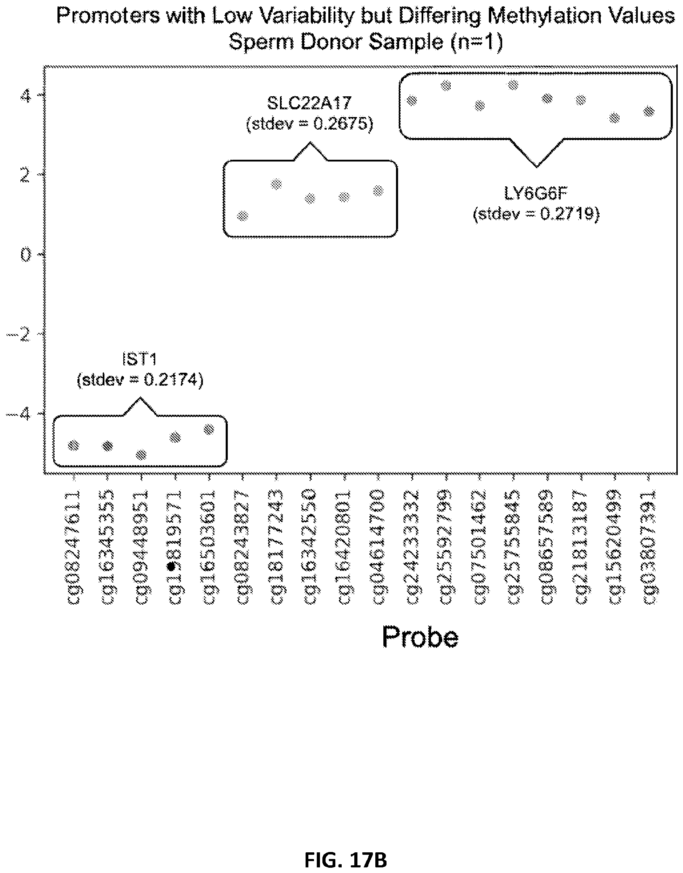

**FIGS. 17A-B** show examples of promoters with low methylation variability but varying levels of methylation. **FIG. 17A** shows a boxplot of methylation values of 31 sperm donor samples at 3 gene promoters with low methylation variability. **FIG. 17B** shows a dotplot of methylation values of 1 sperm donor sample at 3 gene promoters with low methylation variability.

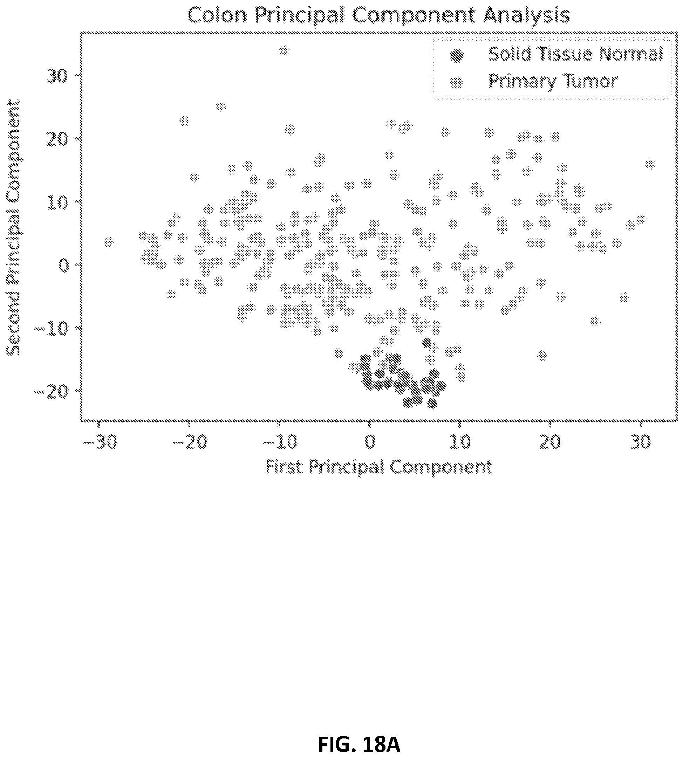

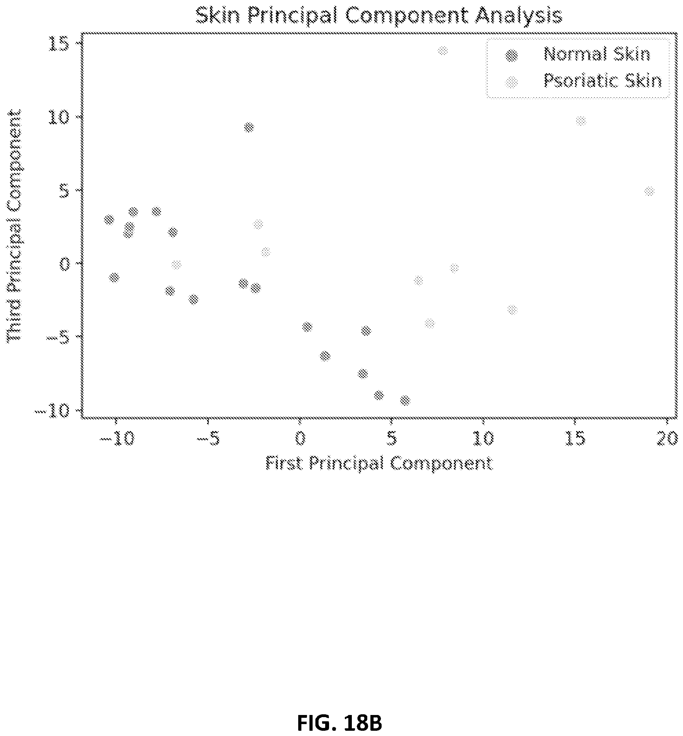

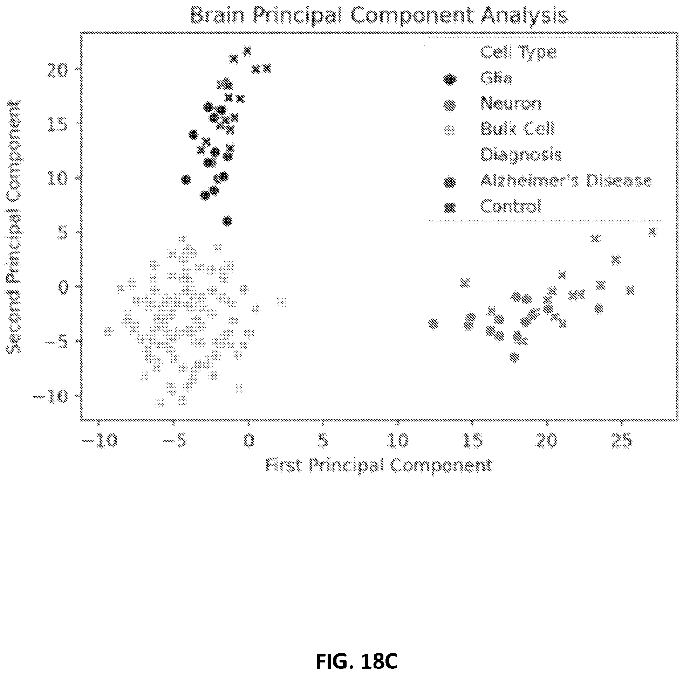

**FIGS. 18A-C** show principal component analysis (PCA) plots of diseased tissue samples have patterns of gene methylation promoter variability compared to healthy samples. **FIG. 18A** shows the principal component analysis of promoter variability values from primary colon tumors and normal colon tissue. **FIG. 18B** shows the principal component analysis of promoter variability values from matched psoriatic lesion and healthy skin samples. **FIG. 18C** shows the principal component analysis of promoter variability among neurons, glial cells, and bulk cells from postmortem brains of individuals with Alzheimer's disease as well as controls.

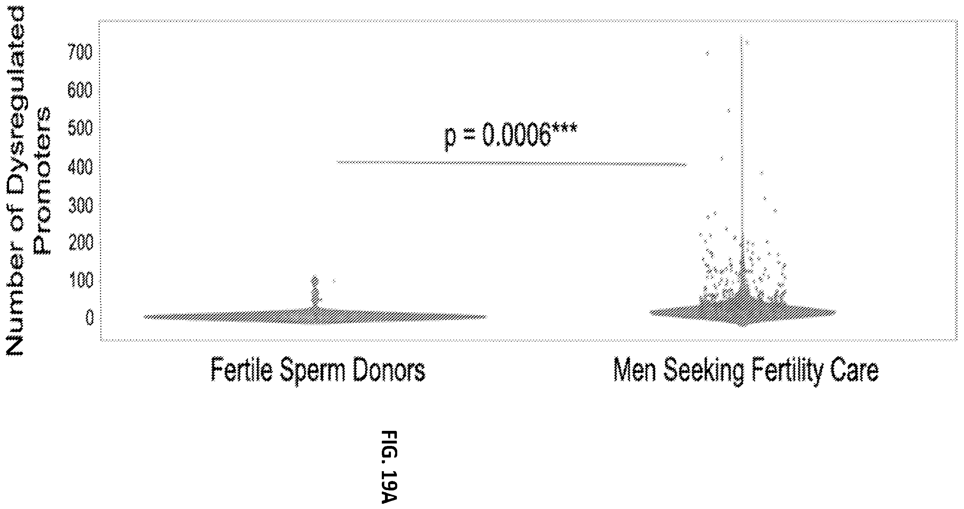

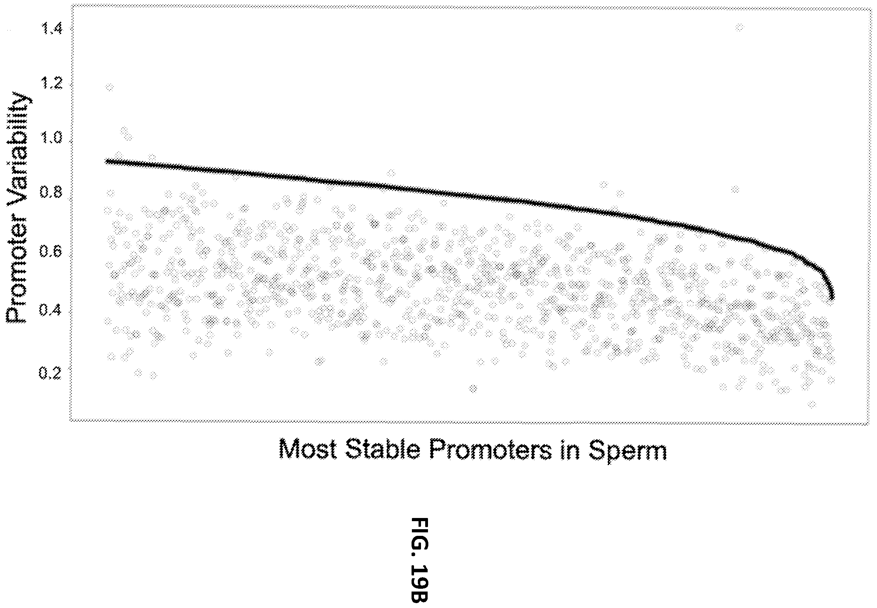

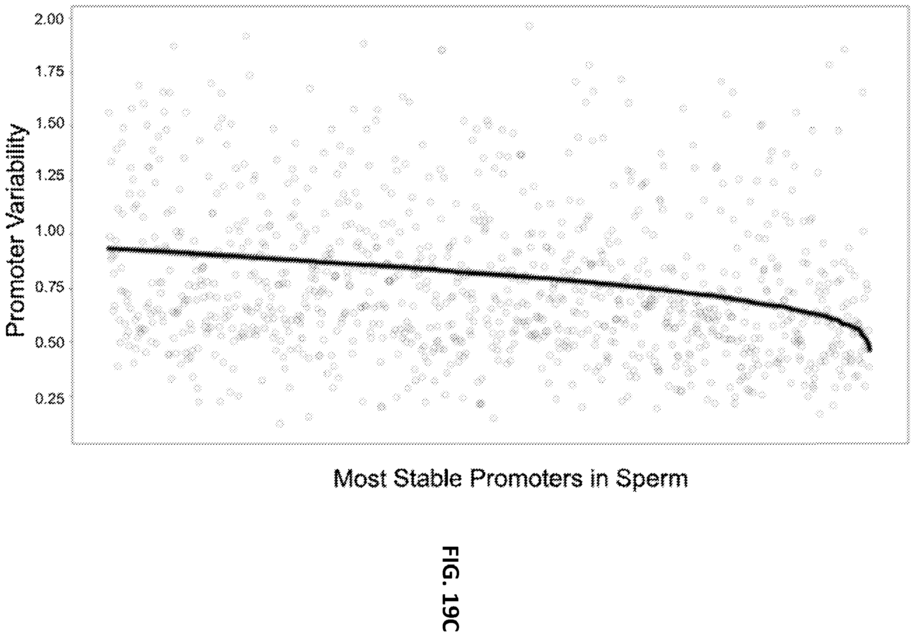

**FIGS. 19A-C** show dysregulated promoters are enriched in men seeking fertility care compared to fertile controls. **FIG. 19A** shows how many dysregulated promoters were in samples from five independent studies. The most stable sperm promoters and corresponding stability thresholds were calculated from a cohort of fertile sperm donor samples. **FIG. 19B** depicts the promoter variability at the most stable sperm promoters in a single fertile donor sperm sample (dots). The stability threshold for these promoters are shown in black. A dot above the black line indicates a dysregulated promoter. **FIG. 19C** depicts this analysis in a single patient being treated for male factor infertility.

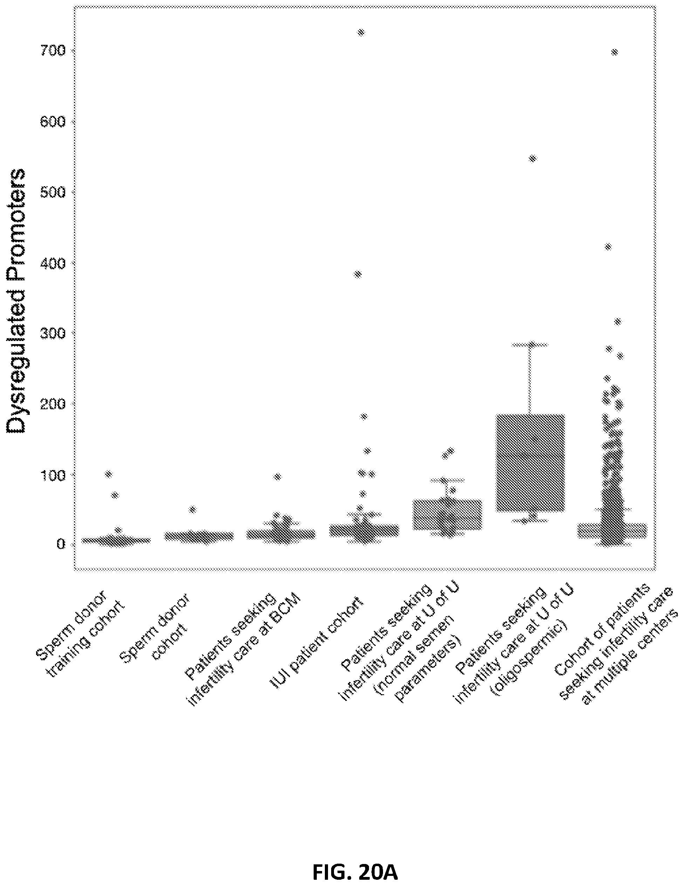

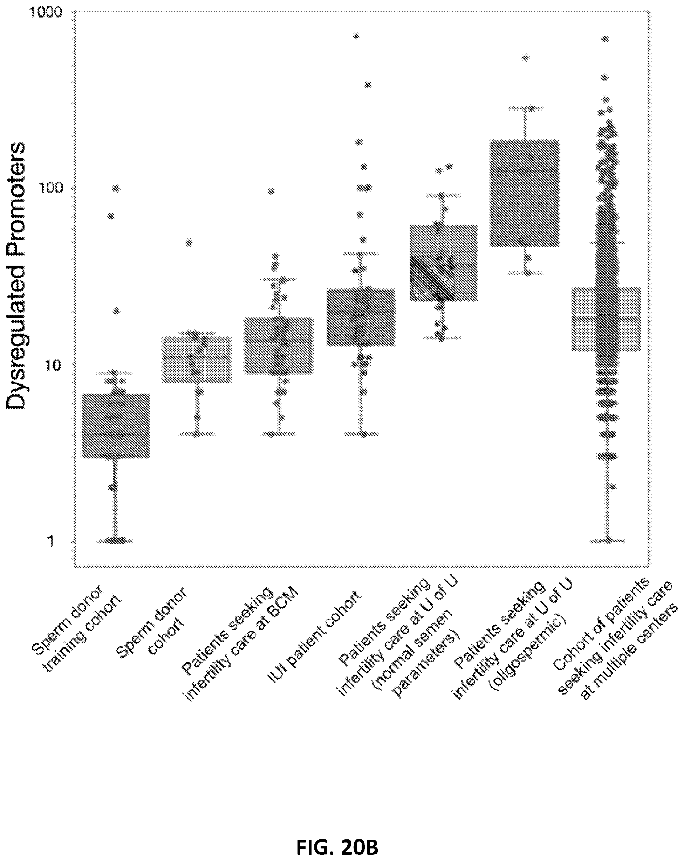

**FIGS. 20A-B** show an N-of-1 dysregulated promoter analysis of sperm samples from multiple cohorts. The plots look at the number of dysregulated promoters from the most stable promoters in sperm. The "Sperm donor cohort (training)" is the cohort used to find the most stable promoters in sperm and set the promoter variability thresholds. **FIG. 20A** shows the number of dysregulated promoters in various sperm sample cohorts on a linear scale and **FIG. 20B** shows the same plot but on a logarithmic scale.

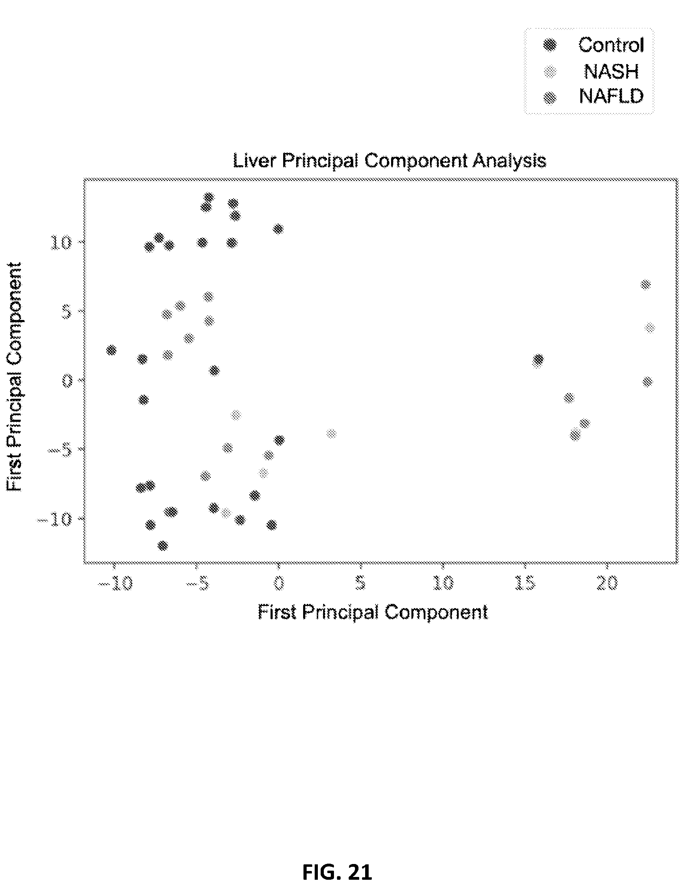

**FIG. 21** shows a principal component analysis of diseased and control samples. The plot shows the principal component analysis of liver samples from healthy individuals and those with nonalcoholic fatty liver disease (NAFLD) and nonalcoholic steatohepatitis (NASH).

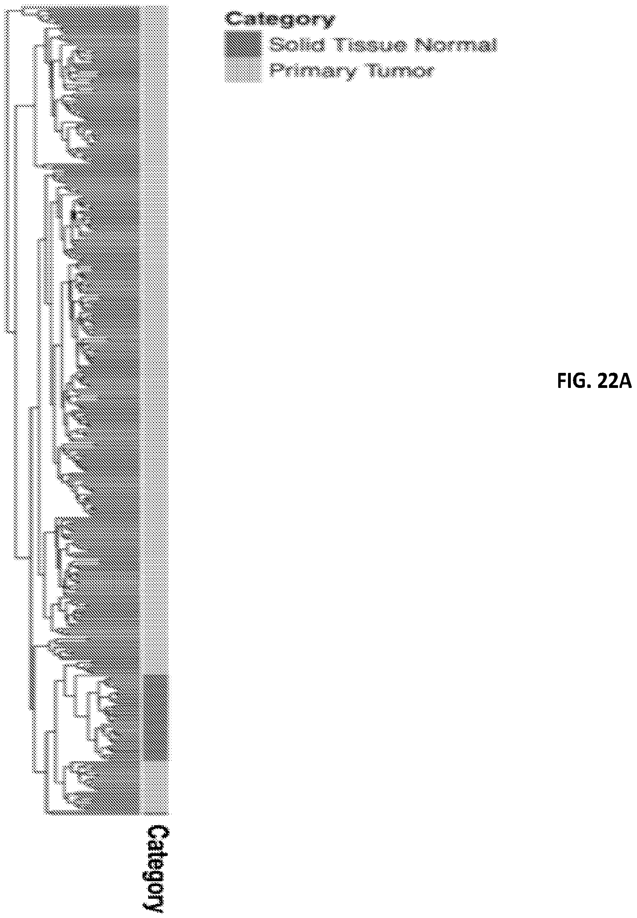

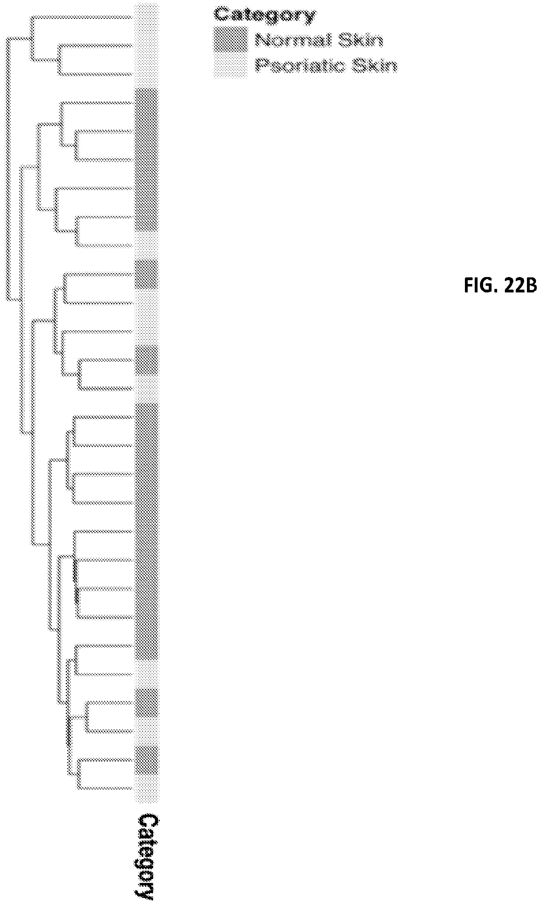

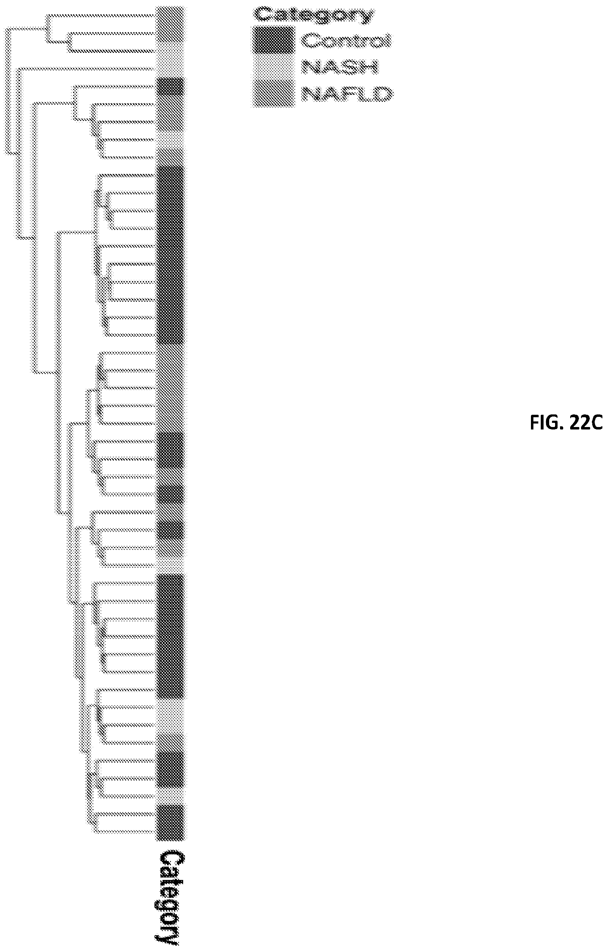

**FIGS. 22A-C** show plots of hierarchical clustering of diseased and control tissue samples. Clustering is based on the variability values of all promoters. **FIG. 22A** shows a plot of hierarchical clustering of colon primary tumor samples and normal colon tissue samples. **FIG. 22B** shows a plot of hierarchical clustering of paired psoriatic skin lesion samples and normal skin samples. **FIG. 22C** shows a plot of hierarchical clustering of control, nonalcoholic fatty liver disease (NAFLD), and nonalcoholic steatohepatitis (NASH) liver samples.

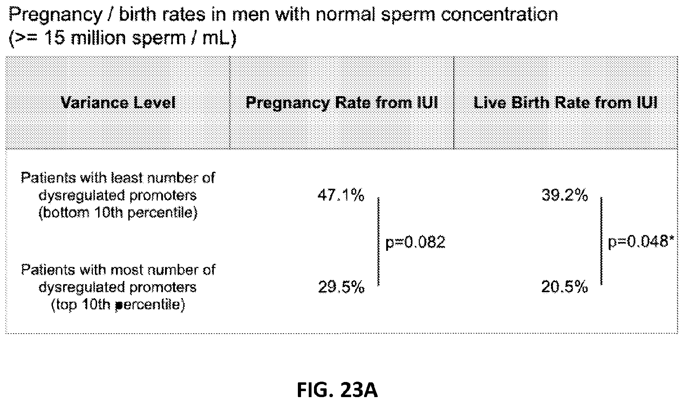

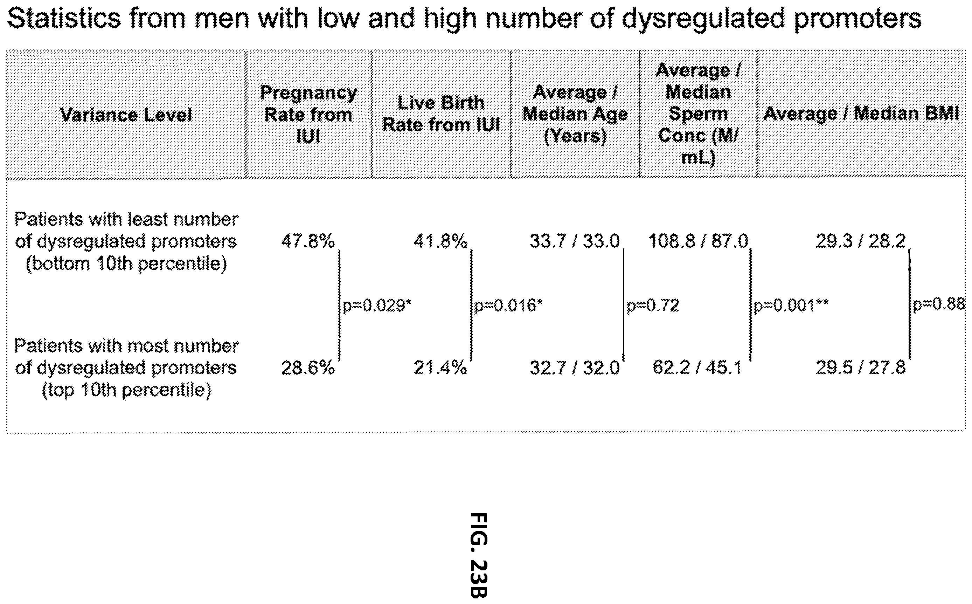

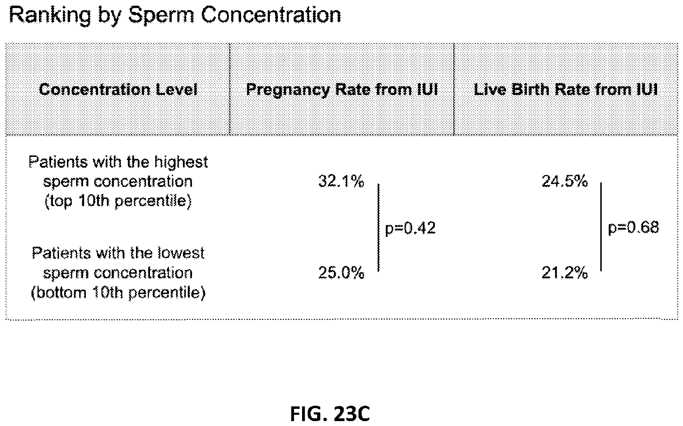

**FIGS. 23A-C** show further assessments of sperm promoter dysregulation and sperm concentration within a data set. **FIG. 23A** shows pregnancy rates and birth rates in men with normal sperm concentration ($\geq$ 15 million sperm / mL). **FIG. 23B** shows statistics from men with low and high number of dysregulated promoters. **FIG. 23C** shows the statistics of pregnancy rate from IUI and live birth rate from IUI from men with the highest sperm concentration vs men with the lowest sperm concentration from a data set.

## DETAILED DESCRIPTION OF THE DISCLOSURE

### Definitions

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Unless otherwise indicated, open terms for example "contain," "containing," "include," "including," and the like mean comprising.

The singular forms "a", "an", and "the" can be used herein to include plural references unless the context clearly dictates otherwise. For example, the term "a sample" includes a plurality of samples, including mixtures thereof.

As used herein, the term "about" or "approximately" a number can refer to that number plus or minus 10% of that number. In some cases, about or approximately can refer to that number plus or minus 5% of that number. The term about or approximately a range can refer to that range minus 10% of its lowest value and plus 10% of its greatest value. In some cases, the term about or approximately a range can refer to that range minus 5% of its lowest value and plus 5% of its greatest value. Alternatively, particularly with respect to biological systems or processes, the term can mean within an order of magnitude, within 5-fold, or within 2-fold, of a value. Where particular values or values of a range are described in the application and claims, unless otherwise stated the term "about" meaning within an acceptable error range for the particular value should be assumed.

The term "substantially" or "essentially" can refer to a qualitative condition that exhibits an entire or nearly total range or degree of a feature or characteristic of interest. In some cases, substantially can refer to at least about: 70%, 75%, 80%, 85%, 90%, 91%, 92%, 93%, 94%, 95%, 96%, 97%, 98%, 99% or 100% of the total range or degree of a feature or characteristic of interest.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 5 should be considered to have specifically disclosed subranges such as from 1 to 2, from 1 to 3, from 1 to 4, from 2 to 4, from 3 to 5, etc., as well as individual numbers within that range, for example, 1, 2, 3, 4, and 5. This applies regardless of the breadth of the range.

The terms "determining", "measuring", "evaluating", "assessing," "assaying," and "analyzing" are often used interchangeably herein to refer to forms of measurement and include determining if an element may be present or not (for example, detection). These terms can include quantitative, qualitative or quantitative, and qualitative determinations. Assessing can be alternatively relative or absolute. "Detecting the presence of" includes determining the amount of something present, as well as determining whether it may be present or absent.

The terms "subject," "individual," or "patient" are often used interchangeably herein. A "subject" can be a biological entity. The biological entity can be an animal, a plant, or a microorganism. The subject can be tissues, cells and their progeny of a biological entity obtained *in vivo* or cultured *in vitro*. The subject can be a mammal. The mammal can be a human. The subject may be a child, an infant, or an adult. In some cases, the human is a male. The subject can be a male human of reproductive age (*e.g.*, older than 10 years of age). The subject can be about 1 day old to about 18 years old. In some cases, the subject can be about 1 day old to about 1 year old. In some cases, the subject can be older than 18 years of age. In some cases, the subject can be older than about 10 years, 30 years, 40 years, 50 years, 60 years, 70 year, 80 years or 90 years. The subject can be about 1, 5, 10, 15, 20, 25, 30, 35, 40, 45, 50, 55, 60, 65, 70, 75, 80, 85, 90, 95, 100, 105, 110, 115, 120, 125 or 130 years old. In some cases, the subject can be older than 60 or 65 years of age. The subject may be diagnosed or suspected of being at high risk for a disease. In some cases, the subject may not be necessarily diagnosed or suspected of being at high risk for the disease.

The term "*in vitro*" can be used to describe an event that takes place contained in a container for holding laboratory reagent such that it can be separated from the living biological source organism from which the material may be obtained. *In vitro* assays can encompass cell-based assays in which cells alive or dead are employed. *In vitro* assays can also encompass a cell-free assay in which no intact cells are employed.

The term "*in vivo*" can be used to describe an event that takes place in a subject's body.

The term "*ex vivo*" can be used to describe an event that takes place outside of a subject's body. An "*ex vivo*" assay may not be performed on a subject. Rather, it can be performed upon a sample separate from a subject. An example of an "*ex vivo*" assay performed on a sample can be an "*in vitro*" assay.

As used herein, the terms "treatment" or "treating" refers to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of one or more symptoms or of an underlying disorder being treated. For example, a therapeutic benefit can comprise treating a male reproductive disorder. Also, a therapeutic benefit can be achieved with the eradication or amelioration of one or more of the physiological symptoms associated with the underlying disorder such that an improvement may be observed in the subject, notwithstanding that the subject may still be afflicted with the underlying disorder. A prophylactic effect can include delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

As used herein, the terms "effective amount" or "therapeutically effective amount" of a drug used to treat a disease can be an amount that can reduce the severity of a disease, reduce the severity of one or more symptoms associated with the disease or its treatment, or delay the onset of more serious symptoms or a more serious disease that can occur with some frequency following the treated condition. An "effective amount" may be determined empirically and in a routine manner, in relation to the stated purpose.

As used herein, the term "unit dose" or "dosage form" can be used interchangeably and can be meant to refer to pharmaceutical drug products in the form in which they are marketed for use, with a specific mixture of active ingredients and inactive components or excipients, in a particular configuration, and apportioned into a particular dose to be delivered. The term "unit dose" can also sometimes refer to the particles comprising a pharmaceutical composition or therapy, and to any mixtures involved. Types of unit doses may vary with the route of administration for drug delivery, and the substance(s) being delivered. A solid unit dose can be the solid form of a dose of a chemical compound used as a pharmaceutically acceptable drug or medication intended for administration or consumption.

As used herein, "pharmaceutically acceptable salt" can refer to pharmaceutical drug molecules, which may be formed as a weak acid or base, chemically made into their salt forms, most frequently as the hydrochloride, sodium, or sulfate salts. Drug products synthesized as salts may enhance drug dissolution, boost absorption into the bloodstream, facilitate therapeutic effects, and increase its effectiveness. Pharmaceutically acceptable salts may also facilitate the development of controlled-release dosage forms, improve drug stability, extend shelf life, enhance targeted drug delivery, and improve drug effectiveness.

An "epimutation," or "epigenetic modification," as used herein generally can refer to modifications of cellular DNA that affect gene expression without altering the DNA sequence. The epigenetic modifications can be both mitotically and meiotically stable, for example, after the DNA in a cell (or cells) of an organism has been epigenetically modified, the pattern of modification can persist throughout the lifetime of the cell and can be passed to progeny cells via both mitosis and meiosis. Therefore, with the organism's lifetime, the pattern of DNA modification and consequences thereof, can remain consistent in the cells derived from the parental cell that was originally modified. Further, if the epigenetically modified cell undergoes meiosis to generate gametes (*e.g.* sperm), the pattern of epigenetic modification may be retained in the gametes and thus can be inherited by offspring. In other words, the patterns of epigenetic DNA modification are transgenerationally transmissible or inheritable, even though the DNA nucleotide sequence per se has not been altered or mutated. Exemplary epigenetic modifications include, but are not limited, to DNA methylation, histone modifications, chromatin structure modifications, and non-coding RNA modifications, etc. Further, the term "epigenetic modification" as used herein, may be any covalent modification of a nucleic acid base. In some cases, a covalent modification may comprise (i) adding a methyl group, a hydroxymethyl group, a carbon atom, an oxygen atom, or any combination thereof to one or more bases of a nucleic acid sequence, (ii) changing an oxidation state of a molecule associated with a nucleic acid sequence, such as an oxygen atom, or (iii) a combination thereof. A covalent modification may occur at any base, such as a cytosine, a thymine, a uracil, an adenine, a guanine, or any combination thereof. In some cases, an epigenetic modification may comprise an oxidation or a reduction. A nucleic acid sequence may comprise one or more epigenetically modified bases. An epigenetically modified base may comprise any base, such as a cytosine, a uracil, a thymine, adenine, or a guanine. An epigenetically modified base may comprise a methylated base, a hydroxymethylated base, a formylated base, or a carboxylic acid containing base or a salt thereof. An epigenetically modified base may comprise a 5-methylated base, such as a 5-methylated cytosine (5-mC). An epigenetically modified base may comprise a 5 -hydroxymethylated base, such as a 5 -hydroxymethylated cytosine (5-hmC). An epigenetically modified base may comprise a 5-formylated base, such as a 5-formylated cytosine (5-fC). An epigenetically modified base may comprise a 5-carboxylated base or a salt thereof, such as a 5-carboxylated cytosine (5-caC). In some cases, an epigenetically modified base may comprise a methyltransferase-directed transfer of an activated group (TAG). In some cases, DNA methylation is an epigenetic mechanism that occurs when a methyl group is added onto the C5 position of cytosine, thereby modifying gene function and affecting gene expression. Most DNA methylation occurs at cytosine residues that precede guanine residues, called CpG dinucleotides.

Epigenetic modifications may be caused by exposure to any of a variety of factors, examples of which include but are not limited to: chemical compounds *e.g.* endocrine disruptors such as vinclozolin; chemicals such as those used in the manufacture of plastics *e.g.* bispheol A (BPA); bis(2-ethylhexyl)phthalate (DEHP); dibutyl phthalate (DBP); insect repellants such as N, N-diethyl-meta-toluamide (DEET); pyrethroids such as permethrin; various polychlorinated dibenzodioxins, known as PCDDs or dioxins *e.g.* 2, 3,7,8- tetrachlorodibenzo-p-dioxin (TCDD); extreme conditions such as abnormal nutrition, starvation, chemotherapeutic agents which include alkylating agents such as ifosfamide and cyclophosphamide, anthracyclines such as daunorubicin and doxorubicine, taxanes such as paclitaxel and docetaxel, epothilones, histone deacetylase inhibitors, topoisomerase inhibitors, kinase inhibitors such as gefitinib, platinum-based agents such as cisplatin, retinoids, and vinca alkaloids, etc.

"Promoter dysregulation" or "a dysregulated promoter" can be used herein to refer to methylation variability amongst regions of a promoter that deviates from a control methylation variability level to an extent that is indicative of a dysregulated region. For example, the variability of methylation of a promoter may be different from a control variability of methylation of the same promoter. In another example, a control variability of methylation of a promoter may be different (*e. g.*, decreased) as compared to a sample that has is dysregulated in the same promoter. In some cases, a dysregulated promoter can be a promoter that is above a corresponding variability threshold. In some cases, a variability threshold can be 2, 3, 4, 5, 6, or more standard deviations from a control methylation variability level of an individual promoter. In some cases, a variability threshold can be: 3, greater than or equal to 3, or greater than 3 standard deviations from a control methylation variability level of an individual promoter. In some cases, a variability level (*e.g.*, variability value) can be a standard deviation of the methylation of an individual promoter, for example a control sample or reference sample's promoter. In some cases, the methylation of an individual promoter can be the average standard deviation of the methylation of 5 or more regions of a promoter. In some cases, the methylation of an individual promoter can be the average standard deviation of the methylation of 5, 6, 7, 8, 9, 10, 11, 12, 13, 14, 15, 16, 17, 18, 19, 20, 21, or 22 or more regions of a promoter. In some cases, a region of a promoter can be a region associated with a probe, for example a probe used to identify methylation. In some cases, a reference or control methylation variability level and/or variability threshold can be from a sample of a subject who is fertile. In some cases, a reference or control methylation variability level and/or variability threshold can be from a sample of a subject who has diminished fertility. In some embodiments, the methylation of an individual promoter is generated with a computer program executed on a computer. In some embodiments, the variability level of an individual promoter is generated with a computer program executed on a computer. In some embodiments, the variability threshold of an individual promoter is generated with a computer program executed on a computer. In some cases, detecting if a dysregulated promoter is above a variability threshold can be determined with a computer program executed on a computer. In some cases, the average standard deviation of methylation can be generated with a computer program executed on a computer.

In some cases, a reference variability level of methylation ( *e.g.*, a reference standard deviation of methylation) is an the variability of methylation in a region, for example a promoter region. In some cases, a reference variability level of methylation is a control variability level of methylation. In some cases, a control variability level of methylation is a reference variability level of methylation. In some cases, a reference or control variability level of methylation can be obtained from a diseased individual or a group of diseased individuals such as a group of individuals who are infertile. In some cases, a reference or control variability level of methylation can be obtained can be obtained from a healthy individual or a group of healthy individuals without a disease or condition, such as a group of individuals who are fertile. In some cases, a variability threshold can be obtained from variability level from a reference or control. In some cases, a variability threshold can be 3, or greater than 3 more standard deviations from a variability level.

As used herein, the term "reference sequence", can refer to a known nucleotide sequence, *e.g.* a chromosomal region whose sequence is deposited at NCBI's Genbank database or other databases. A reference sequence can be a wild type sequence.

A "promoter" as used herein is the genomic region one kilobase upstream and/or one kilobase downstream from the transcription start site of a given gene, for example a promoter of **Table 1** is shown as one kilobase upstream and one kilobase downstream from the transcription start site of the gene indicated on **Table 1.**

The term "nucleic acid" and "polynucleotide" can be used interchangeably herein to describe a polymer of any length, *e.g.*, greater than about 2 bases, greater than about 10 bases, greater than about 100 bases, greater than about 500 bases, greater than 1000 bases, up to about 10,000 or more bases composed of nucleotides, *e.g.*, deoxyribonucleotides or ribonucleotides, and may be produced enzymatically or synthetically (*e.g.*, peptide nucleic acid (PNA)) which can hybridize with naturally occurring nucleic acids in a sequence specific manner analogous to that of two naturally occurring nucleic acids, *e.g.*, can participate in Watson-Crick base pairing interactions. Naturally-occurring nucleotides can include guanine, cytosine, adenine, uracil and thymine (G, C, A, U and T, respectively). In some cases, a nucleic acid can be single stranded. In some cases, a nucleic acid can be double stranded. In some cases, a nucleic acid can comprise a ribonucleic acid (RNA), deoxyribonucleic acid (DNA), or both. In some cases, a polynucleotide may have a modified base.

"Homology" or "identity" or "similarity" can refer to sequence similarity between two peptides or between two nucleic acid molecules. Homology can be determined by comparing a position in each sequence which can be aligned for purposes of comparison. When a position in the compared sequence can be occupied by the same base or amino acid, then the molecules can be homologous at that position. A degree of homology between sequences can be a function of the number of matching or homologous positions shared by the sequences. An "unrelated" or "non-homologous" sequence shares less than 40% identity, or alternatively less than 25% identity, with one of the sequences of the disclosure. Sequence homology can refer to a % identity of a sequence to a reference sequence. As a practical matter, whether any particular sequence can be at least 50%, 60%, 70%, 80%, 85%, 90%, 92%, 95%, 96%, 97%, 98% or 99% identical to any sequence described herein (which can correspond with a particular nucleic acid sequence described herein), such particular polypeptide sequence can be determined conventionally using known computer programs such the Bestfit program (Wisconsin Sequence Analysis Package, Version 8 for Unix, Genetics Computer Group, University Research Park, 575 Science Drive, Madison, Wis. 53711). When using Bestfit or any other sequence alignment program to determine whether a particular sequence is, for instance, 95% identical to a reference sequence, the parameters can be set such that the percentage of identity can be calculated over the full- length of the reference sequence and that gaps in sequence homology of up to 5% of the total reference sequence can be allowed. In some cases, any sequence disclosed herein can also comprise sequences with at least about: 70%, 70%, 80%, 85%, 90%, 92%, 95%, 96%, 97%, 98% or 99% sequence identity to the disclosed sequence.

In some cases, the identity between a reference sequence (query sequence) and a subject sequence, also referred to as a global sequence alignment, can be determined using the FASTDB computer program-based on the algorithm of Brutlag et al. (Comp. App. Biosci. 6:237-245 (1990)). In some embodiments, parameters for a particular embodiment in which identity can be narrowly construed, used in a FASTDB amino acid alignment, can include: Scoring Scheme=PAM (Percent Accepted Mutations) 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject sequence, whichever can be shorter. According to this embodiment, if the subject sequence can be shorter than the query sequence due to N- or C-terminal deletions, not because of internal deletions, a manual correction can be made to the results to take into consideration the fact that the FASTDB program does not account for N- and C-terminal truncations of the subject sequence when calculating global percent identity. For subject sequences truncated at the N- and C-termini, relative to the query sequence, the percent identity can be corrected by calculating the number of residues of the query sequence that can be lateral to the N- and C-terminal of the subject sequence, which can be not matched/aligned with a corresponding subject residue, as a percent of the total bases of the query sequence. A determination of whether a residue can be matched/aligned can be determined by results of the FASTDB sequence alignment. This percentage can be then subtracted from the percent identity, calculated by the FASTDB program using the specified parameters, to arrive at a final percent identity score. This final percent identity score can be used for the purposes of this embodiment. In some cases, only residues to the N- and C-termini of the subject sequence, which can be not matched/aligned with the query sequence, can be considered for the purposes of manually adjusting the percent identity score. That is, only query residue positions outside the farthest N- and C-terminal residues of the subject sequence can be considered for this manual correction. For example, a 90-residue subject sequence can be aligned with a 100-residue query sequence to determine percent identity. The deletion occurs at the N-terminus of the subject sequence, and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% can be subtracted from the percent identity score calculated by the FASTDB program. If the remaining 90 residues were perfectly matched, the final percent identity can be 90%. In another example, a 90-residue subject sequence can be compared with a 100-residue query sequence. This time the deletions can be internal deletions, so there can be no residues at the N- or C-termini of the subject sequence which can be not matched/aligned with the query. In this case, the percent identity calculated by FASTDB can be not manually corrected. Once again, only residue positions outside the N- and C-terminal ends of the subject sequence, as displayed in the FASTDB alignment, which can be not matched/aligned with the query sequence can be manually corrected for.

The section headings used herein are for organizational purposes only and are not to be construed as limiting the subject matter described.

**Overview**

A better diagnosis is needed for male infertility. In some cases, the mainstay of male infertility diagnosis is the standard semen analysis (*e.g.*, sperm concentration, motility, and morphology). In some instances, a small number of studies have sought to evaluate the prognostic value of the parameters evaluated by the standard semen analysis and have shown the predictive value of the semen analysis for fertility is modest at best, with the exception of severely diminished sperm concentration or motility. In some cases, current semen analysis only has a 14.8% sensitivity in diagnosing male infertility. In cases where there is an absence of female infertility factors and standard semen analysis parameters fall within normal ranges, undiagnosed male infertility may be the missing piece. In some cases, the methylation of sperm DNA can provide information on male infertility, thus provide medical professionals with the information necessary to develop a successful treatment plan.

Disclosed herein is methods and systems for determining the methylation variability (*e.g.*, standard deviation for methylation) for an individual promoter and determining if a sample is dysregulated based on increased methylation variability at one or more individual promoters as compared to a control variability levels. As the number of dysregulated promoters increases in a sample of a subject, the subject may have an increased likelihood of infertility. The methods, systems, and kits described herein can be used in detecting infertility or diminished infertility in subjects.

The methods, systems, and kits described herein can be used with methods of treatment for fertility related disease. The methods herein can be used to guide clinical care for multiple types of male infertility, which currently lack diagnostic tests. The methods, systems, and kits described herein can be used with one or more computer processors, computer memories, and computer programs to implement the steps described herein for identifying dysregulated promoters of sperm DNA.

**Methods of Identifying Methylated Dysregulated Promoters of Sperm**

Disclosed herein are methods and systems of identifying dysregulated promoters of a sperm or a semen sample. In some embodiments, the methods disclosed herein can be a method of detecting diminished fertility or infertility of a male subject. In some embodiments, the methods disclosed herein can be a method of detecting fertility of a male subject. In some instances, a method can comprise: a) obtaining a biological sample from a male subject, wherein the biological sample comprises seminal fluid; b) extracting DNA from a sperm in the biological sample, extracting cell free DNA from the biological sample, or both; and c) detecting in an *in vitro* analytical assay, methylation present in at least 5 regions of an individual promoter comprised in the extracted DNA, the extracted cell free DNA, or both from the biological sample. In some cases, the promoter is selected from **Table** 1. In some cases, the method can comprise determining if an average standard deviation of the at least 5 regions of the individual promoter is greater than, or greater than or equal to three standard deviations from a reference average standard deviation of the at least 5 regions of the individual promoter. In some cases, the method can comprise determining independently a standard deviation for methylation in each of the at least 5 regions of the individual promoter. In some cases, the method can comprise calculating an average standard deviation for methylation of the individual promoter from the standard deviation of methylation in each of the at least 5 regions of the individual promoter.

In some cases, the methods can comprise: obtaining a biological sample from a male subject. In some instances, the biological sample comprises seminal fluid. In some embodiments, a method can comprise extracting DNA from a sperm in the biological sample, extracting cell free DNA from the biological sample, or both. In some cases, extracting DNA can be completed with the use of a DNA extraction kit. In some cases, extracting DNA can be completed with the use of an ionic detergent, sodium dodecyl sulfate (SDS), proteinase K, dithiothreitol (DTT), 2-mercaptoethanol (βME) or any combination thereof. In some cases DNA can be isolated by a silica-based spin column or by ethanol precipitation.

In some embodiments, a method or system can comprise detecting in an *in vitro* analytical assay, methylation present in at least 5 regions of an individual promoter comprised in the extracted DNA, the extracted cell free DNA, or both from the biological sample. In some cases, an *in vitro* analytical assay can comprise sodium bisulfite conversion, a sequencing, a differential enzymatic cleavage of DNA, an affinity capture of methylated DNA, an array, or any combination thereof. In some cases, a method or system for detecting DNA methylation can comprise a microarray. In some cases, a method or system for detecting DNA methylation can comprise a methylated DNA immunoprecipitation (MeDIP), a sequencing, a bisulfite treatment, a bisulfite conversion, a deamination of an unmethylated cytosine base, employing an array, or any combination of these. In some instances, MeDIP can be used to isolate methylated DNA from a sample. In some cases, a method or system for detecting DNA methylation can comprise amplification of an isolated fragmented methylated DNA, sequencing the isolated fragmented methylated DNA, an amplicon thereof, or both, employing an array (*e.g.* a microarray), or any combination of these. In some cases, a method or system for detecting DNA methylation can comprise target enrichment and sequencing using one or more probes and/or primers. In some cases, detecting can employ a computer processor. In some cases, the detecting can employ a computer processor operably connected to a computer memory. In some cases, the detecting can employ a computer program executed on a computer.

In some cases, the method or system can comprise detecting methylation of a promoter. In some cases, the promoter can be selected from **Table 1. Table 1** shows 1) the promoter region with respect to genes as annotated in the Reference genome HG19; 2) the region designation, in reference to reference genome HG19, indicates the chromosome number (*e.g.* chr22) and the start and stop location on the chromosome of the promoter region (*e.g.*, 42295947-42297947); 3) the number of methylation probes per region; and 4) the 3X standard deviation cutoff value (*e.g.*, reference variability threshold value). The region designation is in reference to the reference genome is HG19 (GenBank assembly accession: GCA_000001405.1). The National Center for Biotechnology Information (NCBI) description for the genome is provided in **Table 6.**

**Table 1: Sperm Promoters with Low Methylation Variability**

| Promoter Region | Region Designation | Number of Probes in Region | Standard Deviation Cutoff |
|---|---|---|---|
| MIR33A | chr22:42295947-42297947 | 6 | 0.3815 |
| TRAF3IP3 | chr1:209928397-209930397 | 7 | 0.4213 |
| RTP3 | chr3:46538465-46540465 | 6 | 0.4360 |
| SCOC | chr4:141177461-141179461 | 5 | 0.4367 |
| PARVG | chr22:44567835-44569835 | 10 | 0.4433 |
| CST8 | chr20:23470753-23472753 | 7 | 0.4442 |
| CARMN | chr5:148785407-148787407 | 6 | 0.4445 |
| GPD1 | chr12:50496790-50498790 | 6 | 0.4677 |
| PLPP2 | chr19:280042-282042 | 6 | 0.4679 |
| MCHR1 | chr22:41074181-41076181 | 13 | 0.4751 |
| ADGRG3 | chr16:57701217-57703217 | 8 | 0.4768 |
| CCDC33 | chr15:74527666-74529666 | 5 | 0.4866 |
| C16orf78 | chr16:49406714-49408714 | 6 | 0.4891 |
| NCR2 | chr6:41302345-41304345 | 7 | 0.4911 |

(continued)

| Promoter Region | Region Designation | Number of Probes in Region | Standard Deviation Cutoff |
|---|---|---|---|
| EVPLL | chr17:18280091-18282091 | 5 | 0.4913 |
| LINC00488 | chr3:108896011-108898011 | 5 | 0.4938 |
| CACNA1C-IT2 | chr12:2156517-2158517 | 7 | 0.4970 |
| HAP1 | chr17:39872990-39874990 | 5 | 0.5014 |
| C10orf99 | chr10:85932556-85934556 | 6 | 0.5022 |
| LOC388282 | chr16:57843548-57845548 | 9 | 0.5062 |
| TAS2R5 | chr7:141489016-141491016 | 6 | 0.5097 |
| SMTNL1 | chr11:57309113-57311113 | 6 | 0.5107 |
| C17orf99 | chr17:76141465-76143465 | 5 | 0.5119 |
| PRM1 | chr16:11373697-11375697 | 9 | 0.5119 |
| IGSF23 | chr19:45115872-45117872 | 11 | 0.5158 |
| SASH3 | chrX:128912924-128914924 | 10 | 0.5166 |
| LOC284950 | chr2:86041252-86043252 | 5 | 0.5176 |
| ACACB | chr12:109553391-109555391 | 8 | 0.5180 |
| LINC01433 | chr20:4172736-4174736 | 5 | 0.5202 |
| DRAIC | chr15:69853058-69855058 | 6 | 0.5219 |
| OR10V1 | chr11:59479388-59481388 | 6 | 0.5259 |
| AXL | chr19:41724127-41726127 | 9 | 0.5259 |
| UBASH3A | chr21:43823010-43825010 | 10 | 0.5305 |
| FAM110D | chr1:26484569-26486569 | 5 | 0.5310 |
| SIGLEC7 | chr19:51644557-51646557 | 6 | 0.5315 |
| NKX2-6 | chr8:23558963-23560963 | 5 | 0.5325 |
| SPATA3 | chr2:231859838-231861838 | 7 | 0.5359 |
| SNORD115-20 | chr15:25450408-25452408 | 5 | 0.5376 |
| TSPAN16 | chr19:11405835-11407835 | 5 | 0.5383 |
| BPIFB6 | chr20:31618453-31620453 | 6 | 0.5399 |
| NR2E3 | chr15:72101893-72103893 | 9 | 0.5402 |
| SNORA71C | chr20:37057309-37059309 | 6 | 0.5409 |
| RNF186 | chr1:20139521-20141521 | 5 | 0.5436 |
| NUDT14 | chr14:105638275-105640275 | 8 | 0.5438 |
| CCR4 | chr3:32992135-32994135 | 6 | 0.5484 |
| CACNAL1I | chr22:39965757-39967757 | 7 | 0.5486 |
| HSD17B7 | chr1:162759491-162761491 | 5 | 0.5487 |
| RHBDD3 | chr22:29654840-29656840 | 5 | 0.5517 |
| FGF21 | chr19:49257780-49259780 | 9 | 0.5535 |
| URI1 | chr19:30413563-30415563 | 5 | 0.5542 |
| AOC3 | chr17:41002200-41004200 | 7 | 0.5543 |
| PLD4 | chr14:105390215-105392215 | 7 | 0.5549 |
| SNORD114-12 | chr14:101434284-101436284 | 6 | 0.5552 |

(continued)

| Promoter Region | Region Designation | Number of Probes in Region | Standard Deviation Cutoff |
|---|---|---|---|
| CSF2 | chr5:131408481-131410481 | 6 | 0.5554 |
| FAM167A-AS1 | chr8:11224910-11226910 | 6 | 0.5566 |
| SNORD116-24 | chr15:25338182-25340182 | 6 | 0.5576 |
| PABPC4-AS1 | chr1:40029741-40031741 | 8 | 0.5581 |
| IL17C | chr16:88703979-88705979 | 7 | 0.5588 |
| LINC02408 | chr12:67912861-67914861 | 6 | 0.5589 |
| LY6D | chr8:143865297-143867297 | 6 | 0.5591 |
| MIR1301 | chr2:25550508-25552508 | 6 | 0.5592 |
| LINC02153 | chr8:20830496-20832496 | 7 | 0.5601 |
| IFI27 | chr14:94576081-94578081 | 9 | 0.5603 |
| MIR422A | chr15:64162128-64164128 | 6 | 0.5608 |
| RRAD | chr16:66954581-66956581 | 6 | 0.5630 |
| UBOX5 | chr20:3087218-3089218 | 5 | 0.5642 |
| PRC1-AS1 | chr15:91508598-91510598 | 5 | 0.5645 |
| SNHG12 | chr1:28904049-28906049 | 5 | 0.5658 |
| SERPINE3 | chr13:51914167-51916167 | 6 | 0.5658 |
| ACSM4 | chr12:7455927-7457927 | 5 | 0.5666 |
| FLJ45513 | chr17:47922247-47924247 | 6 | 0.5668 |
| AUP1 | chr2:74752778-74754778 | 9 | 0.5670 |
| LINC00851 | chr20:18358692-18360692 | 5 | 0.5674 |
| SLC22A7 | chr6:43264997-43266997 | 5 | 0.5681 |
| MGC16025 | chr2:240114026-240116026 | 6 | 0.5682 |
| LINC01656 | chr22:44838206-44840206 | 5 | 0.5683 |
| MYOZ2 | chr4:120055938-120057938 | 5 | 0.5686 |
| GUSBP1 | chr5:21458588-21460588 | 7 | 0.5687 |
| C14orf180 | chr14:105045100-105047100 | 11 | 0.5696 |
| ACTG2 | chr2:74119134-74121134 | 10 | 0.5706 |
| SNORD116-2 | chr15:25298355-25300355 | 6 | 0.5718 |
| MIR2116 | chr15:59462381-59464381 | 5 | 0.5726 |
| SIRT2 | chr19:39368194-39370194 | 9 | 0.5729 |
| LINC00996 | chr7:150129741-150131741 | 5 | 0.5746 |
| ITIH1 | chr3:52810614-52812614 | 10 | 0.5755 |
| LCA10 | chrX:153145126-153147126 | 5 | 0.5772 |
| PRAM1 | chr19:8553939-8555939 | 6 | 0.5786 |
| SLC34A1 | chr5:176810434-176812434 | 8 | 0.5793 |
| MIR646 | chr20:58882531-58884531 | 6 | 0.5820 |
| TOGARAM2 | chr2:29203163-29205163 | 7 | 0.5829 |
| MIR151B | chr14:100574755-100576755 | 5 | 0.5839 |
| NCRNA00250 | chr8:135849311-135851311 | 5 | 0.5839 |

(continued)

| Promoter Region | Region Designation | Number of Probes in Region | Standard Deviation Cutoff |
|---|---|---:|---:|
| KCNE2 | chr21:35735322-35737322 | 5 | 0.5845 |
| MIR1286 | chr22:20235656-20237656 | 7 | 0.5850 |
| MIR940 | chr16:2320747-2322747 | 5 | 0.5866 |
| SGK2 | chr20:42186666-42188666 | 8 | 0.5872 |
| APOC2 | chr19:45448307-45450307 | 11 | 0.5876 |
| MIR1287 | chr10:100153974-100155974 | 5 | 0.5882 |
| CETP | chr16:56994861-56996861 | 5 | 0.5886 |
| S100A3 | chr1:153518804-153520804 | 6 | 0.5909 |
| ZNF185 | chrX:152081985-152083985 | 5 | 0.5918 |
| GPR35 | chr2:241543846-241545846 | 5 | 0.5919 |
| MYO16 | chr13:109247252-109249252 | 6 | 0.5919 |
| UBA5 | chr3:132372289-132374289 | 5 | 0.5936 |
| NEURL1-AS1 | chr10:105238359-105240359 | 10 | 0.5942 |
| TNP1 | chr2:217723180-217725180 | 6 | 0.5961 |
| MIR483 | chr11:2154363-2156363 | 7 | 0.5977 |
| SPRR4 | chr1:1562942123-152944123 | 7 | 0.5977 |
| SPNS3 | chr17:4336234-4338234 | 11 | 0.5984 |
| C2orf92 | chr2:98285205-98287205 | 5 | 0.5992 |
| MSH2-OT1 | chr2:47753675-47755675 | 7 | 0.6009 |
| TULP4 | chr6:158732496-158734496 | 9 | 0.6019 |
| SNORA5C | chr7:45143504-45145504 | 15 | 0.6025 |
| CARD 11 | chr7:2944708-2946708 | 5 | 0.6031 |
| KLKB1 | chr4:187147660-187149660 | 7 | 0.6031 |
| TUB | chr11:8059179-8061179 | 8 | 0.6036 |
| ZMIZ1 | chr10:80827722-80829722 | 11 | 0.6053 |
| MIR1250 | chr17:79105995-79107995 | 7 | 0.6063 |
| MUC5AC | chr11:1150579-1152579 | 6 | 0.6073 |
| EGF | chr4:110833038-110835038 | 7 | 0.6099 |
| AGXT | chr2:241807240-241809240 | 11 | 0.6101 |
| LOC105373609 | chr2:128316619-128318619 | 5 | 0.6103 |
| SNORA5A | chr7:45142947-45144947 | 13 | 0.6104 |
| LOC100506551 | chr12:117414244-117416244 | 5 | 0.6111 |
| LDB3 | chr10:88425544-88427544 | 6 | 0.6114 |
| LOC100130283 | chr16:8942325-8944325 | 8 | 0.6119 |
| MIR1275 | chr6:33966748-33968748 | 5 | 0.6121 |
| ACOX3 | chr4:8367008-8369008 | 5 | 0.6122 |
| IGFLR1 | chr19:36228701-36230701 | 6 | 0.6126 |
| SIX5 | chr19:46267042-46269042 | 7 | 0.6130 |
| NUDT8 | chr11:67394408-67396408 | 6 | 0.6131 |

(continued)

| Promoter Region | Region Designation | Number of Probes in Region | Standard Deviation Cutoff |
|---|---|---|---|
| SLC14A1 | chr18:43303145-43305145 | 6 | 0.6146 |
| MIR7160 | chr8:2023668-2025668 | 6 | 0.6176 |
| DNAH1 | chr3:52349334-52351334 | 5 | 0.6178 |
| DAPP1 | chr4:100736983-100738983 | 7 | 0.6182 |
| MIR6840 | chr7:99953273-99955273 | 10 | 0.6191 |
| DCUN1D2 | chr13:114109133-114111133 | 8 | 0.6193 |
| SPINK7 | chr5:147690985-147692985 | 7 | 0.6193 |
| GGT1 | chr22:24978717-24980717 | 9 | 0.6199 |
| CLCA1 | chr1:86933609-86935609 | 6 | 0.6202 |
| MIR187 | chr18:33483780-33485780 | 7 | 0.6213 |
| TEX45 | chr19:7561433-7563433 | 7 | 0.6222 |
| FAM151A | chr1:55073853-55075853 | 6 | 0.6236 |
| KCNU1 | chr8:36640891-36642891 | 10 | 0.6244 |
| DOCK9-AS1 | chr13:99483337-99485337 | 5 | 0.6248 |
| GJA10 | chr6:90603187-90605187 | 6 | 0.6260 |
| SNORD115-18 | chr15:25447373-25449373 | 5 | 0.6286 |
| MIR205 | chr1:209604477-209606477 | 5 | 0.6287 |
| NRAD1 | chr13:44595470-44597470 | 5 | 0.6293 |
| LOC104613533 | chr15:93012462-93014462 | 5 | 0.6300 |
| NEUROD2 | chr17:37759020-37761020 | 5 | 0.6312 |
| ODF1 | chr8:103562816-103564816 | 7 | 0.6318 |
| LINC01805 | chr2:64712486-64714486 | 6 | 0.6321 |
| MLIP | chr6:53882713-53884713 | 6 | 0.6323 |
| DRP2 | chrX:100473932-100475932 | 6 | 0.6333 |
| SNORD36A | chr9:136216310-136218310 | 6 | 0.6337 |
| SNORD36C | chr9:136216700-136218700 | 6 | 0.6337 |
| ITGB2 | chr21:46304867-46306867 | 5 | 0.6340 |
| ACR | chr22:51175631-51177631 | 8 | 0.6345 |
| DNASE1 | chr16:3701939-3703939 | 9 | 0.6357 |
| WFDC13 | chr20:44329676-44331676 | 6 | 0.6369 |
| LOC100506175 | chr20:49261007-49263007 | 5 | 0.6369 |
| SNORD125 | chr22:29728151-29730151 | 5 | 0.6370 |
| SCARNA4 | chr1:155894748-155896748 | 5 | 0.6373 |
| RGL4 | chr22:24032047-24034047 | 6 | 0.6379 |
| FGF7 | chr15:49714438-49716438 | 6 | 0.6383 |
| GRAMD2B | chr5:125694811-125696811 | 7 | 0.6383 |
| C1QB | chr1:22978728-22980728 | 7 | 0.6388 |
| CD19 | chr16:28942291-28944291 | 10 | 0.6392 |
| ACTR5 | chr20:37376102-37378102 | 12 | 0.6394 |

(continued)

| Promoter Region | Region Designation | Number of Probes in Region | Standard Deviation Cutoff |
|---|---|---|---|
| CA14 | chr1:150229168-150231168 | 9 | 0.6397 |
| KLHL25 | chr15:86301556-86303556 | 5 | 0.6398 |
| SERPINB13 | chr18:61253533-61255533 | 6 | 0.6400 |
| HKDC1 | chr10:70979087-70981087 | 6 | 0.6405 |
| ELF3 | chr1:201978714-201980714 | 13 | 0.6410 |
| SNORD116-3 | chr15:2530 1005-25303005 | 5 | 0.6415 |
| TRPC2 | chr11:3646689-3648689 | 7 | 0.6417 |
| LY6G6FLY6G6D | chr6:31673642-31675642 | 7 | 0.6426 |
| LY6G6F | chr6:31673642-31675642 | 7 | 0.6426 |
| C8orf31 | chr8:144119625-144121625 | 9 | 0.6428 |
| SNORD114-13 | chr14:101435215-101437215 | 5 | 0.6433 |
| EPX | chr17:56269086-56271086 | 8 | 0.6434 |
| C15orf32 | chr15:93013906-93015906 | 6 | 0.6434 |
| KCCAT333 | chr7:17413540-17415540 | 5 | 0.6435 |
| ZBTB25 | chr14:64914823-64916823 | 6 | 0.6441 |
| CLCN1 | chr7:143012203-143014203 | 7 | 0.6458 |
| IL24 | chr1:207069787-207071787 | 7 | 0.6459 |
| GAMT | chr19: 1396024-1398024 | 5 | 0.6462 |
| EFCAB8 | chr20:31445728-31447728 | 7 | 0.6465 |
| LINC02520 | chr6:37474123-37476123 | 5 | 0.6470 |
| MIOX | chr22:50924287-50926287 | 10 | 0.6473 |
| LINC01517 | chr10:29031578-29033578 | 5 | 0.6474 |
| CNTFR-AS1 | chr9:34567009-34569009 | 5 | 0.6474 |
| APOBR | chr16:28504963-28506963 | 7 | 0.6475 |
| LOC100506098 | chr7:20256199-20258199 | 7 | 0.6475 |
| LINC02499 | chr4:74373519-74375519 | 6 | 0.6479 |
| LCE5A | chr1:152482278-152484278 | 7 | 0.6482 |
| RAB44 | chr6:36664602-36666602 | 7 | 0.6486 |
| SLC25A34 | chr1:16061752-16063752 | 12 | 0.6488 |
| TBC1D2B | chr15:78286326-78288326 | 7 | 0.6492 |
| SPACA4 | chr19:49108998-49110998 | 13 | 0.6493 |
| ESS2 | chr22:19116791-19118791 | 9 | 0.6498 |
| LINC02694 | chr15:38987798-38989798 | 7 | 0.6504 |
| LOC101928012 | chr7:112261435-112263435 | 5 | 0.6505 |
| TJP3 | chr19:3707381-3709381 | 13 | 0.6527 |
| LINC00618 | chr14:97408915-97410915 | 5 | 0.6554 |
| FLRT1 | chr11:63870361-63872361 | 7 | 0.6558 |
| SPINK13 | chr5:147647356-147649356 | 5 | 0.6566 |
| ATP11AUN | chr13:113300357-113302357 | 6 | 0.6591 |

(continued)

| Promoter Region | Region Designation | Number of Probes in Region | Standard Deviation Cutoff |
|---|---|---|---|
| DIAPH3-AS1 | chr13:60585851-60587851 | 8 | 0.6591 |
| PEX10 | chr1:2335240-2337240 | 9 | 0.6595 |
| AQP6 | chr12:50365729-50367729 | 8 | 0.6595 |
| HOGA1 | chr10:99343101-99345101 | 8 | 0.6598 |
| FMO6P | chr1:171105878-171107878 | 5 | 0.6600 |
| LINC00852 | chr3:10325102-10327102 | 6 | 0.6601 |
| HRG | chr3:186382802-186384802 | 5 | 0.6618 |
| GABBR1 | chr6:29569004-29571004 | 5 | 0.6621 |
| LINC01732 | chr1:181142619-181144619 | 6 | 0.6629 |
| NPPA-AS1 | chr1:11899375-11901375 | 6 | 0.6630 |
| ATP1A2 | chr1:160084548-160086548 | 6 | 0.6636 |
| CDH5 | chr16:66399593-66401593 | 10 | 0.6639 |
| LCE6A | chr1:152814331-152816331 | 7 | 0.6644 |
| GPR107 | chr9:132814984-132816984 | 13 | 0.6649 |
| DCTN1-AS1 | chr2:74611629-74613629 | 8 | 0.6660 |
| MIR7515 | chr2:6789504-6791504 | 9 | 0.6664 |
| PLEKHM1 | chr17:43512265-43514265 | 5 | 0.6668 |
| MIR6816 | chr22:20101208-20103208 | 6 | 0.6669 |
| LINC02172 | chr4:138465884-138467884 | 5 | 0.6671 |
| LINC01975 | chr17:3879396-3881396 | 6 | 0.6672 |
| DTHD1 | chr4:36282237-36284237 | 8 | 0.6676 |
| SMIM25 | chr20:48883022-48885022 | 8 | 0.6684 |
| ZFP92 | chrX:152682780-152684780 | 8 | 0.6701 |
| ERLNC1 | chr1:204109535-204111535 | 9 | 0.6713 |
| RBMS3 | chr3:29321561-29323561 | 11 | 0.6721 |
| KSR2 | chr12:117889816-117891816 | 8 | 0.6728 |
| PPP1CA | chr11:67164653-67166653 | 6 | 0.6733 |
| LINC01690 | chr21:34330195-34332195 | 5 | 0.6734 |
| TMEM179 | chr14:105056200-105058200 | 5 | 0.6737 |
| LOC101929237 | chr8:22734484-22736484 | 7 | 0.6738 |
| PDCL3 | chr2:101178454-101180454 | 14 | 0.6741 |
| SLC5A11 | chr16:24856183-24858183 | 14 | 0.6755 |
| NELFE | chr6:31918863-31920863 | 8 | 0.6759 |
| NLRP3 | chr1:247578457-247580457 | 12 | 0.6762 |
| DENND6B | chr22:50746458-50748458 | 7 | 0.6765 |
| PAPOLG | chr2:60982402-60984402 | 9 | 0.6778 |
| LOC339862 | chr3:18003043-18005043 | 5 | 0.6782 |
| IL1R2 | chr2:102607421-102609421 | 6 | 0.6796 |
| SCARNA27 | chr6:8085640-8087640 | 6 | 0.6798 |

(continued)

| Promoter Region | Region Designation | Number of Probes in Region | Standard Deviation Cutoff |
|---|---|---|---|
| MIR8055 | chr8:6478644-6480644 | 8 | 0.6798 |
| PSMB8 | chr6:32807493-32809493 | 15 | 0.6806 |
| LOC90246 | chr3:128225677-128227677 | 6 | 0.6806 |
| CST13P | chr20:23498782-23500782 | 6 | 0.6814 |
| FLICR | chrX:49121682-49123682 | 5 | 0.6815 |
| LINC00311 | chr16:85315563-85317563 | 8 | 0.6815 |
| SNORD170 | chr5:14463091-14465091 | 5 | 0.6819 |
| MIR3196 | chr20:61869130-61871130 | 6 | 0.6825 |
| C1QC | chr1:22969125-22971125 | 5 | 0.6830 |
| TEK | chr9:27108138-27110138 | 7 | 0.6832 |
| NIBAN3 | chr19:17633109-17635109 | 8 | 0.6838 |
| LINC01875 | chr2:544804-546804 | 5 | 0.6840 |
| ACRBP | chr12:6746240-6748240 | 5 | 0.6843 |
| IL36G | chr2:113734582-113736582 | 8 | 0.6848 |
| MIR190A | chr15:63115155-63117155 | 9 | 0.6855 |
| DPRX | chr19:54134309-54136309 | 9 | 0.6860 |
| MIR150 | chr19:50003041-50005041 | 5 | 0.6861 |
| PDZK1 | chr1:145726665-145728665 | 8 | 0.6864 |
| PROK1 | chr1:110992770-110994770 | 8 | 0.6864 |
| LOC100506274 | chr2:7560391-7562391 | 5 | 0.6868 |
| PLB1 | chr2:28717926-28719926 | 7 | 0.6869 |
| IL1RL1 | chr2:102926961-102928961 | 5 | 0.6881 |
| LINC02217 | chr5:17403127-17405127 | 6 | 0.6883 |
| MDC1-AS1 | chr6:30669843-30671843 | 5 | 0.6885 |
| C6orf47 | chr6:31625074-31627074 | 10 | 0.6888 |
| MYLK2 | chr20:30406158-30408158 | 12 | 0.6890 |
| MIR451A | chr17:27187386-27189386 | 5 | 0.6892 |
| MIR451B | chr17:27187388-27189388 | 5 | 0.6892 |
| MIR144 | chr17:27187550-27189550 | 5 | 0.6892 |
| MIR4732 | chr17:27187672-27189672 | 5 | 0.6892 |
| CLIP4 | chr2:29319541-29321541 | 11 | 0.6892 |
| RPL13AP17 | chr7:77975558-77977558 | 5 | 0.6894 |
| GTSF1L | chr20:42353803-42355803 | 6 | 0.6903 |
| EMC9 | chr14:24607173-24609173 | 6 | 0.6905 |
| BPIFB1 | chr20:31870019-31872019 | 10 | 0.6910 |
| NNMT | chr11:114127527-114129527 | 6 | 0.6918 |
| MIR1976 | chr1:26880032-26882032 | 12 | 0.6923 |
| IQCJ | chr3:158786040-158788040 | 9 | 0.6924 |
| IQCJ-SCHIP1 | chr3:158786040-158788040 | 9 | 0.6924 |

(continued)

| Promoter Region | Region Designation | Number of Probes in Region | Standard Deviation Cutoff |
|---|---|---|---|
| STRIP2 | chr7:129073272-129075272 | 9 | 0.6940 |
| KCNIP1 | chr5:169779490-169781490 | 11 | 0.6949 |
| MIR25 | chr7:99690182-99692182 | 16 | 0.6950 |
| MIR106B | chr7:99690615-99692615 | 16 | 0.6950 |
| MIR93 | chr7:99690390-99692390 | 16 | 0.6950 |
| TMEM239 | chr20:2795947-2797947 | 9 | 0.6955 |
| MIR765 | chr1:156904922-156906922 | 5 | 0.6957 |
| SLC22A20P | chr11:64980310-64982310 | 9 | 0.6962 |
| LPIN1 | chr2:11816669-11818669 | 9 | 0.6964 |
| LINC00670 | chr17:12452284-12454284 | 6 | 0.6965 |
| ARID1A | chr1:27021505-27023505 | 7 | 0.6966 |
| OR52W1 | chr11:6219453-6221453 | 6 | 0.6968 |
| MIR943 | chr4:1987110-1989110 | 10 | 0.6968 |
| UMODL1 | chr21:43481985-43483985 | 8 | 0.6980 |
| SMIM6 | chr17:73641330-73643330 | 12 | 0.6990 |
| SLC19A1 | chr21:46931478-46933478 | 5 | 0.6991 |
| MIR148A | chr7:25988538-25990538 | 6 | 0.6994 |
| UBD | chr6:29522291-29524291 | 9 | 0.7000 |
| KRT13 | chr17:39656232-39658232 | 5 | 0.7004 |
| SLC51B | chr15:65336724-65338724 | 5 | 0.7007 |
| ADIPOQ | chr3:186559498-186561498 | 5 | 0.7008 |
| CES4A | chr16:67021491-67023491 | 8 | 0.7009 |
| KRTAP19-5 | chr21:31872974-31874974 | 5 | 0.7021 |
| RARRES2 | chr7:150034417-150036417 | 5 | 0.7023 |
| CDK15 | chr2:202670151-202672151 | 5 | 0.7024 |
| MIR379 | chr14:101487402-101489402 | 9 | 0.7027 |
| MIR206 | chr6:52008146-52010146 | 6 | 0.7041 |
| CD27 | chr12:6553050-6555050 | 7 | 0.7042 |
| GRAMD1A | chr19:35484630-35486630 | 5 | 0.7044 |
| SCART1 | chr10:135266431-135268431 | 6 | 0.7046 |
| CTRC | chr1:15763938-15765938 | 7 | 0.7050 |
| CNPPD1 | chr2:220035618-220037618 | 5 | 0.7053 |
| CX3CL1 | chr16:57405401-57407401 | 11 | 0.7057 |
| CSF2RB | chr22:37308669-37310669 | 5 | 0.7061 |
| LINC01749 | chr20:61639734-61641734 | 5 | 0.7070 |
| MTOR-AS1 | chr1:11202954-11204954 | 5 | 0.7072 |
| MIR593 | chr7:127720912-127722912 | 5 | 0.7075 |
| ZMYM1 | chr1:35524386-35526386 | 6 | 0.7082 |
| SHANK2-AS1 | chr11:70476198-70478198 | 5 | 0.7085 |

(continued)

| Promoter Region | Region Designation | Number of Probes in Region | Standard Deviation Cutoff |
|---|---|---|---|
| ACOT8 | chr20:44469359-44471359 | 5 | 0.7087 |
| EXOC3L4 | chr14:103565480-103567480 | 8 | 0.7088 |
| RNF224 | chr9:140121017-140123017 | 5 | 0.7090 |
| SLC22A17 | chr14:23814519-23816519 | 5 | 0.7092 |
| S100A14 | chr1:153585730-153587730 | 5 | 0.7095 |
| C2orf80 | chr2:209029070-209031070 | 5 | 0.7097 |
| HAUS7 | chrX:152712122-152714122 | 5 | 0.7103 |
| HLA-DOB | chr6:32779539-32781539 | 5 | 0.7103 |
| LINC00896 | chr22:20192854-20194854 | 5 | 0.7113 |
| SEPTIN8 | chr5:132085510-132087510 | 5 | 0.7117 |
| SLC12A4 | chr16:67976376-67978376 | 14 | 0.7123 |
| CARHSP1 | chr16:8945798-8947798 | 5 | 0.7126 |
| MCM7 | chr7:99689350-99691350 | 5 | 0.7133 |
| PRR5L | chr11:36316837-36318837 | 5 | 0.7135 |
| LOC100131635 | chr3:187419153-187421153 | 11 | 0.7138 |
| IL25 | chr14:23841017-23843017 | 5 | 0.7142 |
| SNORD107 | chr15:25226140-25228140 | 5 | 0.7146 |
| PWARSN | chr15:25226140-25228140 | 5 | 0.7146 |
| LINC01353 | chr1:203255279-203257279 | 5 | 0.7146 |
| ADGRL2 | chr1:81770876-81772876 | 5 | 0.7149 |
| DCST1 | chr1:155005281-155007281 | 12 | 0.7150 |
| PANX3 | chr11:124480323-124482323 | 8 | 0.7153 |
| CPA1 | chr7:130019334-130021334 | 7 | 0.7156 |
| CCDC166 | chr8:144787863-144789863 | 5 | 0.7157 |
| TPPP2 | chr14:21497375-21499375 | 6 | 0.7158 |
| DSCR10 | chr21:39577249-39579249 | 5 | 0.7158 |
| RRAGB | chrX:55743181-55745181 | 9 | 0.7159 |
| SNORD116-15 | chr15:25325432-25327432 | 5 | 0.7159 |
| MIR629 | chr15:70370710-70372710 | 7 | 0.7160 |
| OR1S1 | chr11:57981216-57983216 | 6 | 0.7160 |
| FLJ42969 | chr8:102063281-102065281 | 6 | 0.7162 |
| PRSS38 | chr1:228002417-228004417 | 9 | 0.7169 |
| SCP2D1 | chr20:18793426-18795426 | 6 | 0.7169 |
| ISLR | chr15:74465050-74467050 | 10 | 0.7169 |
| BPIFA1 | chr20:31822801-31824801 | 8 | 0.7171 |
| MIR543 | chr14:101497323-101499323 | 10 | 0.7172 |
| MIR1471 | chr2:232755951-232757951 | 5 | 0.7182 |
| TNRC6C | chr17:75999317-76001317 | 5 | 0.7184 |
| MIR27A | chr19: 13946253-13948253 | 12 | 0.7188 |

(continued)

| Promoter Region | Region Designation | Number of Probes in Region | Standard Deviation Cutoff |
|---|---|---|---|
| P4HB | chr17:79800041-79802041 | 12 | 0.7189 |
| TRIM69 | chr15:45027725-45029725 | 5 | 0.7197 |
| SNORD115-47 | chr15:25512663-25514663 | 5 | 0.7201 |
| BTNL8 | chr5:180325076-180327076 | 6 | 0.7202 |
| LST1 | chr6:31553053-31555053 | 9 | 0.7202 |
| LOC102546294 | chr5:147646869-147648869 | 7 | 0.7207 |
| MIR1185-2 | chr14:101509534-101511534 | 9 | 0.7215 |
| CLEC4M | chr19:7827128-7829128 | 5 | 0.7220 |
| LINC02090 | chr17:16890642-16892642 | 6 | 0.7229 |
| TNFAIP6 | chr2:152213105-152215105 | 6 | 0.7231 |
| RNF215 | chr22:30773809-30775809 | 5 | 0.7235 |
| SNORD115-32 | chr15:25473113-25475113 | 6 | 0.7249 |
| GSDMA | chr17:38118256-38120256 | 8 | 0.7249 |
| R3HDML | chr20:42964797-42966797 | 8 | 0.7251 |
| WDFY4 | chr10:49891917-49893917 | 17 | 0.7254 |
| LINC00636 | chr3:107601051-107603051 | 7 | 0.7258 |
| CIRBP-AS1 | chr19: 1266469-1268469 | 6 | 0.7259 |
| TRIM50 | chr7:72725531-72727531 | 9 | 0.7273 |
| SFRP5 | chr10:99525507-99527507 | 5 | 0.7277 |
| C1orf226 | chr1:162347630-162349630 | 9 | 0.7278 |
| SNORD41 | chr19: 12816262-12818262 | 8 | 0.7287 |
| MIR298 | chr20:57392280-57394280 | 7 | 0.7291 |
| MAGEB5 | chrX:26233285-26235285 | 5 | 0.7295 |
| TMPRSS4 | chr11:117946726-117948726 | 8 | 0.7296 |
| CARS-AS1 | chr11:3049623-3051623 | 5 | 0.7299 |
| UNC45B | chr17:33473835-33475835 | 9 | 0.7301 |
| MROH7-TTC4 | chr1:55106412-55108412 | 5 | 0.7306 |
| MROH7 | chr1:55106412-55108412 | 5 | 0.7306 |
| LOC100128593 | chr9:139639612-139641612 | 6 | 0.7306 |
| C20orf141 | chr20:2794632-2796632 | 7 | 0.7307 |
| KRTAP6-3 | chr21:31963758-31965758 | 6 | 0.7311 |
| OSTM1-AS1 | chr6:108443836-108445836 | 9 | 0.7316 |
| SNORD160 | chr1:45226706-45228706 | 5 | 0.7321 |
| SP140L | chr2:231190885-231192885 | 8 | 0.7325 |
| VARS1 | chr6:31744294-31746294 | 17 | 0.7327 |
| CGRRF1 | chr14:54975623-54977623 | 12 | 0.7348 |
| SSPO | chr7:149472130-149474130 | 5 | 0.7348 |
| TGM3 | chr20:2275646-2277646 | 9 | 0.7357 |
| SNORD115-33 | chr15:25474984-25476984 | 8 | 0.7358 |

(continued)

| Promoter Region | Region Designation | Number of Probes in Region | Standard Deviation Cutoff |
|---|---|---|---|
| DDX41 | chr5:176937577-176939577 | 7 | 0.7358 |
| VASH1-AS1 | chr14:77247075-77249075 | 7 | 0.7362 |
| TUBGCP2 | chr10:135091133-135093133 | 5 | 0.7363 |
| FCRLA | chr1:161676018-161678018 | 6 | 0.7367 |
| TLR4 | chr9:120465680-120467680 | 5 | 0.7377 |
| SNORD116-9 | chr15:25317252-25319252 | 9 | 0.7379 |
| LCE4A | chr1:152680522-152682522 | 6 | 0.7380 |
| USP7 | chr16:8984950-8986950 | 5 | 0.7384 |
| ABCC2 | chr10:101541396-101543396 | 7 | 0.7384 |
| MNT | chr17:2286366-2288366 | 5 | 0.7390 |
| UBE2Q1-AS1 | chr1:154525084-154527084 | 6 | 0.7392 |
| MIR557 | chr1:168343761-168345761 | 5 | 0.7395 |
| PRSS16 | chr6:27214479-27216479 | 8 | 0.7410 |
| CCDC13-AS1 | chr3:42773066-42775066 | 5 | 0.7410 |
| MIR23A | chr19:13946400-13948400 | 13 | 0.7410 |
| EPN3 | chr17:48609095-48611095 | 8 | 0.7410 |
| MIR24-2 | chr19:13946100-13948100 | 11 | 0.7413 |
| CHRNA9 | chr4:40336349-40338349 | 10 | 0.7417 |
| C5AR2 | chr19:47834431-47836431 | 9 | 0.7424 |
| TIAF1 | chr17:27399538-27401538 | 6 | 0.7443 |
| IL3 | chr5:131395347-131397347 | 10 | 0.7445 |
| CLEC3B | chr3:45066794-45068794 | 7 | 0.7451 |
| METTL11B | chr1:170114187-170116187 | 7 | 0.7454 |
| INHBA-AS1 | chr7:41732516-41734516 | 5 | 0.7454 |
| HTR3C | chr3:183769834-183771834 | 7 | 0.7461 |
| ZNF56 | chr19: 19886382-19888382 | 9 | 0.7462 |
| SERINC2 | chr1:31881623-31883623 | 8 | 0.7465 |
| NCKAP1L | chr12:54890535-54892535 | 6 | 0.7466 |
| BNIPL | chr1:151008061-151010061 | 5 | 0.7473 |
| NEURL2 | chr20:44516110-44518110 | 9 | 0.7476 |
| ADGRG5 | chr16:57575554-57577554 | 6 | 0.7476 |
| HRCT1 | chr9:35905198-35907198 | 7 | 0.7478 |
| MIR487A | chr14:101517782-101519782 | 6 | 0.7478 |
| SYNPO | chr5:149979637-149981637 | 7 | 0.7479 |
| LINC02622 | chr10:72697809-72699809 | 5 | 0.7480 |
| LINC01487 | chr3:154957733-154959733 | 5 | 0.7488 |
| SLC25A29 | chr14:100756452-100758452 | 8 | 0.7494 |
| SNORD115-30 | chr15:25469349-25471349 | 5 | 0.7494 |
| SLC22A11 | chr11:64322412-64324412 | 8 | 0.7494 |

(continued)

| Promoter Region | Region Designation | Number of Probes in Region | Standard Deviation Cutoff |
|---|---|---|---|
| LOC285804 | chr6:170574756-170576756 | 5 | 0.7499 |
| CLK3 | chr15:74899712-74901712 | 5 | 0.7503 |
| FSCN3 | chr7:127232789-127234789 | 7 | 0.7505 |
| XKR4 | chr8:56013587-56015587 | 6 | 0.7513 |
| SPRR3 | chr1:152973222-152975222 | 5 | 0.7518 |
| KIR2DL4 | chr19:55314064-55316064 | 10 | 0.7524 |
| LOC728485 | chr19:37263054-37265054 | 6 | 0.7528 |
| TLK2 | chr17:60555478-60557478 | 6 | 0.7528 |
| ZCCHC24 | chr10:81141080-81143080 | 6 | 0.7536 |
| CLDN20 | chr6:155584146-155586146 | 6 | 0.7552 |
| LOC100130548 | chr9:136918411-136920411 | 6 | 0.7552 |
| GNRH2 | chr20:3023267-3025267 | 8 | 0.7554 |
| GALNT10 | chr5:153569292-153571292 | 5 | 0.7556 |
| MIR326 | chr11:75045135-75047135 | 6 | 0.7557 |
| CCL22 | chr16:57391694-57393694 | 7 | 0.7558 |
| RDH5 | chr12:56113176-56115176 | 7 | 0.7559 |
| ESR1 | chr6:152010630-152012630 | 9 | 0.7563 |
| LGALS7B | chr19:39278839-39280839 | 6 | 0.7574 |
| DSPP | chr4:88528680-88530680 | 8 | 0.7577 |
| SNORD114-18 | chr14:101441161-101443161 | 5 | 0.7580 |
| SLC25A3P1 | chr1:53903042-53905042 | 7 | 0.7583 |
| LY96 | chr8:74902563-74904563 | 6 | 0.7586 |
| FLJ34503 | chr6:114224550-114226550 | 6 | 0.7588 |
| ITGB2-AS1 | chr21:46339949-46341949 | 14 | 0.7589 |
| LOC283038 | chr10:127370811-127372811 | 6 | 0.7593 |
| GPBAR1 | chr2:219123218-219125218 | 6 | 0.7599 |
| GCDH | chr19: 13000942-13002942 | 16 | 0.7607 |
| MIR1272 | chr15:65053585-65055585 | 9 | 0.7608 |
| CXCL8 | chr4:74605285-74607285 | 5 | 0.7623 |
| LGALS9 | chr17:25957213-25959213 | 6 | 0.7626 |
| SNORD83B | chr22:39708823-39710823 | 6 | 0.7631 |
| LOC105371046 | chr16:1629527-1631527 | 9 | 0.7634 |
| C20orf197 | chr20:58629979-58631979 | 8 | 0.7635 |
| LOC283177 | chr11:134305375-134307375 | 8 | 0.7635 |
| MIR128-2 | chr3:35784967-35786967 | 9 | 0.7643 |
| MIR1257 | chr20:60527601-60529601 | 7 | 0.7644 |
| KLB | chr4:39407549-39409549 | 8 | 0.7652 |
| RPS6KA2-AS1 | chr6:167316185-167318185 | 6 | 0.7661 |
| SNORA5B | chr7:45144566-45146566 | 8 | 0.7663 |

(continued)

| Promoter Region | Region Designation | Number of Probes in Region | Standard Deviation Cutoff |
|---|---|---|---|
| MIR877 | chr6:30551108-30553108 | 6 | 0.7667 |
| C17orf113 | chr17:40189249-40191249 | 5 | 0.7673 |
| ABHD17A | chr19: 1875808-1877808 | 7 | 0.7679 |
| PLEKHS1 | chr10:115510440-115512440 | 5 | 0.7682 |
| KLHDC4 | chr16:87740417-87742417 | 8 | 0.7686 |
| ZNF226 | chr19:44668214-44670214 | 13 | 0.7699 |
| MIR4640 | chr6:30857659-30859659 | 8 | 0.7707 |
| SOAT2 | chr12:53496269-53498269 | 8 | 0.7709 |
| GJB5 | chr1:35219647-35221647 | 8 | 0.7712 |
| CD4 | chr12:6897693-6899693 | 9 | 0.7714 |
| MIR421 | chrX:73437211-73439211 | 6 | 0.7716 |
| MIR374B | chrX:73437381-73439381 | 6 | 0.7716 |
| MIR374C | chrX:73437383-73439383 | 6 | 0.7716 |
| MIR296 | chr20:57391669-57393669 | 5 | 0.7717 |
| MAZ | chr16:29816444-29818444 | 9 | 0.7723 |
| AKT3 | chr1:243650534-243652534 | 7 | 0.7726 |
| MIR26B | chr2:219266368-219268368 | 7 | 0.7726 |
| PGBD4 | chr15:34393283-34395283 | 13 | 0.7728 |
| LCN9 | chr9:138554167-138556167 | 6 | 0.7733 |
| PWAR5 | chr15:25229006-25231006 | 7 | 0.7734 |
| SNORD64 | chr15:25229246-25231246 | 7 | 0.7734 |
| SNORD115-19 | chr15:25448503-25450503 | 7 | 0.7737 |
| SNORD116-11 | chr15:25320074-25322074 | 10 | 0.7740 |
| DCBLD1 | chr6:117802766-117804766 | 5 | 0.7745 |
| SERPINB7 | chr18:61419280-61421280 | 5 | 0.7745 |
| MIR1290 | chr1:19222564-19224564 | 5 | 0.7747 |
| LAX1 | chr1:203733310-203735310 | 9 | 0.7748 |
| LINC01270 | chr20:48908256-48910256 | 5 | 0.7748 |
| OLFML1 | chr11:7505736-7507736 | 7 | 0.7750 |
| SNORD115-28 | chr15:25466500-25468500 | 5 | 0.7757 |
| LY9 | chr1:160764963-160766963 | 6 | 0.7762 |
| IL1RN | chr2:113874469-113876469 | 7 | 0.7764 |
| EIF4ENIF1 | chr22:31834352-31836352 | 5 | 0.7764 |
| EFCAB14-AS1 | chr1:47138707-47140707 | 7 | 0.7767 |
| LINC00608 | chr2:219840005-219842005 | 7 | 0.7777 |
| MIR4496 | chr12:109028585-109030585 | 6 | 0.7780 |
| MIR129-1 | chr7:127846924-127848924 | 5 | 0.7797 |
| FITM1 | chr14:24599674-24601674 | 9 | 0.7804 |
| ST7-AS1 | chr7:116591499-116593499 | 10 | 0.7807 |

(continued)

| Promoter Region | Region Designation | Number of Probes in Region | Standard Deviation Cutoff |
|---|---|---|---|
| VPS28 | chr8:145647983-145649983 | 6 | 0.7809 |
| TRIM25 | chr17:54964269-54966269 | 6 | 0.7810 |
| MIR1276 | chr15:86312726-86314726 | 9 | 0.7816 |
| MIR521-2 | chr19:54218847-54220847 | 5 | 0.7818 |
| DNASE2B | chr1:84863214-84865214 | 8 | 0.7820 |
| PDE7B | chr6:136171838-136173838 | 8 | 0.7828 |
| IL33 | chr9:6214148-6216148 | 5 | 0.7828 |
| LINC01544 | chr18:59414408-59416408 | 6 | 0.7830 |
| LY6G6D | chr6:31682132-31684132 | 7 | 0.7831 |
| ABCC10 | chr6:43394278-43396278 | 12 | 0.7833 |
| SNORD115-22 | chr15:25454064-25456064 | 9 | 0.7834 |
| EDDM3A | chr14:21213045-21215045 | 6 | 0.7834 |
| LINC01802 | chr2:208124168-208126168 | 6 | 0.7841 |
| C2orf16 | chr2:27798388-27800388 | 7 | 0.7849 |
| SLC15A2 | chr3:121612247-121614247 | 5 | 0.7858 |
| C11orf94 | chr11:45927084-45929084 | 12 | 0.7858 |
| TBC1D10C | chr11:67170383-67172383 | 7 | 0.7861 |
| C1QA | chr1:22962120-22964120 | 5 | 0.7862 |
| C3orf35 | chr3:37426759-37428759 | 6 | 0.7864 |
| LOC101928881 | chr2:233876323-233878323 | 11 | 0.7864 |
| PATE1 | chr11:125615173-125617173 | 6 | 0.7865 |
| EHHADH-AS1 | chr3:184879688-184881688 | 7 | 0.7868 |
| IDH2 | chr15:90625276-90627276 | 5 | 0.7869 |
| OR2B11 | chr1:247613330-247615330 | 5 | 0.7870 |
| MIR637 | chr19:3960411-3962411 | 14 | 0.7871 |
| SNORD88B | chr19:51301285-51303285 | 10 | 0.7873 |
| LOC645177 | chr12:25149508-25151508 | 9 | 0.7878 |
| CCL25 | chr19:8116645-8118645 | 8 | 0.7879 |
| MAGEA8 | chrX:149008940-149010940 | 6 | 0.7881 |
| FCRLB | chr1:161690333-161692333 | 7 | 0.7881 |
| ZNF516-DT | chr18:74206294-74208294 | 10 | 0.7884 |
| CLEC3A | chr16:78055444-78057444 | 6 | 0.7887 |
| MIR218-1 | chr4:20528897-20530897 | 5 | 0.7887 |
| IL23R | chr1:67631136-67633136 | 6 | 0.7888 |
| LFNG | chr7:2551162-2553162 | 8 | 0.7893 |
| EXOSC2 | chr9:133568146-133570146 | 10 | 0.7899 |
| ZNF407 | chr18:72341918-72343918 | 5 | 0.7900 |
| TMEM213 | chr7:138481738-138483738 | 10 | 0.7900 |
| ANGPTL7 | chr1:11248411-11250411 | 6 | 0.7902 |

(continued)

| Promoter Region | Region Designation | Number of Probes in Region | Standard Deviation Cutoff |
|---|---|---:|---:|
| BTBD2 | chr19: 1984446-1986446 | 5 | 0.7911 |
| MIR4254 | chr1:32223260-32225260 | 8 | 0.7912 |
| HP | chr16:72087403-72089403 | 7 | 0.7916 |
| GRK1 | chr13:114320533-114322533 | 12 | 0.7916 |
| PADI6 | chr1:17697690-17699690 | 8 | 0.7925 |
| MIR2117HG | chr17:41521074-41523074 | 6 | 0.7930 |
| LOC105373051 | chr22:43607679-43609679 | 6 | 0.7935 |
| CALML6 | chr1:1845698-1847698 | 10 | 0.7938 |
| ABCG8 | chr2:44065109-44067109 | 21 | 0.7941 |
| KCNK18 | chr10:118955999-118957999 | 13 | 0.7946 |
| MIR643 | chr19:52784049-52786049 | 6 | 0.7957 |
| LOC105371485 | chr17: 1920009-1922009 | 7 | 0.7957 |
| ZNF595 | chr4:52178-54178 | 12 | 0.7959 |
| RNVU1-14 | chr1:148240464-148242464 | 6 | 0.7961 |
| KRT73-AS1 | chr12:53002634-53004634 | 5 | 0.7961 |
| RILP | chr17: 1548443-1550443 | 9 | 0.7970 |
| LINC00592 | chr12:52603713-52605713 | 6 | 0.7972 |
| ITK | chr5:156606906-156608906 | 6 | 0.7973 |
| PHLDB1 | chr11:118476150-118478150 | 7 | 0.7982 |
| CASP10 | chr2:202046620-202048620 | 8 | 0.7983 |
| MIR486-1 | chr8:41516958-41518958 | 11 | 0.7985 |
| MIR486-2 | chr8:41516961-41518961 | 11 | 0.7985 |
| LINC02508 | chr4:189696810-189698810 | 5 | 0.7986 |
| KRTAP5-9 | chr11:71258465-71260465 | 9 | 0.7990 |
| LY6G6C | chr6:31685424-31687424 | 7 | 0.7991 |
| NCF4 | chr22:37256029-37258029 | 8 | 0.8000 |
| UPK2 | chr11:118826007-118828007 | 5 | 0.8006 |
| RTP1 | chr3:186914273-186916273 | 7 | 0.8007 |
| MIR1913 | chr6:166921841-166923841 | 6 | 0.8009 |
| ICOS | chr2:204800485-204802485 | 7 | 0.8010 |
| SNORD94 | chr2:86361992-86363992 | 6 | 0.8010 |
| KRTAP22-1 | chr21:31972405-31974405 | 6 | 0.8015 |
| TRIM39-RPP21 | chr6:30296087-30298087 | 12 | 0.8018 |
| GCSIR | chr2:231750260-231752260 | 6 | 0.8019 |
| KRT19 | chr17:39678868-39680868 | 8 | 0.8023 |
| AQP10 | chr1:154292568-154294568 | 5 | 0.8025 |
| SNORD99 | chr1:28904254-28906254 | 5 | 0.8034 |
| MIR10B | chr2:177014030-177016030 | 15 | 0.8036 |
| CREB5 | chr7:28337939-28339939 | 5 | 0.8039 |

(continued)

| Promoter Region | Region Designation | Number of Probes in Region | Standard Deviation Cutoff |
|---|---|---|---|
| CA6 | chr1:9004926-9006926 | 9 | 0.8040 |
| GHRL | chr3:10326349-10328349 | 5 | 0.8043 |
| S100P | chr4:6694604-6696604 | 9 | 0.8053 |
| SNORD114-31 | chr14:101458572-101460572 | 5 | 0.8058 |
| SNORD102 | chr13:27828200-27830200 | 5 | 0.8058 |
| SNORA27 | chr13:27828537-27830537 | 5 | 0.8058 |
| GUCA2B | chr1:42618080-42620080 | 10 | 0.8059 |
| FBXW12 | chr3:48412708-48414708 | 10 | 0.8064 |
| AOC1 | chr7:150548604-150550604 | 9 | 0.8064 |
| KCNK2 | chr1:215177884-215179884 | 9 | 0.8065 |
| LINC01872 | chr19:51773519-51775519 | 7 | 0.8068 |
| MIR7159 | chr6:33865911-33867911 | 11 | 0.8071 |
| SCARNA5 | chr2:234183371-234185371 | 6 | 0.8077 |
| CTC1 | chr17:8127132-8129132 | 7 | 0.8088 |
| EPS15L1 | chr19:16465054-16467054 | 6 | 0.8088 |
| SAG | chr2:234215461-234217461 | 11 | 0.8089 |
| GNAS-AS1 | chr20:57392972-57394972 | 5 | 0.8094 |
| INMT | chr7:30790750-30792750 | 7 | 0.8094 |
| INMT-MINDY4 | chr7:30790750-30792750 | 7 | 0.8094 |
| LINC02010 | chr3:146638709-146640709 | 5 | 0.8095 |
| LOC100507156 | chr8:23192720-23194720 | 5 | 0.8098 |
| PRSS55 | chr8:10382041-10384041 | 6 | 0.8098 |
| SH3TC2-DT | chr5:148441879-148443879 | 9 | 0.8102 |
| SARS1 | chr1:109755514-109757514 | 10 | 0.8110 |
| SLC22A12 | chr11:64357281-64359281 | 11 | 0.8124 |
| SLC5A2 | chr16:31493443-31495443 | 7 | 0.8126 |
| OR10D3 | chr11:124054922-124056922 | 6 | 0.8133 |
| G6PC | chr17:41051815-41053815 | 9 | 0.8135 |
| FXYD2 | chr11:117689771-117691771 | 5 | 0.8136 |
| FXYD6-FXYD2 | chr11:117689795-117691795 | 5 | 0.8136 |
| LIN37 | chr19:36238476-36240476 | 13 | 0.8136 |
| ZDHHC18 | chr1:27152178-27154178 | 5 | 0.8136 |
| MIR381HG | chr14:101510493-101512493 | 9 | 0.8140 |
| KLRF2 | chr12:10033087-10035087 | 5 | 0.8143 |
| MIR1207 | chr8:129060397-129062397 | 7 | 0.8144 |
| IFI44L | chr1:79085132-79087132 | 5 | 0.8153 |
| LINC01585 | chr15:91202464-91204464 | 7 | 0.8153 |
| LINC00856 | chr10:80007381-80009381 | 6 | 0.8157 |
| MIR890 | chrX:145074792-145076792 | 5 | 0.8166 |

(continued)

| Promoter Region | Region Designation | Number of Probes in Region | Standard Deviation Cutoff |
|---|---|---|---|
| SNORD131 | chr11:1969560-1971560 | 5 | 0.8175 |
| NENF | chr1:212605261-212607261 | 10 | 0.8183 |
| BPIFB3 | chr20:31642136-31644136 | 6 | 0.8185 |
| LOC154449 | chr6:170562421-170564421 | 5 | 0.8186 |
| DDX54 | chr12:113593977-113595977 | 8 | 0.8189 |
| CELA2A | chr1:15782226-15784226 | 5 | 0.8190 |
| ECI1 | chr16:2288402-2290402 | 5 | 0.8190 |
| LINC02020 | chr3:186157133-186159133 | 5 | 0.8194 |
| MIR5571 | chr22:23227446-23229446 | 6 | 0.8195 |
| ANKFN1 | chr17:54229835-54231835 | 9 | 0.8197 |
| PCID2 | chr13:113830849-113832849 | 5 | 0.8198 |
| ZCWPW1 | chr7:99997438-99999438 | 5 | 0.8199 |
| ZNF548 | chr19:57900217-57902217 | 12 | 0.8206 |
| FAM53B-AS1 | chr10:126391596-126393596 | 7 | 0.8214 |
| TGIF2LY | chrY:3446125-3448125 | 7 | 0.8229 |
| MR1 | chr1:181001560-181003560 | 7 | 0.8230 |
| THRSP | chr11:77773906-77775906 | 5 | 0.8234 |
| OSBPL10-AS1 | chr3:31744704-31746704 | 8 | 0.8239 |
| LUZP6 | chr7:135610507-135612507 | 5 | 0.8240 |
| MTPN | chr7:135610507-135612507 | 5 | 0.8240 |
| C15orf62 | chr15:41061177-41063177 | 11 | 0.8242 |
| LINC01259 | chr4:38510387-38512387 | 6 | 0.8244 |
| LINC00929 | chr15:26359959-26361959 | 5 | 0.8245 |
| SMPDL3B | chr1:28260465-28262465 | 9 | 0.8246 |
| ZBED6CL | chr7:150025937-150027937 | 5 | 0.8250 |
| SCNN1D | chr1:1214815-1216815 | 6 | 0.8260 |
| LOC105375131 | chr7:4166243-4168243 | 7 | 0.8267 |
| PBX2 | chr6:32151509-32153509 | 7 | 0.8268 |
| NOS3 | chr7:150687104-150689104 | 5 | 0.8272 |
| SNORD115-27 | chr15:25464649-25466649 | 7 | 0.8275 |
| ICA1 | chr7:8151813-8153813 | 6 | 0.8276 |
| LOC101928279 | chr4:4762486-4764486 | 7 | 0.8278 |
| DMWD | chr19:46285204-46287204 | 6 | 0.8278 |
| SNORD115-39 | chr15:25485892-25487892 | 5 | 0.8279 |
| TEX37 | chr2:88823166-88825166 | 6 | 0.8279 |
| CCRL2 | chr3:46447749-46449749 | 17 | 0.8280 |
| MIR30D | chr8:135816118-135818118 | 5 | 0.8283 |
| BSND | chr1:55463605-55465605 | 11 | 0.8285 |
| BPIFA4P | chr20:31780410-31782410 | 8 | 0.8286 |

(continued)

| Promoter Region | Region Designation | Number of Probes in Region | Standard Deviation Cutoff |
|---|---|---|---|
| ZNF804B | chr7:88388013-88390013 | 8 | 0.8287 |
| PLEKHB1 | chr11:73356625-73358625 | 14 | 0.8291 |
| MIR190B | chr1:154165140-154167140 | 7 | 0.8294 |
| C20orf204 | chr20:62664079-62666079 | 7 | 0.8300 |
| SNORD116-7 | chr15:25311933-25313933 | 5 | 0.8303 |
| SP140 | chr2:231089444-231091444 | 9 | 0.8304 |
| MIR200C | chr12:7071861-7073861 | 9 | 0.8304 |
| MIR141 | chr12:7072259-7074259 | 9 | 0.8304 |
| PRKAR1B | chr7:587833-589833 | 7 | 0.8308 |
| LCN2 | chr9:130910731-130912731 | 5 | 0.8313 |
| C1orf68 | chr1:152690997-152692997 | 6 | 0.8316 |
| ADGRG7 | chr3:100327444-100329444 | 7 | 0.8323 |
| GJD4 | chr10:35893268-35895268 | 11 | 0.8328 |
| PLCD4 | chr2:219471621-219473621 | 8 | 0.8329 |
| MIR2117 | chr17:41521173-41523173 | 5 | 0.8329 |
| TMEM140 | chr7:134831823-134833823 | 14 | 0.8333 |
| KIF25-AS1 | chr6:168393866-168395866 | 6 | 0.8334 |
| TRIM54 | chr2:27504296-27506296 | 9 | 0.8339 |
| EHF | chr11:34641639-34643639 | 5 | 0.8342 |
| PRKAR1B-AS1 | chr7:641481-643481 | 7 | 0.8344 |
| ECE1-AS1 | chr1:21618782-21620782 | 6 | 0.8346 |
| ZSCAN5C | chr19:56716282-56718282 | 5 | 0.8360 |
| ABCG1 | chr21:43618798-43620798 | 5 | 0.8361 |
| MRGPRD | chr11:68746489-68748489 | 6 | 0.8361 |
| FLJ44635 | chrX:71363033-71365033 | 7 | 0.8361 |
| LINGO3 | chr19:2288782-2290782 | 6 | 0.8364 |
| SNORD115-31 | chr15:25471255-25473255 | 8 | 0.8365 |
| HSD17B2 | chr16:82067857-82069857 | 8 | 0.8371 |
| SERPINE1 | chr7:100769384-100771384 | 8 | 0.8372 |
| CCM2L | chr20:30597240-30599240 | 5 | 0.8373 |
| SNORD116-12 | chr15:25321196-25323196 | 8 | 0.8376 |
| TUBB1 | chr20:57593483-57595483 | 6 | 0.8380 |
| UCN2 | chr3:48598150-48600150 | 8 | 0.8381 |
| CHI3L2 | chr1:111769280-111771280 | 8 | 0.8384 |
| LOC105370489 | chr14:51287514-51289514 | 8 | 0.8384 |
| TRIM55 | chr8:67038363-67040363 | 8 | 0.8387 |
| RABEPK | chr9:127961820-127963820 | 9 | 0.8391 |
| CEACAM16 | chr19:4520 1420-45203420 | 8 | 0.8396 |
| LINC01387 | chr18:6510414-6512414 | 6 | 0.8397 |

(continued)

| Promoter Region | Region Designation | Number of Probes in Region | Standard Deviation Cutoff |
|---|---|---|---|
| ZNF547 | chr19:57873802-57875802 | 15 | 0.8410 |
| TRAPPC2B | chr19:57873918-57875918 | 15 | 0.8410 |
| NUDT22 | chr11:63992729-63994729 | 14 | 0.8411 |
| C16orf82 | chr16:27077247-27079247 | 7 | 0.8417 |
| MIR1260A | chr14:77731560-77733560 | 6 | 0.8418 |
| LCE2A | chr1:152669820-152671820 | 7 | 0.8419 |
| MIR370 | chr14:101376475-101378475 | 7 | 0.8428 |
| LOC105372179 | chr18:67136166-67138166 | 5 | 0.8429 |
| FCGR2A | chr1:161474246-161476246 | 7 | 0.8432 |
| VRTN | chr14:74814173-74816173 | 7 | 0.8435 |
| MUC17 | chr7:100662361-100664361 | 5 | 0.8437 |
| LINC00954 | chr2:20067614-20069614 | 7 | 0.8438 |
| NKAPD1 | chr11:111944022-111946022 | 16 | 0.8441 |
| MIR4512 | chr15:66788295-66790295 | 6 | 0.8443 |
| RMDN2-AS1 | chr2:38176476-38178476 | 6 | 0.8445 |
| C1S | chr12:7167021-7169021 | 10 | 0.8453 |
| PATE3 | chr11:125657021-125659021 | 5 | 0.8456 |
| KIAA0100 | chr17:26940457-26942457 | 9 | 0.8461 |
| SLC34A3 | chr9:140124208-140126208 | 6 | 0.8467 |
| TSPO2 | chr6:41009205-41011205 | 8 | 0.8468 |
| OR4S2 | chr11:55417379-55419379 | 5 | 0.8478 |
| MIR6890 | chr3:49136286-49138286 | 6 | 0.8479 |
| OR51T1 | chr11:4901929-4903929 | 5 | 0.8482 |
| DNAJA3 | chr16:4474805-4476805 | 13 | 0.8483 |
| TSHZ1 | chr18:72921751-72923751 | 7 | 0.8485 |
| CD2 | chr1:117296051-117298051 | 5 | 0.8490 |
| FAM71F2 | chr7:128311319-128313319 | 5 | 0.8495 |
| ZNF331 | chr19:54023267-54025267 | 20 | 0.8503 |
| CCDC24 | chr1:44456267-44458267 | 16 | 0.8504 |
| GPR142 | chr17:72362644-72364644 | 5 | 0.8506 |
| GLIS3-AS1 | chr9:3897645-3899645 | 5 | 0.8509 |
| CHRM1 | chr11:62675150-62677150 | 5 | 0.8513 |
| ALS2 | chr2:202563989-202565989 | 5 | 0.8513 |
| MIR6773 | chr16:68266328-68268328 | 5 | 0.8516 |
| BHLHE22 | chr8:65491921-65493921 | 12 | 0.8519 |
| LINC01162 | chr7:20874049-20876049 | 5 | 0.8522 |
| MIR942 | chr1:117636264-117638264 | 8 | 0.8523 |
| PTH1R | chr3:46918210-46920210 | 6 | 0.8530 |
| DSG4 | chr18:28955739-28957739 | 6 | 0.8531 |

(continued)

| Promoter Region | Region Designation | Number of Probes in Region | Standard Deviation Cutoff |
|---|---|---|---|
| MIR8059 | chr17:48845010-48847010 | 6 | 0.8532 |
| IL17RE | chr3:9943295-9945295 | 9 | 0.8541 |
| CCDC183 | chr9:139689789-139691789 | 7 | 0.8558 |
| KRTAP5-6 | chr11:1717424-1719424 | 5 | 0.8559 |
| KIF13B | chr8:28923794-28925794 | 5 | 0.8564 |
| S100A9 | chr1:153329329-153331329 | 11 | 0.8566 |
| MIR584 | chr5:148440875-148442875 | 5 | 0.8568 |
| LINC00222 | chr6:109071856-109073856 | 6 | 0.8568 |
| MIR645 | chr20:49201322-49203322 | 5 | 0.8574 |
| GPR20 | chr8:142365569-142367569 | 8 | 0.8576 |
| LINC02763 | chr11:112351954-112353954 | 5 | 0.8594 |
| UBOX5-AS1 | chr20:3086556-3088556 | 6 | 0.8596 |
| VIT | chr2:36922832-36924832 | 6 | 0.8610 |
| KCNIP1-OT1 | chr5:169815496-169817496 | 9 | 0.8614 |
| PDE6C | chr10:95371293-95373293 | 9 | 0.8621 |
| PCAT1 | chr8:128024398-128026398 | 5 | 0.8622 |
| NPHP4 | chr1:5921870-5923870 | 7 | 0.8624 |
| MIR4689 | chr1:5921731-5923731 | 7 | 0.8624 |
| KRTAP9-3 | chr17:39387700-39389700 | 6 | 0.8627 |
| SNORA36C | chr2:69746175-69748175 | 5 | 0.8633 |
| CSF3 | chr17:38170692-38172692 | 7 | 0.8642 |
| CCT5 | chr5:10249032-10251032 | 14 | 0.8648 |
| GALNT15 | chr3:16215186-16217186 | 13 | 0.8656 |
| CHRND | chr2:233389869-233391869 | 10 | 0.8657 |
| LINC01844 | chr5:142124164-142126164 | 5 | 0.8659 |
| CMTM5 | chr14:23845254-23847254 | 9 | 0.8662 |
| PIK3CG | chr7:106504726-106506726 | 8 | 0.8666 |
| MIR802 | chr21:37092012-37094012 | 7 | 0.8671 |
| SERPIND1 | chr22:21127400-21129400 | 6 | 0.8674 |
| CCL13 | chr17:32682498-32684498 | 6 | 0.8676 |
| SCARNA12 | chr12:7075499-7077499 | 5 | 0.8680 |
| LINC02288 | chr14:77506391-77508391 | 7 | 0.8680 |
| DSCAM-AS1 | chr21:41754009-41756009 | 5 | 0.8684 |
| ACTA2-AS1 | chr10:90691440-90693440 | 5 | 0.8690 |
| MIR1303 | chr5:154064335-154066335 | 5 | 0.8697 |
| STAU2-AS1 | chr8:74331308-74333308 | 9 | 0.8697 |
| MYO5C | chr15:52483518-52485518 | 7 | 0.8703 |
| PARVB | chr22:44394090-44396090 | 9 | 0.8706 |
| FHOD1 | chr16:67262291-67264291 | 6 | 0.8707 |

(continued)

| Promoter Region | Region Designation | Number of Probes in Region | Standard Deviation Cutoff |
|---|---|---|---|
| TRIM40 | chr6:30102916-30104916 | 10 | 0.8708 |
| SPINK5 | chr5:147442534-147444534 | 7 | 0.8709 |
| TLDC2 | chr20:35503523-35505523 | 9 | 0.8713 |
| SNORD115-25 | chr15:25459687-25461687 | 5 | 0.8725 |
| FOLR1 | chr11:71899752-71901752 | 6 | 0.8729 |
| SNORD63 | chr5:137895731-137897731 | 8 | 0.8736 |
| PRR9 | chr1:153189059-153191059 | 6 | 0.8737 |
| LNCNEF | chr20:22567159-22569159 | 5 | 0.8738 |
| NAE1 | chr16:66835780-66837780 | 5 | 0.8740 |
| BATF | chr14:75987811-75989811 | 9 | 0.8742 |
| SNORD139 | chr22:39711846-39713846 | 9 | 0.8750 |
| PRM3 | chr16:11366055-11368055 | 7 | 0.8751 |
| ITIH4-AS1 | chr3:52856950-52858950 | 6 | 0.8754 |
| TDGF1 | chr3:46615024-46617024 | 5 | 0.8758 |
| PADI1 | chr1:17530622-17532622 | 6 | 0.8762 |
| MOG | chr6:29623868-29625868 | 6 | 0.8763 |
| LOC102723838 | chr11:102336985-102338985 | 5 | 0.8770 |
| CELA2B | chr1:15801598-15803598 | 6 | 0.8774 |
| LINC02517 | chr4:8320902-8322902 | 5 | 0.8777 |
| EMILIN1 | chr2:27300482-27302482 | 10 | 0.8777 |
| MIR3591 | chr18:56117311-56119311 | 5 | 0.8788 |
| MIR122 | chr18:56117305-56119305 | 5 | 0.8788 |
| TIGAR | chr12:4429378-4431378 | 9 | 0.8790 |
| AIF1 | chr6:31582010-31584010 | 6 | 0.8791 |
| FMO1 | chr1:171216609-171218609 | 7 | 0.8792 |
| LOC105371458 | chr1:157894756-157896756 | 6 | 0.8794 |
| WNT8B | chr10:102221765-102223765 | 6 | 0.8795 |
| DDOST | chr1:20977259-20979259 | 5 | 0.8797 |
| OR51Q1 | chr11:5442340-5444340 | 5 | 0.8799 |
| WFIKKN1 | chr16:679984-681984 | 7 | 0.8803 |
| AKR7A2P1 | chr1:113464971-113466971 | 9 | 0.8808 |
| MORC2-AS1 | chr22:31317294-31319294 | 12 | 0.8811 |
| RXFP4 | chr1:155910479-155912479 | 8 | 0.8816 |
| ZBTB17 | chr1:16267363-16269363 | 6 | 0.8816 |
| HIGD1B | chr17:42922696-42924696 | 6 | 0.8819 |
| SNORD116-18 | chr15:25329530-25331530 | 5 | 0.8819 |
| GNLY | chr2:85920480-85922480 | 9 | 0.8824 |
| FAM83A | chr8:124190286-124192286 | 8 | 0.8827 |
| APOBEC3A | chr22:39352613-39354613 | 6 | 0.8827 |

(continued)

| Promoter Region | Region Designation | Number of Probes in Region | Standard Deviation Cutoff |
|---|---|---|---|
| C3orf56 | chr3:126910973-126912973 | 11 | 0.8829 |
| LINC00445 | chr13:36270660-36272660 | 10 | 0.8832 |
| CD36 | chr7:80230522-80232522 | 6 | 0.8837 |
| SNORD115-17 | chr15:25445469-25447469 | 7 | 0.8842 |
| IVL | chr1:152880020-152882020 | 7 | 0.8843 |
| MX2 | chr21:42732953-42734953 | 9 | 0.8844 |
| VWA3A | chr16:22102861-22104861 | 7 | 0.8851 |
| LINC01150 | chr11:1916988-1918988 | 5 | 0.8854 |
| SNORA49 | chr12:132514768-132516768 | 6 | 0.8855 |
| PHKA2 | chrX:18909415-18911415 | 5 | 0.8855 |
| CLDN24 | chr4:184241916-184243916 | 10 | 0.8873 |
| LINC02125 | chr16:76667894-76669894 | 5 | 0.8874 |
| SNORD115-41 | chr15:25489624-25491624 | 5 | 0.8875 |
| C7orf65 | chr7:47693841-47695841 | 9 | 0.8876 |
| MUC2 | chr11:1073874-1075874 | 9 | 0.8881 |
| ITGB1BP2 | chrX:70520599-70522599 | 8 | 0.8882 |
| MAP3K15 | chrX:19377175-19379175 | 5 | 0.8897 |
| XAF1 | chr17:6657765-6659765 | 13 | 0.8898 |
| MIR518F | chr19:54202268-54204268 | 6 | 0.8901 |
| TBC1D26 | chr17: 15634590-15636590 | 8 | 0.8902 |
| SNORD114-25 | chr14:101451393-101453393 | 5 | 0.8905 |
| PSD4 | chr2:113930547-113932547 | 7 | 0.8908 |
| LINC00595 | chr10:80026098-80028098 | 6 | 0.8914 |
| MIR3131 | chr2:219922409-219924409 | 8 | 0.8918 |
| LENEP | chr1:154965061-154967061 | 7 | 0.8923 |
| MS4A14 | chr11:60162486-60164486 | 5 | 0.8926 |
| LINC00347 | chr13:75125979-75127979 | 5 | 0.8928 |
| DMP1 | chr4:88570431-88572431 | 8 | 0.8930 |
| TCAP | chr17:37820601-37822601 | 7 | 0.8940 |
| TMC4 | chr19:54662845-54664845 | 15 | 0.8942 |
| CENPU | chr4:185614238-185616238 | 5 | 0.8950 |
| HTR3A | chr11:113844796-113846796 | 8 | 0.8951 |
| CAPN9 | chr1:230882129-230884129 | 9 | 0.8952 |
| CD53 | chr1:111412820-111414820 | 5 | 0.8953 |
| CDA | chr1:20914589-20916589 | 7 | 0.8957 |
| C 19orf38 | chr19:10958090-10960090 | 5 | 0.8962 |
| RORC | chr1:151777546-151779546 | 7 | 0.8964 |
| CYP21A2 | chr6:32005082-32007082 | 5 | 0.8967 |
| IL34 | chr16:70612797-70614797 | 9 | 0.8972 |

(continued)

| Promoter Region | Region Designation | Number of Probes in Region | Standard Deviation Cutoff |
|---|---|---|---|
| KLK3 | chr19:51357170-51359170 | 6 | 0.8982 |
| OPTC | chr1:203462280-203464280 | 5 | 0.8990 |
| MIR1265 | chr10:14477574-14479574 | 6 | 0.8993 |
| SNORD21 | chr1:93301845-93303845 | 5 | 0.8996 |
| HTR3D | chr3:183748331-183750331 | 6 | 0.8996 |
| SULF1 | chr8:70377858-70379858 | 10 | 0.9001 |
| CXCR5 | chr11:118753600-118755600 | 11 | 0.9007 |
| ADGRF4 | chr6:47665315-47667315 | 5 | 0.9009 |
| SULT1C4 | chr2:108993409-108995409 | 10 | 0.9010 |
| SNORD116-1 | chr15:25295622-25297622 | 9 | 0.9011 |
| HSD11B1 | chr1:209858524-209860524 | 6 | 0.9011 |
| PRG4 | chr1:186264404-186266404 | 8 | 0.9013 |
| SNORD12 | chr20:47896219-47898219 | 8 | 0.9016 |
| IL4 | chr5:132008677-132010677 | 6 | 0.9018 |
| ANXA2R | chr5:43038181-43040181 | 6 | 0.9021 |
| PDZD7 | chr10:102766435-102768435 | 6 | 0.9023 |
| CHRM5 | chr15:34259697 -34261697 | 8 | 0.9025 |
| MIR30A | chr6:72112253-72114253 | 5 | 0.9032 |
| KIRREL3-AS2 | chr11:126809641-126811641 | 5 | 0.9038 |
| NTRK2 | chr9:87282372-87284372 | 11 | 0.9040 |
| RNU6-8 | chr14:32671368-32673368 | 8 | 0.9040 |
| LRRC17 | chr7:102552446-102554446 | 7 | 0.9041 |
| CD52 | chr1:26643448-26645448 | 11 | 0.9044 |
| LINCR-0001 | chr8:10331074-10333074 | 5 | 0.9050 |
| RNVU1-17 | chr1:149193105-149195105 | 8 | 0.9050 |
| LINC01445 | chr7:54397389-54399389 | 5 | 0.9053 |
| SPATA17 | chr1:217803685-217805685 | 11 | 0.9054 |
| F7 | chr13:113759101-113761101 | 8 | 0.9060 |
| CSMD2-AS1 | chr1:34333556-34335556 | 5 | 0.9065 |
| MIR320B1 | chr1:117213367-117215367 | 5 | 0.9068 |
| MYLK-AS2 | chr3:123407490-123409490 | 5 | 0.9073 |
| MIR3142HG | chr5:159894257-159896257 | 5 | 0.9074 |
| SLC3A1 | chr2:44501618-44503618 | 5 | 0.9079 |
| FGF14-AS1 | chr13:103018879-103020879 | 5 | 0.9082 |
| SNORD4A | chr17:27048599-27050599 | 8 | 0.9087 |
| MIR507 | chrX:146311501-146313501 | 6 | 0.9091 |
| GPR150 | chr5:94954790-94956790 | 10 | 0.9092 |
| OTOR | chr20:16727997-16729997 | 8 | 0.9094 |
| NEU4 | chr2:242749287-242751287 | 17 | 0.9095 |

(continued)

| Promoter Region | Region Designation | Number of Probes in Region | Standard Deviation Cutoff |
|---|---|---:|---:|
| DBP | chr19:49132286-49134286 | 7 | 0.9097 |
| GRAMD1B | chr11:123228129-123230129 | 5 | 0.9098 |
| PTGER1 | chr19:14582277-14584277 | 6 | 0.9100 |
| DEFB127 | chr20:137094-139094 | 6 | 0.9102 |
| GPR182 | chr12:57387275-57389275 | 8 | 0.9108 |
| KRTAP10-3 | chr21:45976672-45978672 | 5 | 0.9109 |
| HSH2D | chr19:16243837-16245837 | 5 | 0.9117 |
| ZDHHC7 | chr16:85006780-85008780 | 6 | 0.9120 |
| UBALD1 | chr16:4657884-4659884 | 5 | 0.9122 |
| MLLT10 | chr10:21821277-21823277 | 6 | 0.9123 |
| FURIN | chr15:91410817-91412817 | 5 | 0.9123 |
| CD79A | chr19:42380348-42382348 | 5 | 0.9127 |
| MISP3 | chr19:14182820-14184820 | 14 | 0.9130 |
| FXYD4 | chr10:43866083-43868083 | 8 | 0.9133 |
| ACAA1 | chr3:38163200-38165200 | 7 | 0.9134 |
| PRSS56 | chr2:233384097-233386097 | 6 | 0.9134 |
| LRRC74A | chr14:77291750-77293750 | 6 | 0.9146 |
| ZNF833P | chr19: 11783812-11785812 | 14 | 0.9146 |
| DLGAP1-AS1 | chr18:3593111-3595111 | 11 | 0.9154 |
| CHST4 | chr16:71559022-71561022 | 6 | 0.9155 |
| TSPAN17 | chr5:176073484-176075484 | 12 | 0.9158 |
| TTR | chr18:29170729-29172729 | 7 | 0.9159 |
| GJA8 | chr1:147373920-147375920 | 6 | 0.9173 |
| FAT4 | chr4:126236566-126238566 | 11 | 0.9177 |
| GPR21 | chr9:125794921-125796921 | 11 | 0.9179 |
| S100Z | chr5:76144838-76146838 | 6 | 0.9180 |
| SRRM5 | chr19:44115239-44117239 | 7 | 0.9183 |
| LOC105371730 | chr17:30467244-30469244 | 5 | 0.9187 |
| UTP14C | chr13:52597826-52599826 | 7 | 0.9190 |
| RNASE6 | chr14:21248426-21250426 | 5 | 0.9191 |
| SNORD4B | chr17:27049698-27051698 | 5 | 0.9192 |
| CHMP4C | chr8:82643682-82645682 | 10 | 0.9194 |
| FRMD6 | chr14:51954845-51956845 | 9 | 0.9199 |
| GPX5 | chr6:28492657-28494657 | 12 | 0.9200 |
| ARHGAP25 | chr2:68960942-68962942 | 7 | 0.9200 |
| LOC101927969 | chr5:168132931-168134931 | 5 | 0.9204 |
| LOC100506178 | chr7:22601955-22603955 | 5 | 0.9205 |
| DEFB129 | chr20:206898-208898 | 5 | 0.9206 |
| RAB19 | chr7:140102857-140104857 | 10 | 0.9208 |

(continued)

| Promoter Region | Region Designation | Number of Probes in Region | Standard Deviation Cutoff |
|---|---|---|---|
| PSTPIP1 | chr15:77286020-77288020 | 11 | 0.9208 |
| RP1 | chr8:55527655-55529655 | 5 | 0.9209 |
| BCO1 | chr16:81271293-81273293 | 7 | 0.9213 |
| LINC00514 | chr16:3038054-3040054 | 5 | 0.9215 |
| RNF166 | chr16:88761908-88763908 | 7 | 0.9218 |
| DEFB118 | chr20:29955403-29957403 | 5 | 0.9219 |
| LINC02656 | chr10:6391277-6393277 | 5 | 0.9221 |
| MIR6727 | chr1:1246881-1248881 | 6 | 0.9225 |
| LOC105371566 | chr17:18010004-18012004 | 7 | 0.9225 |
| DCSTAMP | chr8:105351023-105353023 | 5 | 0.9229 |
| SNORD116-14 | chr15:25324287-25326287 | 6 | 0.9233 |
| HHLA2 | chr3:108014336-108016336 | 5 | 0.9235 |
| TLR3 | chr4:186989308-186991308 | 6 | 0.9237 |
| UTRN | chr6:144611872-144613872 | 8 | 0.9238 |
| LINC02085 | chr3:101658702-101660702 | 5 | 0.9239 |
| LINC00570 | chr2:11533106-11535106 | 5 | 0.9239 |
| MYOM1 | chr18:3065804-3067804 | 6 | 0.9240 |
| DPEP1 | chr16:89678715-89680715 | 5 | 0.9243 |
| FBXW4P1 | chr22:23603953-23605953 | 5 | 0.9247 |
| DNAJB5 | chr9:34988637-34990637 | 13 | 0.9249 |
| CPA4 | chr7:129931973-129933973 | 5 | 0.9250 |
| LINC00911 | chr14:85859222-85861222 | 6 | 0.9254 |
| LINC01440 | chr20:54038580-54040580 | 6 | 0.9259 |
| SNORD115-23 | chr15:25455942-25457942 | 7 | 0.9259 |
| PWAR4 | chr15:25455838-25457838 | 7 | 0.9259 |
| LINC02035 | chr3:122604359-122606359 | 6 | 0.9259 |
| LINC00513 | chr7:130597222-130599222 | 5 | 0.9259 |
| KRTAP10-10 | chr21:46056272-46058272 | 6 | 0.9262 |
| SCGB2A2 | chr11:62036629-62038629 | 6 | 0.9264 |
| A2M-AS1 | chr12:9216772-9218772 | 12 | 0.9269 |
| LOC101929106 | chr3:186913877-186915877 | 10 | 0.9272 |
| MIR9-3 | chr15:89910247-89912247 | 9 | 0.9273 |
| MIR1231 | chr1:201776738-201778738 | 6 | 0.9273 |
| LOC101927964 | chr10:4092917-4094917 | 6 | 0.9276 |
| SLC6A6 | chr3:14443075-14445075 | 11 | 0.9282 |
| SNORA41B | chr15:45828448-45830448 | 5 | 0.9284 |
| LINC01634 | chr22:18511150-18513150 | 5 | 0.9290 |
| XPC | chr3:14185646-14187646 | 5 | 0.9294 |
| LINC01568 | chr16:73419703-73421703 | 6 | 0.9301 |

(continued)

| Promoter Region | Region Designation | Number of Probes in Region | Standard Deviation Cutoff |
|---|---|---:|---:|
| CLEC4A | chr12:8275227-8277227 | 5 | 0.9306 |
| DDN | chr12:49387931-49389931 | 5 | 0.9307 |
| ARSH | chrX:2923586-2925586 | 6 | 0.9307 |
| COPZ2 | chr17:46102532-46104532 | 5 | 0.9308 |
| NCOA6 | chr20:33301577-33303577 | 5 | 0.9310 |
| PCK1 | chr20:56135165-56137165 | 8 | 0.9316 |
| C7 | chr5:40908598-40910598 | 5 | 0.9316 |
| HEPACAM | chr11:124788100-124790100 | 5 | 0.9323 |
| HEPN1 | chr11:124788145-124790145 | 5 | 0.9323 |
| NR1H4 | chr12:100866643-100868643 | 8 | 0.9330 |
| MYO18B | chr22:26137154-26139154 | 11 | 0.9334 |
| SET | chr9:131445071-131447071 | 5 | 0.9335 |
| NHLH1 | chr1:160335860-160337860 | 8 | 0.9339 |
| LOC400622 | chr17:75522082-75524082 | 6 | 0.9350 |
| MIR544A | chr14:101513994-101515994 | 10 | 0.9356 |
| ISG20 | chr15:89177862-89179862 | 6 | 0.9358 |
| SNORD115-2 | chr15:25416781-25418781 | 6 | 0.9361 |
| EMSLR | chr7:100950587-100952587 | 6 | 0.9364 |
| ZAP70 | chr2:98329030-98331030 | 10 | 0.9364 |
| MIR516A1 | chr19:54258994-54260994 | 5 | 0.9366 |
| CERCAM | chr9:131180438-131182438 | 7 | 0.9368 |
| GPA33 | chr1:167021072-167023072 | 6 | 0.9374 |
| ZP1 | chr11:60634014-60636014 | 7 | 0.9376 |
| PRLR | chr5:35047860-35049860 | 9 | 0.9378 |
| PRMT8 | chr12:3489514-3491514 | 5 | 0.9380 |
| EPGN | chr4:75173186-75175186 | 5 | 0.9380 |
| LINC00207 | chr22:44964219-44966219 | 8 | 0.9380 |
| NGF | chr1:115827537-115829537 | 6 | 0.9381 |
| SRGN | chr10:70846358-70848358 | 7 | 0.9382 |
| BLK | chr8:11350895-11352895 | 7 | 0.9389 |
| LOC644656 | chr11:9480102-9482102 | 6 | 0.9392 |
| SNORD116-22 | chr15:25334068-25336068 | 9 | 0.9397 |
| IL36A | chr2:113762035-113764035 | 8 | 0.9398 |
| TM4SF1-AS1 | chr3:149094564-149096564 | 11 | 0.9401 |
| ALDH3B1 | chr11:67775016-67777016 | 8 | 0.9405 |
| PTCRA | chr6:42882726-42884726 | 8 | 0.9406 |
| GALNT9 | chr12:132679916-132681916 | 6 | 0.9410 |
| PRSS48 | chr4:152197324-152199324 | 6 | 0.9411 |
| SNORD115-35 | chr15:25478393-25480393 | 5 | 0.9419 |

(continued)

| Promoter Region | Region Designation | Number of Probes in Region | Standard Deviation Cutoff |
|---|---|---|---|
| NT5DC4 | chr2:113478062-113480062 | 8 | 0.9422 |
| SNORD78 | chr1:173833759-173835759 | 8 | 0.9424 |
| SEBOX | chr17:26690289-26692289 | 5 | 0.9425 |
| MCCD1 | chr6:31495738-31497738 | 14 | 0.9426 |
| SLC25A18 | chr22:18042138-18044138 | 9 | 0.9430 |
| SGCA | chr17:48242365-48244365 | 7 | 0.9430 |
| TEPSIN | chr17:79201076-79203076 | 8 | 0.9432 |
| SEMG2 | chr20:43849013-43851013 | 6 | 0.9453 |
| CAPN11 | chr6:44125556-44127556 | 7 | 0.9453 |
| LINC02222 | chr5:180110095-180112095 | 7 | 0.9470 |
| LY6G5B | chr6:31637727-31639727 | 7 | 0.9473 |
| LAMC2 | chr1:183154398-183156398 | 11 | 0.9475 |
| ADGRG1 | chr16:57652649-57654649 | 13 | 0.9485 |
| RNF103 | chr2:86829515-86831515 | 6 | 0.9491 |
| STAM | chr10:17685149-17687149 | 14 | 0.9491 |
| IQCF5-AS1 | chr3:51906611-51908611 | 5 | 0.9497 |
| KRTAP10-12 | chr21:46116086-46118086 | 7 | 0.9498 |
| OPN5 | chr6:47748774-47750774 | 7 | 0.9499 |
| IRGC | chr19:44219227-44221227 | 5 | 0.9499 |
| PVRIG2P | chr7:99948940-99950940 | 7 | 0.9507 |
| CSN1S2BP | chr4:70998320-71000320 | 5 | 0.9520 |
| RPA4 | chrX:96137906-96139906 | 5 | 0.9524 |
| IPO5 | chr13:98604928-98606928 | 5 | 0.9527 |
| POMC | chr2:25382721-25384721 | 5 | 0.9528 |
| MIR575 | chr4:83673489-83675489 | 6 | 0.9530 |
| OR8S1 | chr12:48918414-48920414 | 6 | 0.9538 |
| BEST2 | chr19: 12861604-12863604 | 11 | 0.9541 |
| PHKA2-AS1 | chrX:18907413-18909413 | 6 | 0.9541 |
| ZNF530 | chr19:58110252-58112252 | 14 | 0.9542 |
| MYMX | chr6:44183662-44185662 | 5 | 0.9543 |
| TM4SF5 | chr17:4674180-4676180 | 9 | 0.9544 |
| GRK5 | chr10:120966082-120968082 | 10 | 0.9550 |
| SNORD127 | chr14:45579077-45581077 | 5 | 0.9551 |
| LINC01780 | chr1:119869874-119871874 | 8 | 0.9552 |
| VPREB1 | chr22:22598197-22600197 | 6 | 0.9555 |
| TOMM20L | chr14:58861647-58863647 | 14 | 0.9560 |
| GOLGA3 | chr12:133344499-133346499 | 10 | 0.9564 |
| ATF7 | chr12:53900639-53902639 | 5 | 0.9572 |
| ZSWIM1 | chr20:44508865-44510865 | 10 | 0.9583 |

(continued)

| Promoter Region | Region Designation | Number of Probes in Region | Standard Deviation Cutoff |
|---|---|---|---|
| ADAMDEC1 | chr8:24240797-24242797 | 7 | 0.9589 |
| TRPM2 | chr21:45772483-45774483 | 11 | 0.9590 |
| MYCNUT | chr2:16059520-16061520 | 5 | 0.9595 |
| CASS4 | chr20:54986091-54988091 | 10 | 0.9595 |
| PIP | chr7:142828169-142830169 | 6 | 0.9597 |
| C16orf92 | chr16:30033654-30035654 | 6 | 0.9610 |
| TAT-AS1 | chr16:71597918-71599918 | 5 | 0.9611 |
| LINC02037 | chr3:193964436-193966436 | 6 | 0.9611 |
| EDN1 | chr6:12289593-12291593 | 10 | 0.9612 |
| FIGNL2 | chr12:52210675-52212675 | 5 | 0.9619 |
| ENHO | chr9:34520040-34522040 | 5 | 0.9622 |
| MIR1199 | chr19: 14183172-14185172 | 11 | 0.9622 |
| LCT-AS1 | chr2:136576760-136578760 | 6 | 0.9628 |
| MIR502 | chrX:49778205-49780205 | 5 | 0.9638 |
| A2ML1 | chr12:8974216-8976216 | 7 | 0.9643 |
| SCARNA9 | chr11:93453679-93455679 | 6 | 0.9645 |
| ASGR1 | chr17:7075749-7077749 | 6 | 0.9647 |
| MIR541 | chr14:101529831-101531831 | 16 | 0.9647 |
| CHRNB3 | chr8:42551508-42553508 | 7 | 0.9648 |
| LOC101928404 | chr1:163130464-163132464 | 5 | 0.9652 |
| LINC02868 | chr1:117235733-117237733 | 5 | 0.9658 |
| LLCFC1 | chr7:142635580-142637580 | 7 | 0.9659 |
| CAV3 | chr3:8774487-8776487 | 7 | 0.9661 |
| SFTPA1 | chr10:81369694-81371694 | 6 | 0.9663 |
| LOC91450 | chr15:78284574-78286574 | 7 | 0.9664 |
| TEX36-AS1 | chr10:127261939-127263939 | 7 | 0.9664 |
| SYT8 | chr11:1854656-1856656 | 11 | 0.9667 |
| NQO1 | chr16:69742303-69744303 | 6 | 0.9673 |
| CYMP | chr1:111022387-111024387 | 5 | 0.9680 |
| HPR | chr16:72096124-72098124 | 7 | 0.9685 |
| VPS72 | chr1:151147779-151149779 | 7 | 0.9687 |
| KLK15 | chr19:51327544-51329544 | 5 | 0.9689 |
| CLN6 | chr15:68498329-68500329 | 7 | 0.9691 |
| RNASE10 | chr14:20977630-20979630 | 6 | 0.9694 |
| HLA-DOA | chr6:32970958-32972958 | 5 | 0.9696 |
| MIR125A | chr19:52195506-52197506 | 6 | 0.9698 |
| AADACL4 | chr1:12703565-12705565 | 7 | 0.9698 |
| MIR767 | chrX:151560892-151562892 | 6 | 0.9703 |
| SNORD79 | chr1:173833487-173835487 | 9 | 0.9703 |

(continued)

| Promoter Region | Region Designation | Number of Probes in Region | Standard Deviation Cutoff |
|---|---|---|---|
| MYO15A | chr17:18011069-18013069 | 8 | 0.9705 |
| NTRK1 | chr1:156784541-156786541 | 8 | 0.9714 |
| SPP1 | chr4:88895801-88897801 | 6 | 0.9714 |
| TANK | chr2:161992465-161994465 | 5 | 0.9714 |
| AGO1 | chr1:36334408-36336408 | 6 | 0.9718 |
| MIR889 | chr14:101513237-101515237 | 9 | 0.9722 |
| TMEM69 | chr1:46152852-46154852 | 10 | 0.9726 |
| CACTIN | chr19:3609642-3611642 | 5 | 0.9727 |
| SLC5A9 | chr1:48687387-48689387 | 5 | 0.9732 |
| ARNT | chr1:150781188-150783188 | 5 | 0.9737 |
| LINC01447 | chr7:47660536-47662536 | 5 | 0.9743 |
| LINC00343 | chr13:106358178-106360178 | 6 | 0.9743 |
| RNU5D-1 | chr1:45195740-45197740 | 5 | 0.9748 |
| LINC02480 | chr4:52909970-52911970 | 5 | 0.9750 |
| MIR199A1 | chr19:10927101-10929101 | 9 | 0.9751 |
| METTL21EP | chr13:103531448-103533448 | 9 | 0.9752 |
| DSCR9 | chr21:38579803-38581803 | 8 | 0.9752 |
| TCN2 | chr22:31002069-31004069 | 17 | 0.9752 |
| LOC100268168 | chr5:172380784-172382784 | 5 | 0.9753 |
| RNASE8 | chr14:21524980-21526980 | 5 | 0.9753 |
| ARPP21 | chr3:35680016-35682016 | 17 | 0.9753 |
| ZC3H7B | chr22:41696528-41698528 | 12 | 0.9756 |
| WFIKKN2 | chr17:48910944-48912944 | 22 | 0.9762 |
| OR6V1 | chr7:142748437-142750437 | 7 | 0.9762 |
| IBSP | chr4:88719705-88721705 | 7 | 0.9767 |
| FERMT3 | chr11:63973151-63975151 | 15 | 0.9770 |
| SNORD116-21 | chr15:25332949-25334949 | 7 | 0.9774 |
| ANKRD2 | chr10:99331197-99333197 | 10 | 0.9776 |
| SPTY2D1OS | chr11:18620335-18622335 | 6 | 0.9780 |
| CTSE | chr1:206316472-206318472 | 8 | 0.9788 |
| LRRTM4-AS1 | chr2:77212090-77214090 | 5 | 0.9789 |
| LOC101928618 | chr7:36133919-36135919 | 5 | 0.9790 |
| HTR2C | chrX:113817550-113819550 | 8 | 0.9791 |
| SQOR | chr15:45922345-45924345 | 6 | 0.9792 |
| MIR506 | chrX:146311237-146313237 | 5 | 0.9792 |
| SFT2D3 | chr2:128458070-128460070 | 13 | 0.9793 |
| MAGEE1 | chrX:75647102-75649102 | 12 | 0.9794 |
| SNORD113-6 | chr14:101404892-101406892 | 10 | 0.9799 |
| MIR655 | chr14:101514886-101516886 | 7 | 0.9800 |

(continued)

| Promoter Region | Region Designation | Number of Probes in Region | Standard Deviation Cutoff |
|---|---|---|---|
| SPEM1 | chr17:7322642-7324642 | 12 | 0.9802 |
| MIA2 | chr14:39702118-39704118 | 5 | 0.9811 |
| CEACAM21 | chr19:42054885-42056885 | 5 | 0.9816 |
| H3C8 | chr6:26270145-26272145 | 11 | 0.9816 |
| CYTH4 | chr22:37677548-37679548 | 11 | 0.9819 |
| SMIM17 | chr19:57153526-57155526 | 9 | 0.9823 |
| SYTL3 | chr6:159070045-159072045 | 8 | 0.9828 |
| DNAJB8 | chr3:128180279-128182279 | 6 | 0.9832 |
| PILRA | chr7:99970109-99972109 | 8 | 0.9833 |
| ADCY6 | chr12:49158976-49160976 | 5 | 0.9837 |
| PHLDB2 | chr3:111450342-111452342 | 7 | 0.9838 |
| PROZ | chr13:113811961-113813961 | 9 | 0.9839 |
| RAB3C | chr5:57876978-57878978 | 13 | 0.9840 |
| OR1M1 | chr19:9202920-9204920 | 7 | 0.9845 |
| DMRTA1 | chr9:22445822-22447822 | 8 | 0.9848 |
| IL37 | chr2:113669547-113671547 | 6 | 0.9851 |
| LOC339260 | chr17:20840878-20842878 | 10 | 0.9852 |
| HGFAC | chr4:3442694-3444694 | 12 | 0.9856 |
| DAPL1 | chr2:159650828-159652828 | 5 | 0.9858 |
| ASB15 | chr7:123240874-123242874 | 11 | 0.9858 |
| ELMO1-AS1 | chr7:37036400-37038400 | 5 | 0.9858 |
| FAM25A | chr10:88779050-88781050 | 6 | 0.9861 |
| AQP4-AS1 | chr18:24444271-24446271 | 6 | 0.9863 |
| CYP2C18 | chr10:96442485-96444485 | 7 | 0.9865 |
| LCN6 | chr9:139637468-139639468 | 6 | 0.9865 |
| LOC643072 | chr2:160470804-160472804 | 9 | 0.9867 |
| LINC02395 | chr12:50304735-50306735 | 7 | 0.9868 |
| LTC4S | chr5:179219986-179221986 | 8 | 0.9868 |
| TAS2R3 | chr7:141462896-141464896 | 6 | 0.9872 |
| MIR139 | chr11:72325106-72327106 | 6 | 0.9873 |
| RNF7 | chr3:141456050-141458050 | 11 | 0.9877 |
| GRM3 | chr7:86272224-86274224 | 11 | 0.9890 |
| SERPINB11 | chr18:61368540-61370540 | 7 | 0.9891 |
| ETFBKMT | chr12:31799093-31801093 | 7 | 0.9902 |
| MRM1 | chr17:34957011-34959011 | 12 | 0.9904 |
| DIO1 | chr1:54358859-54360859 | 9 | 0.9904 |
| SNORD115-13 | chr15:25437467-25439467 | 10 | 0.9904 |
| PLEKHG7 | chr12:93129264-93131264 | 9 | 0.9909 |
| NPEPL1 | chr20:57263186-57265186 | 8 | 0.9910 |

(continued)

| Promoter Region | Region Designation | Number of Probes in Region | Standard Deviation Cutoff |
|---|---|---|---|
| CBX4 | chr17:77805954-77807954 | 7 | 0.9912 |
| CRISPLD2 | chr16:84852590-84854590 | 9 | 0.9916 |
| LGMN | chr14:93169153-93171153 | 5 | 0.9917 |
| CST7 | chr20:24928904-24930904 | 8 | 0.9918 |
| LINC01816 | chr2:70350167-70352167 | 6 | 0.9930 |
| MTCL1 | chr18:8716378-8718378 | 10 | 0.9934 |
| GKN1 | chr2:69200704-69202704 | 5 | 0.9941 |
| MEP1A | chr6:46760125-46762125 | 7 | 0.9941 |
| MOSMO | chr16:22018431-22020431 | 11 | 0.9944 |
| FBLIM1 | chr1:16082132-16084132 | 6 | 0.9949 |
| MIRLET7C | chr21:17911147-17913147 | 6 | 0.9952 |
| TMEM33 | chr4:41936445-41938445 | 12 | 0.9956 |
| PLA2G2F | chr1:20464815-20466815 | 8 | 0.9956 |
| SNORD115-40 | chr15:25487760-25489760 | 8 | 0.9957 |
| POU6F2 | chr7:39016508-39018508 | 8 | 0.9960 |
| RBM8A | chr1:145506556-145508556 | 18 | 0.9961 |
| CROCC | chr1:17247425-17249425 | 11 | 0.9967 |
| CLDN34 | chrX:9934397-9936397 | 7 | 0.9967 |
| ABI3 | chr17:47286588-47288588 | 17 | 0.9971 |
| P2RX3 | chr11:57104825-57106825 | 7 | 0.9972 |
| C3orf36 | chr3:133645988-133647988 | 6 | 0.9977 |
| TMEM72 | chr10:45405763-45407763 | 10 | 0.9979 |
| NR2F1 | chr5:92917927-92919927 | 12 | 0.9979 |
| NDST2 | chr10:75560673-75562673 | 6 | 0.9983 |
| DUSP27 | chr1:167062311-167064311 | 8 | 0.9991 |
| CREB3L3 | chr19:4152627-4154627 | 10 | 0.9991 |
| PDZD3 | chr11:119055183-119057183 | 9 | 0.9991 |
| LMOD2 | chr7:123294919-123296919 | 5 | 0.9995 |
| LBP | chr20:36973884-36975884 | 7 | 0.9995 |
| C18orf63 | chr18:71982073-71984073 | 11 | 0.9996 |
| CPNE6 | chr14:24539045-24541045 | 14 | 1.0018 |
| MRAP2 | chr6:84742490-84744490 | 11 | 1.0018 |
| C3orf20 | chr3:14715647-14717647 | 7 | 1.0019 |
| MIR216A | chr2:56215084-56217084 | 6 | 1.0020 |
| CTRB1 | chr16:75251885-75253885 | 10 | 1.0021 |
| OR5V1 | chr6:29322006-29324006 | 6 | 1.0026 |
| CDHR3 | chr7:105602709-105604709 | 6 | 1.0029 |
| KCNA5 | chr12:5152044-5154044 | 9 | 1.0032 |
| TRIM38 | chr6:25962029-25964029 | 8 | 1.0036 |

(continued)

| Promoter Region | Region Designation | Number of Probes in Region | Standard Deviation Cutoff |
|---|---|---|---|
| LOC100287329 | chr6:31526347-31528347 | 8 | 1.0042 |
| SLC39A5 | chr12:56622834-56624834 | 7 | 1.0049 |
| SLC22A18 | chr11:2919920-2921920 | 16 | 1.0052 |
| ARHGAP5-AS1 | chr14:32543624-32545624 | 5 | 1.0052 |
| PLA2G4B | chr15:42130010-42132010 | 6 | 1.0077 |
| S100A1 | chr1:153599909-153601909 | 13 | 1.0081 |
| LINC01684 | chr21:25800053-25802053 | 6 | 1.0082 |
| GABPB2 | chr1:151042236-151044236 | 13 | 1.0083 |
| EMP1 | chr12:13348659-13350659 | 6 | 1.0089 |
| OR7C2 | chr19:15051300-15053300 | 9 | 1.0094 |

**Table 6: Genbank Assembly Information for HG19 (GRCh37)**

| | |
|---|---|
| **Description** | Genome Reference Consortium Human Build 37 (GRCh37) also known as HG19 |
| **Organism name** | Homo sapiens (human) |
| **BioProject Number** | PRJNA31257 |
| **Submitter** | Genome Reference Consortium |
| **Date** | 2/27/2009 |
| **GenBank assembly accession** | GCA_000001405.1 |
| **RefSeq assembly accession** | GCF_000001405.25 |

In some embodiments, the average standard deviation of methylation of an individual promoter of a sample can be greater than, or greater than or equal to one, two, three, four, five, or more standard deviations from a reference or control sample standard deviation of methylation. In some embodiments, the average standard deviation of methylation of an individual promoter of a sample can be greater than, or greater than or equal to three or more standard deviations from a reference or control sample standard deviation of methylation. In some cases, the average standard deviation of methylation of an individual promoter can be greater than or greater than or equal to the standard deviation cutoff in **Table 1.**

In some cases, infertility or diminished infertility of a subject can be associated with promoter dysregulation, for example an average standard deviation methylation value of a promoter from a sample being greater than or greater than or equal to the standard deviation cutoff value in **Table 1,** for a promoter of **Table 1.** In some cases, infertility or diminished infertility of a subject can be associated with: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more than 100 dysregulated promoters from **Table 1.** In some cases, infertility or diminished infertility of a subject can be associated with 22 or more dysregulated promoters from **Table 1.** In some instances, the average standard deviation of methylation of an individual promoter can be greater than, or greater than or equal to three standard deviations of methylation of a reference sample and can be independently determined in 22 or more different promoters.

In some cases, infertility or diminished infertility can be determined in an assay comprising detecting promoter dysregulation in the 1233 promoters of **Table 1 or** a portion of the 1233 promoters of **Table 1,** wherein in some cases, a portion of the 1233 promoters can comprise about: 1, 2, 3, 4, 5, 6, 7, 8, 9, 10, 11, 12, 13, 14, 15, 16, 17, 18, 19, 20, 21, 22, 23, 24, 25, 50, 100, 200, 300, 400, 500, 600, 700, 800, 900, 1000, 1100, or 1200 promoters from **Table 1.**

In some cases, promoter dysregulation can comprise dysregulation in one or more promoters from **Table 1.** In some cases, promoter dysregulation can comprise dysregulation in any promoter from **Table 1.** In some cases, promoter dysregulation can comprise dysregulation in 1, 2, 3, 4, 5, 6, 7, 8, 9, 10, 11, 12, 13, 14, 15, 16, 17, 18, 19 or all 20 of the following gene promoters: ACTR5, ASGR1, CALML6, SARS1, HSD17B7, H3C8, ABHD17A, VPS28, SCARNA9, AQP10, NAE1, GRAMD1A, KCNU1, TSPAN16, PGBD4, LAMC2, GUSBP1, ITIH1, HSH2D, TBC1D26.

In some cases, fertility of a subject can be associated with reduced promoter dysregulation. In some cases, reduced promoter dysregulation can be determined in the 1233 promoters of **Table 1** or a portion of the 1233 promoters of **Table 1** wherein, in some cases, a portion of the 1233 promoters can comprise about: 1, 2, 3, 4, 5, 6, 7, 8, 9, 10, 11, 12, 13, 14, 15, 16, 17, 18, 19, 20, 21, 22, 23, 24, 25, 50, 100, 200, 300, 400, 500, 600, 700, 800, 900, 1000, 1100, or 1200 promoters from **Table 1**. In some cases, fertility of a subject can be determined by a sample having promoter dysregulation in less than 22 different promoters of **Table 1,** when the average standard deviation for methylation of all promoters of **Table 1** are determined. For example, fertility of a subject can be determined when 21, 20, 19, 18, 17, 16, 15, 14, 13, 12, 11, 10, 9, 8, 7, 6, 5, 4, 3, 2, 1, or 0 promoters of the 1233 promoters are determined to be dysregulated. In some cases, fertility of a subject can be determined when 21, 20, 19, 18, 17, 16, 15, 14, 13, 12, 11, 10, 9, 8, 7, 6, 5, 4, 3, 2, 1, or 0 promoters of a portion of the 1233 promoters are determined to be dysregulated. In some cases, promoter dysregulation can be the average standard deviation methylation value of a promoter from a sample being greater than or greater than or equal to the standard deviation cutoff value in **Table 1**. In some cases, promoter dysregulation can be the average standard deviation methylation value of a promoter from a sample being greater than or greater than or equal to three standard deviations from a reference standard deviation of methylation cutoff value. In some cases, the average standard deviation of an individual promoter can be greater than, or greater than or equal to three standard deviations of methylation of a reference promoter and can be independently determined in less than 22 different promoters. In some cases, the average standard deviations for methylation of the individual promoters are determined in 1233 different promoters.

In some embodiments, the reference standard deviation of methylation for a promoter is derived from a fertile subject or a plurality of fertile subjects. In some cases, a reference standard deviation of methylation for a promoter is derived from an infertile subject or a plurality of infertile subjects. In some cases, the reference standard deviation of methylation is a control standard deviation of methylation. In some cases, a control standard deviation of methylation is a reference standard deviation of methylation.

In some cases, a method can further comprise a semen analysis (e.g., a seminogram or spermiogram). In some cases, a method can further comprise determining a) a morphological characteristic, b) a motility characteristic, c) a concentration, or d) any combination thereof of the sperm. In some cases, a semen analysis can comprise determining one or more of the following characteristics: a sperm count, a motility, a morphology, a volume, an appearance, a fructose level, a pH, a liquefaction, a viscosity, a motile total (MOT), a DNA damage, a total motile spermatozoa, or any combination thereof. In some cases, a method herein can comprise a combination analysis comprising the methylation analysis described herein and a semen analysis. The parameters for semen analysis are determined from a reference standard such as the World Health Organization (WHO) reference standards.

In some embodiments, a method or system can comprise determining independently a standard deviation for methylation in each of the at least 5 regions of an individual promoter. In some cases, a method or system can comprise determining independently a standard deviation for methylation in each of the probed regions of **Table 1** of an individual promoter. In some cases, a method or system can comprise determining independently a standard deviation for methylation in each of the 22, 21, 20, 19, 18, 17, 16, 15, 14, 13, 12, 11, 10, 9, 8, 7, 6, 5, 4, 3, 2, or 1 probe regions of an individual promoter. In some cases, a method or system can comprise determining independently a standard deviation for methylation in each of the 22, 21, 20, 19, 18, 17, 16, 15, 14, 13, 12, 11, 10, 9, 8, 7, 6, and/or 5 probe regions of **Table 1**. In some cases, a method or system can comprise determining independently a standard deviation for methylation in 22, 21, 20, 19, 18, 17, 16, 15, 14, 13, 12, 11, 10, 9, 8, 7, 6, 5, 4, 3, 2, or 1 probe regions of **Table 1**. In some cases, determining independently the standard deviation for methylation in each of the at least 5 regions of the individual promoter employs a computer processor. In some cases, the determining can employ a computer processor operably connected to a computer memory. In some cases, the determining can employ a computer program executed on a computer.

In some embodiments, a region of a promoter can independently be from: about 10 contiguous nucleobases to about 100 contiguous nucleobases; about 20 contiguous nucleobases to about 100 contiguous nucleobases; about 30 contiguous nucleobases to about 100 contiguous nucleobases; about 40 contiguous nucleobases to about 100 contiguous nucleobases; about 50 contiguous nucleobases to about 100 contiguous nucleobases; about 60 contiguous nucleobases to about 100 contiguous nucleobases; about 70 contiguous nucleobases to about 100 contiguous nucleobases; about 80 contiguous nucleobases to about 100 contiguous nucleobases; about 90 contiguous nucleobases to about 100 contiguous nucleobases; about 20 contiguous nucleobases to about 50 contiguous nucleobases; about 30 contiguous nucleobases to about 50 contiguous nucleobases. In some embodiments, a region of a promoter can be about 50 contiguous nucleobases. In some embodiments, a region of a promoter can be about: 40, 41, 42, 43, 44, 45, 46, 47, 48, 49, 50, 51, 52, 53, 54, 55, 56, 57, 58, 59, or 60 contiguous nucleobases.

In some embodiments, a method or a system can comprise calculating an average standard deviation for methylation of an individual promoter from a standard deviation of methylation in each of the at least 5 regions of an individual promoter. In some cases, a method or a system can comprise calculating an average standard deviation for methylation of an individual promoter from a standard deviation of methylation in each of the at least 5 probed regions of an individual promoter of **Table 1**. In some cases, a method or a system can comprise calculating an average standard deviation for methylation of an individual promoter from a standard deviation of methylation in each of the 22, 21, 20, 19, 18, 17, 16, 15, 14, 13, 12, 11, 10, 9, 8, 7, 6, and/or 5 probe regions of an individual promoter. In some cases, a method or a system can comprise calculating an average standard deviation for methylation of an individual promoter from a standard deviation of methylation in each of the 22, 21, 20, 19, 18, 17, 16, 15, 14, 13, 12, 11, 10, 9, 8, 7, 6, 5, 4, 3, or 2 probe regions of an individual promoter. In some cases, a method or a system can comprise calculating an average standard deviation for methylation of an individual promoter from a standard deviation of methylation in each of the 22, 21, 20, 19, 18, 17, 16, 15, 14, 13, 12, 11, 10, 9, 8, 7, 6, and/or 5 probe regions of **Table 1.** In some cases, the calculating the average standard deviation for methylation of the individual promoter from the standard deviation of methylation in each of the at least 5 regions of the individual promoter employs a computer processor. In some cases, the calculating can employ a computer processor operably connected to a computer memory. In some cases, the calculating can employ a computer program executed on a computer.

In some embodiments, a method or system can comprise determining if an average standard deviation for methylation of the individual promoter from a sample is greater than, or granter than or equal to three standard deviations from a reference standard deviation of methylation for the individual promoter. In some cases, a method or system can comprise determining if an average standard deviation for methylation of the individual promoter from a sample is greater than, or granter than or equal to: one, two, three, four, five, or more than five standard deviations from a reference standard deviation of methylation for the individual promoter. In some cases, a method or system can comprise determining if an average standard deviation for methylation of the individual promoter from a sample is greater than, or granter than or equal to the standard deviation cutoff value from **Table 1.** In some cases, the determining if the average standard deviation for methylation of the individual promoter is greater than, or greater than or equal to three standard deviations from a reference standard deviation of methylation for the promoter employs a computer processor. In some cases, the determining can employ a computer processor operably connected to a computer memory. In some cases, the determining can employ a computer program executed on a computer.

In some cases, a method herein can comprise performing a treatment on the subject. In some cases, the subject can be a subject in need of a treatment. In some cases, the treatment comprises *in vitro* fertilization (IVF) or intrauterine insemination (IUI).

Also described herein are methods comprising: a) obtaining a biological sample from a male subject, wherein the biological sample comprises seminal fluid; b) extracting DNA from a sperm in the biological sample, extracting cell free DNA from the biological sample, or both; c) detecting in an *in vitro* analytical assay, methylation present in at least 5 regions of an individual promoter comprised in the extracted DNA, the extracted cell free DNA, or both from the biological sample, wherein the promoter is selected from **Table 1**; d) calculating an average standard deviation for methylation of the individual promoter from the standard deviation of methylation in each of the at least 5 regions of the individual promoter; and e) determining if the average standard deviation for methylation of the individual promoter is greater than, or greater than or equal to three standard deviations from a reference standard deviation of methylation for the individual promoter.

Also described herein are methods comprising obtaining a biological sample from a male subject, wherein the biological sample comprises seminal fluid. In some cases, a method can comprise extracting DNA from a sperm in the biological sample, extracting cell free DNA from the biological sample, or both. In some cases, a method can comprise detecting in an *in vitro* analytical assay, methylation present in at least 5 regions of an individual promoter comprised in the extracted DNA, the extracted cell free DNA, or both from the biological sample. In some cases, the individual promoter can be selected from or is included in the promoters from **Table 1.** In some cases, a method can comprise determining, with a computer program executed on a computer, a standard deviation for methylation in each of the at least 5 regions of the individual promoter. In some cases, a method can comprise calculating, with the computer program executed on the computer, an average standard deviation for methylation of an individual promoter from the standard deviation of methylation in each of the at least 5 regions of the individual promoter. In some cases, a method can comprise determining, with the computer program executed on the computer, if the average standard deviation for methylation of the individual promoter is greater than, or greater than or equal to three standard deviations from a reference standard deviation of methylation for the individual promoter.

**Determining Promoter Methylation Variability**

In some cases, calculating the average standard deviation for methylation of an individual promoter from the standard deviation of methylation in each of the at least 5 regions of the individual promoter can be calculated by following equation:

$$\sigma = \sqrt{\frac{\sum |x_1 - \mu|^2}{N}}$$

,

wherein $\sigma$ = the average standard deviation for methylation of the individual promoter (e.g., the methylation variability value), $x_1$ = an m-value of a given methylation array probe in the individual promoter, N = the total number of methylation probes of the individual promoter, and $\mu$= a mean of probe m-values in the individual promoter. In some cases, independently, on a promoter by promoter basis, N can be 1, 2, 3, 4, 5, 6, 7, 8, 9, 10, 11, 12, 13, 14, 15, 16, 17, 18, 19, 20, 21, 22, 23, 24 or 25. In some cases, Beta values are described as (methylated probe intensity / [methylated + unmethylated probe intensity + 100]) and range from 0-1 with values around 0 being unmethylated and values around 1 being methylated. In some cases, M-values are described as (log(methylated probe intensity / unmethylated probe intensity) and are useful measures of methylation to prevent bias arising from heteroscedasticity seen when analyzing beta values.

In some cases, the promoter variability threshold (*e.g.*, 3 or greater than 3 standard deviations from a reference promoter average standard deviation for methylation) of a reference or control sample can be determined by the following equation:

$$\theta = 1.1\left(\frac{\sum \sigma_1}{N} + 3\sqrt{\frac{\sum |\sigma_1 - \mu|^2}{N}}\right)$$

,

wherein $\theta$ = promoter variability threshold, $\sigma 1$ = the average standard deviation for methylation of a sample at a given promoter, $\mu$= mean of the methylation variability values of a given promoter, and N = the number of samples.

In some cases, the promoter variability threshold of an individual promoter can be determined by: (mean(donor standard deviations of promoter) + standard deviation (donor standard deviations of promoter)*3)*1.1.

In some cases, promoter dysregulation can be determined when an average standard deviation for methylation of an individual promoter is greater than, or greater than or equal to the promoter variability threshold of the individual promoter of a reference sample. In some cases, promoter dysregulation can be determined when an average standard deviation for methylation of an individual promoter of a sample is 3, or greater than 3 standard deviations greater than, or greater than or equal to the promoter variability of the individual promoter of a reference sample.

**Biological Samples**

In some embodiments, a biological sample herein can comprise a blood sample, a seminal fluid sample, a urine sample, a tissue sample, a biological fluid sample, or a semen sample. In some cases, a biological sample can comprise a DNA sample. In some instances, a DNA sample can be a DNA sample extracted from one or more cells, a cell free DNA sample, or a combination thereof. In some cases, a biological sample herein can comprise a pure sperm sample, a tissue sample, a cellular sample, a cell free DNA (cfDNA), or a cell free RNA (cfRNA) sample. In some cases, a cfDNA sample can comprise a cfDNA sample from a blood sample, a seminal fluid sample, a semen sample, a tissue sample, a urine sample, or a mixture thereof. In some cases, cfDNA can comprise DNA from a cell, for example a sperm cell. In some cases, a cellular sample can comprise the cells of a biological sample. In some cases, a biological sample can be treated with an enzyme such as DNase, RNase or a mixture thereof to remove cell free DNA and/or cell free RNA. In some cases, a biological sample herein is obtained from a male subject. In some cases, a biological sample herein can be obtained from a subject who has diminished fertility or who is infertile. In some cases, a biological sample herein can be obtained from a subject who is fertile.

In some cases, a biological sample herein can be obtained from the male reproductive system. In some cases, a biological sample can be obtained from an external urethral orifice, a glans penis, a penis, a urethra, a corpus spongiosum, a corpus cavernosum, a deep perineal pouch, a suspensory ligament, a urinary bladder, a vas deferens, a seminal vesicle, an ejaculatory duct, a prostate gland, a bulbourethral gland, an epididymis, a testis, a spermatic cord, a scrotum, or any combination thereof. In some cases, a biological sample can be obtained from a testis. In some cases, a testis can comprise a rete testis, a tunica albuginea, a seminiferous tubule, a lobule, a scrotum, a tunica vaginalis, a vas deferens, or any combination thereof. In some cases, a method can comprise obtaining a biological sample from a region of the male reproductive system and detecting the presence of sperm.

## Kits and Arrays

Also disclosed herein are kits comprising a primer, a probe, or a combination thereof. In some cases, a kit can comprise a container. A container can be in the form of a glass, a metal, a plastic or any solid container. In some instances, a kit can comprise instructions for use. In some cases, an array, a primer, a probe, or any combination thereof can be used for the manufacture of a diagnostic reagent or kit for determining the presence or absence of dysregulated promoters of DNA in a biological sample. In some instances, a kit can comprise instructions for use. In some cases, an array, a primer, a probe, or any combination thereof can be used for the manufacture of a diagnostic reagent or kit for determining the number of dysregulated promoters in a biological sample. In some instances, described herein is the use of arrays, primers, probes, or combination thereof for determining promoter dysregulation from DNA extracted from biological samples for the manufacture of a diagnostic kit. In some cases, a kit can comprise an array for detecting methylation variability of the promoter regions disclosed herein. In some cases, a diagnostic kit can be employed for determining a male infertility.

Also described herein is the use of an array used in detecting DNA methylation in at least 22 promoters selected from **Table 1** from DNA obtained from a sperm cell, cell free DNA in a seminal sample, or both. Also described herein is the use of an array used in detecting DNA methylation in all the promoters from **Table 1.** In some cases, DNA methylation can be the variability of methylation of the individual promoters from **Table 1,** for example the standard deviation of the methylation of the individual promoters from **Table 1.** In some cases, the DNA methylation can be determined independently in at least 5 regions of an individual promoter for the manufacture of a diagnostic kit for determining male infertility of a human male subject. In some embodiments, the use further comprises: a) determining independently a standard deviation for methylation in each of the at least 5 regions of the individual promoter; b) calculating an average standard deviation for methylation of the individual promoter from the standard deviation of methylation in each of the at least 5 regions of the individual promoter; and c) determining if the average standard deviation for methylation of the individual promoter is greater than, or greater than or equal to three standard deviations from a reference standard deviation of methylation for the individual promoter. In some cases, the determining from a) and c) and the calculating from b) can employ a computer processor configured to run a computer program.

## Methods of treatment

Also disclosed herein are methods of treatment. In some cases, a method described herein, such as identifying dysregulated promoters from the sperm of a subject, can further comprise treating the subject. In some cases, a method can comprise identifying one or more dysregulated promoters of a subject's sperm and treating the subject for infertility. In some cases, a method can comprise identifying 22 or more dysregulated promoters of a subject's sperm and treating the subject for infertility. In some case, treating the subject can comprise treating the subject with a treatment for infertility. In some cases, a subject who is infertile can comprise a subject who has diminished fertility as compared to a subject who is fertile. A subject with diminished fertility can comprise a subject whose sperm has an abnormality such as decreased motility and/or comprises a dysregulated promoter.

In some cases, *in vitro* fertilization (IVF) can be used to treat male infertility. In some cases, an assisted reproductive technique, such as intrauterine insemination (IUI) can be used to treat male infertility. In some cases, an assisted reproductive technique can comprise *in vitro* fertilization-embryo transfer (IVF-ET), gamete intrafallopian transfer (GIFT), zygote intrafallopian transfer (ZIFT), pronuclear stage tubal transfer (PROST), frozen embryo transfer (FET), intracytoplasmic sperm injection (ICSI), or any combination thereof. In some cases, a treatment for infertility can comprise treatment with an antibiotic. In some cases, a treatment for infertility can comprise a treatment for erectile dysfunction such as sildenafil, avanafil, tadalafil, vardenafil, a salt of any of these, or any combination thereof. In some cases, a treatment for erectile dysfunction can comprise a vacuum erection device (VED), a testosterone replacement, a urethral suppository, a penile injection, a penile implant, or any combination thereof. In some cases, a treatment can comprise treatment with human chorionic gonadotropin, recombinant human follicle-stimulating hormone, or both. In some cases, a treatment can comprise the concentration of sperm. In some cases, a treatment for infertility can comprise a surgery or a hormone treatment. In some cases, a surgery can comprise surgical repair of the varicocele. In some cases, a treatment for infertility can comprise concentration of sperm from the subject. In some cases, a treatment for azoospermia can comprise a surgery. In some cases, a treatment for infertility can comprise a vasectomy reversal, a microsurgical testicular sperm extraction (microTESE), a testicular sperm extraction (TESE) a transurethral resection of the ejaculatory ducts (TURED), a circumcision, a surgical correction for scarring, or any combination thereof. In some cases, a treatment for infertility can comprise treatment with clomiphene citrate, clomid, prazosin, phenoxybenzamine, anastrazole, arimidex, a salt of any of these, or any combination thereof. In some cases, a treatment can comprise a vibratory stimulation. In some cases, a treatment for infertility can comprise an aromatase inhibitor (*e.g.*, anastrozole, letrozole, testolactone), an dopamine agonist (*e.g.*, cabergoline), a selective estrogen receptor modulator (SERM) (*e.g.*, clomiphene citrate, tamoxifen, toremifene, raloxifene), a salt of any of these or any combination thereof. In some cases, a hormone treatment can comprise GnRH, human chorionic-gonadotropin (hCG), human menopausal gonadotropin (hMG), recombinant human follicle-stimulating hormone (rhFSH), or any combination thereof.

Methods disclosed herein can be used to treat azoospermia. In some cases, azoospermia can comprise obstructive azoospermia, non-obstructive azoospermia, or both. In some cases, non-obstructive azoospermia can comprise pretesticular non-obstructive azoospermia or testicular non-obstructive azoospermia. In some cases, pretesticular non-obstructive azoospermia can be caused by a hypogonadotropic hypogonadism, a hypothyroidism, use of certain medications, an elevated estradiol, Kallman's syndrome, a pituitary tumor, or a combination thereof. In some cases, testicular non-obstructive azoospermia can be caused by varicoceles, bilateral undescended testicles, cyptorchidism, testicular cancer, gonadotoxins, immunologic cause, Sertoli-cell only syndrome, incomplete development, a genetic syndrome, or a combination thereof. In some cases, obstructive azoospermia can comprise a vasectomy, a cystic fibrosis, an ejaculatory duct obstruction, a surgical complication, a phimosis, a scarring (*e.g.*, from a sexually transmitted infection or an injury that causes scarring), a midline congenital cyst, or any combination thereof. In some cases, methods herein can be used to treat an ejaculatory disorder, a sperm production disorder, a bladder neck obstruction, a varicocele disorder.

In some cases, the methods herein can be used to inform a practitioner the likelihood of the success of a treatment. For example, the identification of dysregulated promoters in sperm could inform the practitioner that a specific therapy could be used to treat the subject.

## Administration

In some embodiments, methods described herein can comprise administering a therapy (*e.g.*, treatment) to a subject in need thereof, for example a subject in need thereof can be a subject suffering from infertility.

In some embodiments, the terms "administer," "administering", "administration," and the like, as used herein, can refer to methods that can be used to deliver therapies described herein. In some cases, delivery can include injection, inhalation, catheterization, gastrostomy tube administration, intravenous administration, intraosseous administration, ocular administration, otic administration, topical administration, transdermal administration, oral administration, rectal administration, nasal administration, intravaginal administration, intracavernous administration, intracerebral administration, transurethral administration, buccal administration, sublingual administration, intrapenile drug delivery, subcutaneous administration, or a combination thereof. Delivery can include a parenchymal injection, an intra-thecal injection, an intra-ventricular injection, or an intra-cisternal injection. A therapy provided herein can be administered by any method. In some cases, a medical professional can administer the therapy described herein. In some cases, a medical professional can comprise a urologist or a reproductive endocrinologist. In some cases, a method herein can comprise diagnosing the subject with a disease or condition such as infertility.

Administration of a therapy disclosed herein can be performed for a treatment duration of at least about: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100, 200, 300, 400, or more consecutive or nonconsecutive days. In some cases, a treatment duration can be from about: 1 to about 30 days, 1 to about 60 days, 1 to about 90 days, 30 days to about 90 days, 60 days to about 90 days, 30 days to about 180 days, from 90 days to about 180 days, or from 180 days to about 360 days.

Administration or application of a therapy disclosed herein can be performed for a treatment duration of at least about 1 week, at least about 2 weeks, at least about 3 weeks, at least about 4 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 1 year, at least about 2 years, or for life. In some embodiments, administering can be performed for about: 1 day to about 8 days, 1 week to about 5 weeks, 1 month to about 12 months, or 1 year to about 3 years.

Administration can be performed repeatedly over a lifetime of a subject, such as once a month or once a year for the lifetime of a subject.

In some cases, administration can comprise administering a second therapy to a subject. In some cases, a second therapy can be administered concurrently or consecutively with a first therapy. In some cases, a second therapy can be any therapy or treatment disclosed herein.

Administration or application of a therapy disclosed herein can be performed at least 1, 2, 3, 4, 5, 6, 7, 8, 9, 10, 11, 12, 13, 14, 15, 16, 17, 18, 19, 20, 21, 22, 23, or 24 times a in a 24 hour period. In some cases, administration or application of a therapy disclosed herein can be performed continuously throughout a 24 hour period. In some cases, administration or application of a therapy disclosed herein can be performed at least 1, 2, 3, 4, 5, 6, 7, 8, 9, 10, or more times a week. In some cases, administration or application of a therapy disclosed herein can be performed at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more times a month. In some cases, a therapy can be administered as a single dose or as divided doses. In some cases, a therapy described herein can be administered at a first time point and a second time point.

In some cases, a therapy herein can be administered at a dose of about 0.0001 grams to about 1000 grams. In some cases, a therapy herein can be administered at a dose of about 1 mg to about 1 gram. In some cases, a therapy herein can be administered at a dose of about: 10 μg, 100 μg, 500 μg 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, or 500 mg. In some cases, a therapy herein can be a pharmaceutical composition. In some cases, a therapy or a pharmaceutical composition can be in unit dose form.

In some cases, a therapy can be administered with an excipient, a carrier, a diluent or any combination thereof. In some cases, a carrier, a diluent, or both, can comprise water, saline, or any pharmaceutically acceptable carrier and/or diluent. In some cases, a diluent can comprise a pH buffer.

In some cases, an excipient can comprise a pharmaceutically acceptable excipient. In some cases, a pharmaceutically acceptable excipient can comprise acacia, acesulfame potassium, acetic acid, glacial, acetone, acetyl tributyl citrate, acetyl triethyl citrate, agar, albumin, alcohol, alginic acid, aliphatic polyesters, alitame, almond oil, alpha tocopherol, aluminum hydroxide adjuvant, aluminum oxide, aluminum phosphate adjuvant, aluminum stearate, ammonia solution, ammonium alginate, ascorbic acid, ascorbyl palmitate, aspartame, attapulgite, bentonite, benzalkonium chloride, benzethonium chloride, benzoic acid, benzyl alcohol, benzyl benzoate, boric acid, bronopol, butylated hydroxyanisole, butylated hydroxytoluene, butylparaben, calcium alginate, calcium carbonate, calcium phosphate, dibasic anhydrous, calcium phosphate, dibasic dihydrate, calcium phosphate, tribasic, calcium stearate, calcium sulfate, canola oil, carbomer, carbon dioxide, carboxymethylcellulose calcium, carboxymethylcellulose sodium, carrageenan, castor oil, castor oil, hydrogenated, cellulose (*e.g.* microcrystalline, powdered, silicified microcrystalline, acetate, acetate phthalate) ceratonia, cetostearyl alcohol, cetrimide, cetyl alcohol, cetylpyridinium chloride, chitosan, chlorhexidine, chlorobutanol, chlorocresol, chlorodifluoroethane, chlorofluorocarbons, chloroxylenol, cholesterol, citric acid monohydrate, colloidal silicon dioxide, coloring agents, copovidone, corn oil, cottonseed oil, cresol, croscarmellose sodium, crospovidone, cyclodextrins, cyclomethicone, denatonium benzoate, dextrates, dextrin, dextrose, dibutyl phthalate, dibutyl sebacate, diethanolamine, diethyl phthalate, difluoroethane, dimethicone, dimethyl ether , dimethyl phthalate , dimethyl sulfoxide , dimethylacetamide , disodium edetate , docusate sodium , edetic acid, erythorbic acid, erythritol, ethyl acetate, ethyl lactate, ethyl maltol, ethyl oleate, ethyl vanillin, ethylcellulose, ethylene glycol palmitostearate, ethylene vinyl acetate, ethylparaben, fructose, fumaric acid, gelatin, glucose, glycerin, glyceryl behenate, glyceryl monooleate, glyceryl monostearate, glyceryl palmitostearate, glycofurol, guar gum, hectorite, heptafluoropropane, hexetidine, hydrocarbons, hydrochloric acid, hydroxyethyl cellulose, hydroxyethylmethyl cellulose, hydroxypropyl cellulose, hydroxypropyl cellulose, low-substituted, hydroxypropyl starch, hypromellose, hypromellose acetate succinate, hypromellose phthalate, honey, imidurea, inulin, iron oxides, isomalt, isopropyl alcohol, isopropyl myristate, isopropyl palmitate, kaolin, lactic acid, lactitol, lactose, anhydrous, lactose, monohydrate, lactose, spray-dried, lanolin, lanolin alcohols, lanolin, hydrous, lauric acid, lecithin, leucine, linoleic acid, macrogol hydroxystearate, magnesium aluminum silicate, magnesium carbonate, magnesium oxide, magnesium silicate, magnesium stearate, magnesium trisilicate, malic acid, maltitol, maltitol solution, maltodextrin, maltol, maltose, mannitol, medium-chain triglycerides, meglumine, menthol, methylcellulose, methylparaben, mineral oil, mineral oil, light, mineral oil and lanolin alcohols, monoethanolamine, monosodium glutamate , monothioglycerol, myristic acid , neohesperidin dihydrochalcone, nitrogen, nitrous oxide, octyldodecanol, oleic acid, oleyl alcohol, olive oil, palmitic acid, paraffin, peanut oil, pectin, petrolatum, petrolatum and lanolin alcohols, phenol, phenoxyethanol, phenylethyl alcohol, phenylmercuric acetate, phenylmercuric borate, phenylmercuric nitrate, phosphoric acid, polacrilin potassium, poloxamer, polycarbophil, polydextrose, polyethylene glycol, polyethylene oxide, polymethacrylates, poly(methyl vinyl ether/maleic anhydride), polyoxyethylene alkyl ethers, polyoxyethylene castor oil derivatives, polyoxyethylene sorbitan fatty acid esters, polyoxyethylene stearates, polyvinyl acetate phthalate, polyvinyl alcohol, potassium alginate, potassium benzoate, potassium bicarbonate, potassium chloride, potassium citrate, potassium hydroxide, potassium metabisulfite, potassium sorbate, povidone, propionic acid, propyl gallate, propylene carbonate, propylene glycol, propylene glycol alginate, propylparaben, 2-pyrrolidone, raffinose, saccharin, saccharin sodium, saponite, sesame oil, shellac, simethicone, sodium acetate, sodium alginate, sodium ascorbate, sodium benzoate, sodium bicarbonate, sodium borate, sodium chloride, sodium citrate dihydrate, sodium cyclamate, sodium hyaluronate, sodium hydroxide, sodium lactate, sodium lauryl sulfate, sodium metabisulfite, sodium phosphate, dibasic, sodium phosphate, monobasic, sodium propionate, sodium starch glycolate, sodium stearyl fumarate, sodium sulfite, sorbic acid, sorbitan esters (sorbitan fatty acid esters), sorbitol, soybean oil, starch, starch (*e.g.* pregelatinized, sterilizable maize), stearic acid, stearyl alcohol, sucralose, sucrose, sugar, compressible, sugar, confectioner's, sugar spheres, sulfobutylether b-cyclodextrin, sulfuric acid, sunflower oil, suppository bases, hard fat, talc, tartaric acid, tetrafluoroethane, thaumatin, thimerosal, thymol, titanium dioxide, tragacanth, trehalose, triacetin, tributyl citrate, triethanolamine, triethyl citrate, vanillin, vegetable oil, hydrogenated, water, wax, anionic emulsifying, wax (*e.g.* carnauba, cetyl esters, microcrystalline, nonionic emulsifying, white, yellow), xanthan gum, xylitol, zein, zinc acetate, zinc stearate, or any combination thereof.

**Computer Methods and Systems**

Also disclosed herein are computer control systems that are programmed to implement methods described herein.

In some embodiments, a system for analyzing the methylation of sperm DNA is described herein. In some cases, the system can comprise a computer system for analyzing a DNA from a sperm cell, a cell free DNA from a seminal sample, or both obtained from a male subject. In some cases, the computer system can comprise a device for receiving sequenced data. In some cases, the computer system can comprise a device for receiving array data, such as a microarray. In some instances, the sequenced data comprises methylation of at least 5 regions of an individual promoter comprised in the DNA from the sperm cell, the cell free DNA from the seminal sample, or both, and wherein the individual promoter is a promoter of **Table 1.** In some instances, the array data comprises methylation of at least 5 regions of an individual promoter comprised in the DNA from the sperm cell, the cell free DNA from the seminal sample, or both, and wherein the individual promoter is a promoter of **Table 1.** In some cases, the computer system comprises a device for determining independently a standard deviation for methylation in each of the at least 5 regions of the individual promoter. In some cases, the computer system comprises a device for calculating an average standard deviation from the standard deviation from methylation in each of the at least 5 regions of the individual promoter. In some cases, the computer system comprises a device for comparing the average standard deviation of the at least 5 regions of the individual promoter to a reference average standard deviation of at least 5 regions of the individual promoter. In some cases, the computer system comprises a device for determining if the average standard deviation is greater than, or greater than or equal to three standard deviations from the reference standard deviation of the individual promoter. In some cases, the sequenced data or the array data comprises all the regions from **Table 1.** In some cases, the methylation standard deviation from each promoter from **Table 1** is determined and compared to a reference standard deviation for each promoter.

In some cases, a device can be used for receiving an array and the data associated with an array. In some cases, a device can be used to compare the data from the array with a control. In some cases, the data from the array can be a methylation of a promoter at different regions. In some cases, a device can be a computer system.

**FIG. 3** shows a computer system 101 that is programmed or otherwise configured to identify the variability of methylation of a promoter from sperm DNA from a patient's semen sample. The computer system 101 can regulate various aspects of the present disclosure, such as, for example determining a likelihood of a subject benefiting from a treatment to treat a disease (such as infertility). In another example, the computer system can be used to determine a standard deviation of methylation of a region of a promoter or an average standard deviation of methylation of a promoter. The computer system 101 can be an electronic device of a user or a computer system that is remotely located with respect to the electronic device. The electronic device can be a mobile electronic device.

The computer system 101 includes a central processing unit (CPU, also "processor" and "computer processor" herein) 105, which can be a single core or multi core processor, or a plurality of processors for parallel processing. The computer system 101 also includes memory or memory location 110 (*e.g.,* random-access memory, read-only memory, flash memory), electronic storage unit 115 *(e.g.,* hard disk), communication interface 120 (*e.g.,* network adapter) for communicating with one or more other systems, and peripheral devices 125, such as cache, other memory, data storage and/or electronic display adapters. The memory 110, storage unit 115, interface 120 and peripheral devices 125 can be in communication with the CPU 105 through a communication bus (solid lines), such as a motherboard. The storage unit 115 can be a data storage unit (or data repository) for storing data. The computer system 101 can be operatively coupled to a computer network ("network") 130 with the aid of the communication interface 120. The network 130 can be the Internet, an internet and/or extranet, or an intranet and/or extranet that is in communication with the Internet. The network 130, in some cases is a telecommunication and/or data network. The network 130 can include one or more computer servers, which can enable distributed computing, such as cloud computing. The network 130, in some cases with the aid of the computer system 101, can implement a peer-to-peer network, which may enable devices coupled to the computer system 101 to behave as a client or a server.

The CPU 105 can execute a sequence of machine-readable instructions, which can be embodied in a program or software. The instructions may be stored in a memory location, such as the memory 110. The instructions can be directed to the CPU 105, which can subsequently program or otherwise configure the CPU 105 to implement methods of the present disclosure. Examples of operations performed by the CPU 105 can include fetch, decode, execute, and writeback.

The CPU 105 can be part of a circuit, such as an integrated circuit. One or more other components of the system 101 can be included in the circuit. In some cases, the circuit is an application specific integrated circuit (ASIC).

The storage unit 115 can store files, such as drivers, libraries and saved programs. The storage unit 115 can store user data, *e.g.,* user preferences and user programs. The computer system 101 in some cases can include one or more additional data storage units that may be external to the computer system 101, such as located on a remote server that is in communication with the computer system 101 through an intranet or the Internet.

The computer system 101 can communicate with one or more remote computer systems through the network 130. For instance, the computer system 101 can communicate with a remote computer system of a user *(e.g.,* a lab technician, or health care professional). Examples of remote computer systems include personal computers *(e.g.,* portable PC), slate or tablet PC's *(e.g.,* Apple® iPad, Samsung® Galaxy Tab), telephones, Smart phones *(e.g.,* Apple® iPhone, Android-enabled device, Blackberry®), or personal digital assistants. The user can access the computer system 101 via the network 130.

Methods as described herein can be implemented by way of machine (e.g., computer processor) executable code stored on an electronic storage location of the computer system 101, such as, for example, on the memory 110 or electronic storage unit 115. The machine executable or machine readable code can be provided in the form of software. During use, the code can be executed by the processor 105. In some cases, the code can be retrieved from the storage unit 115 and stored on the memory 110 for ready access by the processor 105. In some situations, the electronic storage unit 115 can be precluded, and machine-executable instructions can be stored on memory 110.

The code can be pre-compiled and configured for use with a machine having a processer adapted to execute the code or can be compiled during runtime. The code can be supplied in a programming language that can be selected to enable the code to execute in a pre-compiled or as-compiled fashion.

Aspects of the systems and methods provided herein, such as the computer system 101, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory *(e.g.,* read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 101 can include or be in communication with an electronic display 135 that comprises a user interface (UI) 140 for providing, for example, the variability of methylation of a promoter. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 105. The algorithm can, for example, determine if an average standard deviation of methylation for an individual promoter is greater than, or greater than or equal to three standard deviations from a reference standard deviation for the individual promoter.

In some cases, as shown in **FIG. 4,** a sample **202** containing a seminal fluid sample can be obtained from a subject **201,** such as a human subject. A sample **202** can be subjected to one or more methods as described herein, such as identifying the variability of methylation in a promoter present in the DNA sample by an array, by sequencing, by PCR, or any combination thereof. In some cases, an assay can comprise obtaining the average standard deviation of DNA methylation of a promoter region, comparing the average standard deviation of methylation of the promoter region with a reference (*e.g.,* control) sample standard deviation, and displaying that information in a statistical readout (*e.g.,* a spreadsheet, a graph, or a heatmap). One or more results from a method can be input into a processor **204**. One or more input parameters such as a sample identification, subject identification, sample type, a reference, or other information can be input into a processor **204**. One or more metrics from an assay can be input into a processor **204** such that the processor can produce a result, such as determining a difference in the standard deviation of methylation of an individual promoter. In some cases, a change in the variability of methylation (*e.g.,* the standard deviation) of a promoter, or of 22 or more promoters, can indicate diminished fertility of a subject. In some cases, no change or limited change in the variability of methylation of a promoter can indicate fertility of a subject. A processor can send a result, an input parameter, a metric, a reference, or any combination thereof to a display **205,** such as a visual display or graphical user interface. A processor **204** can (i) send a, an input parameter, a metric, or any combination thereof result wirelessly or directly to a server **207,** (ii) receive a result, an input parameter, a metric, or any combination thereof from a server **207,** (iii) or a combination thereof. In some cases, the output can be a heatmap or a statistical output.

**Examples**

**Example 1- DNA Methylation Analysis of Sperm**

The DNA methylation sperm quality test is a DNA methylation analysis that improves the measurement of sperm quality. By detecting poor sperm quality, the DNA methylation sperm quality test can direct patients to *in vitro* fertilization (IVF) treatment and help avoid unnecessary procedures and loss of precious time. The DNA methylation sperm quality test can be an assessment of male fertility. Presence of a DNA methylation biomarker can be associated with poor fertility outcomes. IVF shows potential for overcoming decreased sperm quality detected by the DNA methylation sperm quality test. The DNA methylation sperm quality test can identify one or more male infertility factor that can be missed by the standard semen analysis.

*Sperm epigenetics as a measure for male infertility.*

Studies have reported abnormal sperm DNA methylation patterns are associated with male infertility (2). In some cases, DNA methylation is a common epigenetic modification found on DNA where a $CH_3$ (Methyl) molecule is covalently attached to a base of a nucleotide or nucleoside of DNA for example a cytosine base. This modification can change the expression of genes and is a focus of the DNA methylation sperm quality test. In some cases, specific DNA methylation patterns on genes associated with biological functions such as spermatogenesis (the production and development of sperm) and embryo development have been identified. In some cases, the DNA methylation sperm quality test analyzes the DNA variability of methylation of individual promoters and assesses if a man is at high risk for poor sperm quality.

*Identification of a DNA methylation biomarker.*

DNA methylation analysis of sperm was conducted on 112 men seeking fertility care and 54 men known to be fertile ('fertile controls"). Couples with moderate-to-severe female factor infertility were excluded (including advanced maternal age, severe endometriosis, or polycystic ovarian syndrome). DNA methylation analysis of 10,000 gene promoters showed a statistically significant difference in methylation levels between men seeking fertility care and men known to be fertile, as seen in **FIG. 1. FIG. 1** shows increased methylation variability in the promoters screened in men seeking fertility care as compared to the fertile controls. Investigation of the sites that were differentially methylated indicated a role in sperm development, sperm maturation, and embryogenesis. 70% of men with this DNA methylation biomarker displayed normal concentration and motility semen parameters. The DNA methylation biomarker was promoter dysfunction in a subset of selected gene promoters from the original 10,000 gene promoters.

*Secondary study with 1,336 semen samples and live birth outcomes*

Sperm DNA methylation data from 1,336 men who were seeking fertility care in the NIH FAZST Study were analyzed (3). A blinded analysis of these samples confirmed the existence of the DNA methylation biomarker described previously and showed a negative correlation with live birth outcomes. The DNA methylation biomarker was present in 10% of all semen samples analyzed, and 77% of those men displayed normal semen concentration and motility parameters.

Couples treated with intrauterine insemination (IUI) had a statistically significant lower live birth rate when the DNA methylation biomarker was present in the male partner as shown in **FIG. 2A.** However, in couples treated with IVF, there was no significant difference in live birth rates as shown in **FIG. 2B.** This suggests lower sperm quality, as indicated by the DNA methylation biomarker, can be overcome with the use of IVF. If the male partner has the DNA methylation biomarker, there is an 84% positive predictive value of accurately diagnosing the need for IVF.

*Tertiary study of the DNA methylation biomarker*

From the large data set, DNA methylation biomarker cut offs were established and an analysis algorithm was derived. All further analyses were completed using a fixed algorithm based on the 1,336 semen samples within the confirmatory study. Applying the fixed analysis pipeline to an independent dataset, the sperm DNA methylation of 74 men seeking infertility care and 60 fertile controls were analyzed. 38% of men seeking infertility care presented with the DNA methylation biomarker, compared to only 6.5% of the fertile controls. The difference is statistically significant (p=3.8 E-0.6**) and established a negative predictive value of 94.5% of needing IVF.

*Further validation of the DNA methylation biomarker*

Semen sample collection is continuing from all consenting incoming patients with fertility concerns. To date 78 patient samples have been collected, and associated treatment and outcome data are being collected. Of the current patients, 22% present with the DNA methylation biomarker, of which 73% have normal semen parameters. In analysis of fertility outcomes from non-IVF procedures, 0% of the men with the DNA methylation biomarker have had a successful pregnancy while 20% of the men without the biomarker have since had a pregnancy.

References:

(1) Schlegel PN, et al Diagnosis and Treatment of Infertility in Men: AUA/ASRM Guideline Part I. J Urol. 2021 Jan;205(1):36-43. Barratt CL, et al. Diagnostic tools in male infertility-the question of sperm dysfunction. Asian journal of andrology. 2011;13(1):53-8. Bonde JP, et al. Relation between semen quality and fertility: a population-based study of 430 first-pregnancy planners. Lancet. 1998;352(9135)
(2) Benchaib M,. et al., Quantitation by image analysis of global DNA methylation in human spermatozoa and its prognostic value in in vitro fertilization: a preliminary study. Fertil Steril. 2003;80(4):947-53. Houshdaran S., et al. Widespread epigenetic abnormalities suggest a broad DNA methylation erasure defect in abnormal human sperm. PloS one. 2007;2(12)
(3) Schisterman EF, et al. A Randomized Trial to Evaluate the Effects of Folic Acid and Zinc Supplementation on Male Fertility and Live birth: Design and Baseline Characteristics. Am J Epidemiol. 2020 Jan 3;189(1):8-26.

**Example 2: A microarray method for the DNA methylation sperm quality test (Intra Individual Instability Analysis for Sperm Samples)**

First, the genomic DNA was isolated from sperm cells. Following DNA isolation, the DNA was converted to identify unmethylated cytosines. The converted DNA was analyzed via a microarray. Raw IDAT values from the methylation array were converted into beta values and run through a quality control test to check for any samples from the array that failed. This was done using the minfi package in R. If the sample passes initial quality control, then a check for somatic cell contamination was performed. This was done by taking the mean value of all CpG beta values found on DLK1. Any sample that has a mean value greater than or equal to 0.20 was considered to be contaminated and was removed. If the sample passed quality control, the beta values were then converted into M-values using logit transformation. Using GRch37 as the reference genome (see **Table 6**), each CpG was mapped to its correlating promoter. This was done using bedtools intersect. Only promoters that contain 5 CpG M-values or more were kept for the Intra-Individual analysis (*e.g.,* the DNA methylation sperm quality test). The initial analysis was limited to 10,000 promoters. For each sample, the Standard deviation of M-values at each promoter was calculated. Donor samples were combined to establish what was considered to be "normal" or "healthy" variation within a promoter. An instability score was calculated for each sample. This was done by comparing each promoter to what was calculated to be "normal" or "healthy" variation. The instability score above a defined cutoff was "the biomarker" as discussed in Example 1.

**Example 3: A method for the DNA methylation sperm quality test - Using Targeted Next Generation analysis of sperm chaos scores**

First, the Genomic DNA was isolated from sperm cells. The isolated DNA was converted to identify unmethylated cytosines. The DNA was fragmented. Custom designed biotinylated probes were used to target and capture regions of interest. Polymerase chain reaction (PCR) amplification was completed of the captured sites and then next-generation sequencing (NGS) was performed on the captured sites. The # of reads per site was used to calculate methylation and derive the instability score

**Example 4: Promoter dysregulation via methylation of sperm DNA**

*Methods:* Sperm DNA methylation data from 43 fertile sperm donors was analyzed and compared with sperm DNA methylation data from 1344 men undergoing fertility treatment. Methylation at the 1233 gene promoters with the least variable methylation in fertile patients were used to create two thresholds and three categories of promoter dysregulation in the fertility treatment cohort (poor, average, excellent).
*Results:* After controlling for female factors, IUI pregnancy and live birth outcomes between the poor and excellent groups were significantly different: 19.4% vs. 51.7% (p=0.008) and 19.4% vs. 44.8% (p = .03), respectively. IVF live birth outcomes were not found to be significantly different between any of the three groups.
*Conclusion:* Variable methylation in a panel of 1233 gene promoters is a reliable biomarker for IUI failure.

**Overview**

The complexity of spermatogenesis requires a systems biology approach to fully understand. Investment in research over the last two decades has revealed the multifactorial relationships of DNA, RNA, microRNAs, DNA methylation, chromatin, and the proteome in each step of conception and embryo development. Despite this, male infertility is still assessed through a visual examination of sperm quantity, shape, and movement through a standard semen analysis.
The semen analysis has changed very little over the past decades other than minor modifications in the assessment of morphology made by the World Health Organization in 2021. While numerous studies have evaluated semen analysis parameters as benchmarks in evaluating reproductive potential, its predictive power remains limited, with only azoospermia preventing any chance of natural conception. The introduction of DNA Fragmentation testing has provided additional insights to the molecular function of the sperm by assessing the structural integrity of the sperm DNA. However, due to the lack of correlation to fertility potential, in some cases the current guidelines and research suggests DNA fragmentation should not be tested in the initial assessment of male infertility but instead in the cases of recurrent pregnancy loss. Thus, the semen analysis remains the primary tool for the initial male fertility assessment.
The implementation of the semen analysis as the primary assessment of sperm health leads to an incomplete understanding of a couple seeking fertility care - which has been shown to lead to unnecessary procedures, a longer time to pregnancy, and an increased burden on the female partner. Advances in the assessment of male fertility are needed to develop personalized approaches to diagnosing and treating male fertility.
Epigenetic analysis of sperm DNA has emerged over the last decade as a potential new tool to more comprehensively assess male fertility potential. Epigenetics can refer to the heritable regulation of gene expression that is not dependent on changes to the DNA sequence itself. Specifically, the analysis presented in this example assesses DNA methylation modifications that occur at cytosine-phosphate guanine dinucleotide (CpGs) on the DNA. Understandably, as DNA methylation controls gene expression, the maintenance of proper DNA methylation is important for healthy cellular function, including sperm function. The objective of the presented example is to better understand the epigenetic determinants of sperm quality and to assess a new method for determining male fertility potential using DNA methylation.
Utilizing data from a multi-site NIH clinical trial, a novel method for analysis of aberrant DNA methylation was used that allows for global quantification of genes related to sperm function. After analysis of 1344 semen samples an epigenetic (DNA Methylation) profile was determined to be predictive of sperm quality. In some cases, the epigenetic profile with or without semen analysis could expand the clinical assessment of male fertility potential

**Methods**

*Data Procurement:* Sperm DNA methylation data (Infinium MethylationEPIC Array) from fertile sperm donors was used in this study. Sperm DNA methylation data from a clinical multi-site NIH study of men experiencing infertility were also used.
*Patient Details:* Analysis was completed on 1344 de-identified patient sperm DNA methylation data and clinical outcomes. Outcomes included both live birth and pregnancy data, where pregnancy was defined by either ultrasound and biochemical (hcg) assessment. Patient clinical information for this study is as follows: men from couples undergoing intrauterine insemination (IUI) had an average of 2.5 IUI cycles in the population. For men undergoing *in vitro* fertilization (IVF), there was an average of 1.5 embryos transferred per couple and 76% of fertilizations occurred via IVF with intracytoplasmic sperm injection (ICSI). Additionally, when controlling for female factors, females <35 years old with no diagnosis of PCOS, endometriosis, fibroids, blocked tubes, or diminished ovarian reserves (DOR) were included.

*Data Preprocessing:* The sperm DNA methylation data were preprocessed. The sperm DNA methylation data were preprocessed using the minfi R package without using normalization techniques to produce beta and m-values for each cytosine-guanine dinucleotide (CpG). Density plots of the beta values of each sample were examined to ensure the distribution of beta values followed a bimodal distribution with prominent peaks between 0.0-0.2 and 0.8-1.0 and flat valleys from 0.2-0.8. Any samples not following this distribution were removed. All beta values or m-values with a value of infinity or negative infinity were removed from analysis and all beta values or m-values whose corresponding detection p-values were less than 5e-10 were removed. Any sperm samples that did not have a mean methylation value less than 0.20 of all the CpG beta values in the differentially methylated region of DLK1 was removed from analysis. The methylation states of the probes in this region are a good discriminator between sperm and somatic cells and this procedure ensured analyses were only performed on samples containing sperm DNA methylation and not contaminating somatic cell DNA methylation.

*N-of-1 Analyses:*

The gene promoters with the least variable methylation values (n=1233) in sperm from fertile sperm donors (n=43) and the corresponding gene promoter variability cutoffs were selected as shown in **FIG. 14C.** These promoters and corresponding cutoffs were then used to perform n-of-1 analyses on the sperm methylation data from the men experiencing infertility (n=1344). The promoter methylation variability was examined within the selected promoters and then the number of promoters were counted that fell outside the prescribed gene methylation promoter cutoff, which are referred to as dysregulated promoters. These n-of-1 analyses were performed as outlined below. The variability methylation value of a gene promoter was defined as $\sigma = \sqrt{\dfrac{\sum |x_1 - \mu|^2}{N}}$ , where x1 = m-value of a given array probe in a given gene promoter, $\mu$= mean of the array probes m-values in that given promoter. The equation was modified to calculate the methylation variability cutoff threshold for a given promoter as

$$\theta = 1.1\left(\frac{\sum \sigma_1}{N} + 3\sqrt{\frac{\sum |\sigma_1 - \mu|^2}{N}}\right)$$ , where σ1= promoter methylation variability value of a sample within a given cohort and $\mu$= mean of the methylation variability values of a given promoter, and N=number of samples.

Thresholds were established for the number of dysregulated promoters for samples with "Excellent" ($\leq$ 3 dysregulated promoters), "average" (between 4 to 21 dysregulated promoters), and "Poor sperm" quality ($\geq$ 22 dysregulated promoters). Two-sided t-tests were performed on the live birth rates of men falling into these three sperm quality groups based on the infertility treatments the couple received.

A permutation analysis (n=10,000) was performed by shuffling the live birth results of the couples receiving IUI and comparing the live birth rates of the couples in the Excellent sperm quality category and those in the Poor sperm quality category. The results are shown in **FIG. 8.**

**Results**

*Demographic and Methylome-Wide Analysis of Promoter Dysregulation from 1344 men seeking fertility care*

Full semen parameters and male demographic information is described in **Table 2** . Within the 1344 men analyzed for this study 12% of men had a sperm concentration of less than 15M/mL, 14.3% of men had a total motile count (TMC) less than 20M, and 65.5% had morphology greater than or equal to 4%. An overview of the female partner demographics can be found in **Table 3.** In **Table 3,** PCOS stands for polycystic ovary syndrome and DOR stands for diminished ovarian reserve. In an analysis of IUI outcomes, 21.1 % of the men were removed to help control for the role of female fertility factors.

**Table 2: Statistics of semen parameters of men in various sperm quality groups**

| Sperm Quality Group | Concentration (M/ml), mean | Concentration (M/mL), median | Total Motile, mean | Total Motile, median | Morphology (%), mean | Morphology (%), median | Male BMI, mean | Male BMI, median | Male Age, mean | Male Age, median |
|---|---|---|---|---|---|---|---|---|---|---|
| Excellent | 98.65 | 71.5 | 233.11 | 132.5 | 6.8 | 6 | 30.14 | 29.18 | 32.01 | 32.0 |
| Average | 97.29 | 77.25 | 225.4 | 148.82 | 6.11 | 5.25 | 29.27 | 27.92 | 32.62 | 32.0 |
| Poor | 73.79 | 50.5 | 146.56 | 80.66 | 6.06 | 5.5 | 29.31 | 28.34 | 33.54 | 32.0 |
| All Groups | 94.94 | 74.5 | 217.77 | 139.17 | 6.17 | 5.5 | 29.35 | 28.14 | 32.67 | 32.0 |

**Table 3: Demographics of female partners of all study men**

| Average Age | Age >= 35 (%) | Average BMI | PCOS (%) | Endometriosis (%) | Fibroids (%) | Blocked tubes (%) | DOR (%) |
|---|---|---|---|---|---|---|---|
| 31.0 | 25.1% | 27.9 | 10.6% | 4.1% | 0.7% | 2.0% | 2.0% |

As described in the methods, an n-of-1 gene promoter methylation analysis was performed on sperm samples from 1344 men seeking infertility care to see how many gene promoters had irregular methylation (referred to here as dysregulated promoters) than set thresholds based on fertile controls. **FIG. 5A** shows the distribution of dysregulated promoters among the infertile men with an average of 12.7 dysregulated promoters and a median of 9 dysregulated promoters. A regression analyses was performed between the number of dysregulated promoters and several factors such as male BMI, age, total motile count, concentration and morphology and saw no meaningful relationships to the number of dysregulated promoters as shown in **FIGS. 5B, 5C** and **FIG. 6.**

*Increasing prevalence of dysregulated promoters is associated with lower pregnancy and live birth outcomes in IUI procedures*

Previous data in other disease types has shown that increased promoter dysregulation is associated with pathologic phenotypes. The relationship between the number of dysregulated promoters and clinical outcomes for different fertility treatments was examined while controlling for contributing female factors. To do this, the top and bottom 10th percentile of dysregulated promoters were identified to the nearest integer. The top 10th percentile included men with $\geq$ 22 dysregulated promoters (N= 140) and was designated as the "Poor" group. The bottom 10th percentile of dysregulated promoters included men with $\leq$3 dysregulated promoters (n=114) and was designated as the "Excellent" group. All remaining men with >3 and <22 dysregulated promoters (n=1090) were designated as the "Normal" group. **Table 2** contains a breakdown of the semen parameters and demographics associated with each group. When creating these three distinct groups a statistically significant enrichment was identified of men with low TMC in the Poor group compared to the Excellent group.

Analysis of the percent of live births and pregnancies resulting from men undergoing IUI (N=544) showed a statistically significant difference between the Excellent and Poor groups, as well as between the Average and Poor groups as shown in **FIG. 7A.** Similar pregnancy and live birth results were seen when analyzing men whose female partners had no female infertility factor (N=344) as shown in **FIG. 7B,** indicating a role of DNA methylation promoter dysregulation in a sperm's fertility potential. Permutation analysis was completed to determine if the differences seen in live births could be due to random chance. The differences in live births seen in this analysis were in the 99th percentile of permutations, meaning that there is a 1 in 100 probability these results are due to chance as shown in **FIG. 8.**

When completing the same analysis for men undergoing IVF there was no statistical difference between any of the groups (Excellent, Average, or Poor), with or without controlling for female factors as shown in **FIG. 7C** and **FIG. 7D.** These data together show the analysis of accumulating dysregulated promoters, appears to identify men with lower fertility potential for IUI procedures, and that IVF could be a treatment that overcomes the dysregulated gene promoter methylation.

*The number of dysregulated promoters combined with total motile count is more predictive of pregnancy and live births than either metric alone*

Even with the enrichment of low TMC in the Poor groups, the described analysis identified a new subset of men with Poor fertility potential that would have been missed by semen analysis alone. 77.8% of men with a Poor result had a TMC ≥20M and 75.6% of men with a SpermQT result had both a TMC ≥20M and a concentration ≥15M/mL. Since it is identifying a different subset of sub-fertile men, it was found that the combination of the analysis described herein with TMC can provide an more granular and predictive assessment of pregnancy and live birth as shown in **FIG. 9.**

*An accumulation of dysregulation in multiple biological pathways is analyzed by the methods described herein*

Analysis of the dysregulated promoters in the 1233 target gene promoters across all samples with a 'Poor' score revealed a broad distribution of gene promoters contributing to the accumulation of dysregulation in sperm cells as shown in **FIG. 10,** reflecting the biological complexity and heterogeneity between male patients. Ten gene promoters were epigenetically dysregulated in more than 20% of samples (see **FIG. 10** inset) and the three most dysregulated genes (ACTR5, ASGR1, and HSD17B7) were dysregulated in more than 30% of samples (36.2%, 33.3%, and 31.2%, respectively). The ten most dysregulated promoters were ACTR5, ASGR1, HSD17B7, ABHD17A, CALML6, H3C8, SARS1, VPS28, GRAMD1A, AQP10. ACTR5 is an Actin Repair Protein known for UV-damage repair and double-strand break repair and has been previously identified to be highly expressed in the testis. ASGR1 is a protein subunit of the asialoglycoprotein receptor which is largely known for glycoprotein homeostasis in the liver but has been identified to be enriched in early and late stage spermatids. Glycoproteins are known to be essential for sperm development and proper function. HSD17B7 is an enzyme involved in estrogen and androgen metabolism as well as cholesterol biosynthesis. Deletion of HSD17B7 has been shown to cause reduced testosterone production and early fetal death in mice. Additional analysis of the distribution of dysregulated promoters is shown in **FIG. 11. FIG. 11** shows the 20 most dysregulated promoters of ACTR5, ASGR1, CALML6, SARS1, HSD17B7, H3C8, ABHD17A, VPS28, SCARNA9, AQP10, NAE1, GRAMD1A, KCNU1, TSPAN16, PGBD4, LAMC2, GUSBP1, ITIH1, HSH2D, TBC1D26.

This example showed there are multiple biological pathways that may be disrupted in sperm and may lead to decreased fertility potential, and that these biological pathways are likely different between infertile men. A threshold of epigenetic stability was identified for a sperm to be considered healthy. In some cases, once a sperm crosses this threshold of dysregulation stability there emerges a phenotype of lower fertility potential.

A DNA Methylation assessment as described herein was developed in sperm that has a statistically significant association with accumulative pregnancy and live birth percentages in men undergoing IUI and identifies a subset of men that are largely missed by the current standard of care.

The primarily visual and superficial aspects of the current standard of care (the semen analysis) are important but fall short of a comprehensive diagnostic for male infertility. In some instances, the combination of the methods described herein and semen analysis (particularly TMC) can yield an even more predictive assessment of likely pregnancy and birth outcomes than either assessment does on its own. When combined with the initial semen analysis, this analysis can provide additional guidance to direct treatment and set expectations for patients seeking fertility care. In some cases, the methods described herein, such as the DNA methylation analysis described in this example, can be used alone without semen analysis to provide a predictive assessment.

**Example 5: Promoter methylation of different cells including sperm**

*Background:* Complex diseases can have multifactorial etiologies making clinically actionable diagnostic markers difficult to identify. Tools with higher diagnostic yield and utility in driving personalized care are needed.

Methods: Illumina methylation array data was utilized from 2396 samples to assess DNA methylation patterns in 19 distinct cell types and various diseases. An analysis pipeline was generated for DNA methylation data that focuses on intra-individual methylation variability within gene promoters. The analysis was designed, not to identify single causative gene alterations but instead focuses on any movement away from "healthy" methylation. This approach identifies altered regulation across multiple genes in related pathways. This enables the detection of shifts in gene regulatory activity associated with distinct tissues and phenotypes. Three distinct questions were assessed. 1) Are patterns of epigenetic instability able to distinguish between tissue types? 2) Do diseased tissues exhibit altered instability patterns compared to normal tissue? 3) Can epigenetic instability be detected in complex disease?

*Results:* Unsupervised clustering analyses demonstrated that patterns of epigenetic variability can be tissue specific and that these patterns can be at least as predictive of tissue type as differential methylation analysis even in cases of complex multifactorial diseases.

*Conclusion:* This study demonstrates that patterns of epigenetic instability can differentiate between tissue types. This finding suggests that specific epigenetic instability patterns may be used to predict phenotypic changes in disease states as these are, by definition, functional changes to cellular phenotypes. It was demonstrated that the stability of gene regulatory marks are distinct between healthy and diseased tissue particularly at genes known to be important to cell function of the impacted tissue. While in some cases these regional alterations can be seen across the entire genome, more often the regulatory alterations that define a pathological phenotype can be restricted to genes of known importance to a particular tissue. In the case of sperm, these patterns of instability did have utility in predicting patients who had difficulty conceiving who then could conceive through *in vitro* fertilization (IVF). It appears that epigenetic instability signatures assessed in an n-of-1 context can indicate a shift away from regulatory normalcy. When these epigenetic instability signatures are associated with pathways known to be impactful in the tissue of interest they can predict the presence of disease or dysfunction independently of the presence or absence of rare genetic variants.

**Overview**

In 2003, one of the most profound efforts ever undertaken in the biological sciences, the Human Genome Project, was completed. At the time there was a great deal of hope that unlocking the genetic code was the key to diagnosing and treating the vast majority of diseases. While the discoveries made have been of great interest to many and have opened the door for important genetic and epigenetic findings, clinically meaningful impacts remain elusive for many diseases. In large part, this is due to the complex, multifactorial nature of most disease processes, with etiologies resulting from a constellation of genetic, epigenetic, and environmental perturbations. As a result, approaches to our analysis of genetic and epigenetic data are needed to identify clinically actionable predictors of disease or disease progression.

While the causative factors of complex disease are multifactorial and highly variable, the tissue and cellular phenotypes that occur as a result of the disease are likely to be more uniform. Therefore, diagnostic approaches should focus, not on single genes or independent modifications associated with a pathology, but on a holistic screen of alterations to gene regulatory activity at genes specifically important to the affected tissue.

Certain data types are ideal for analyses focused on perturbations to gene regulatory networks. DNA methylation is of particular interest in this effort. Because a tissue's phenotype is defined by gene activity, and gene activity can be controlled (at least in part) by epigenetic marks. Marks such as DNA methylation can potentially fingerprint a cell and tissue type. It is also possible that diseases such as cancer (1, 2) and type 2 diabetes (3, 4) can induce epigenetic modifications to achieve a perturbed phenotype. Further, unlike the fairly static nature of the genome, DNA methylation is a dynamic biomarker affected by a host of factors such as age (5, 6) and various modifiers including obesity (7, 8), exercise (9, 10), and environment (11).

Herein, a new approach to DNA methylation analysis and an assessment of its efficacy as a clinical predictor is presented. An analysis of promoter DNA methylation variability that allows assessment of gene regulatory networks in a novel way is presented. DNA methylation array data from 2396 samples and 19 tissues from the analysis can be used to identify and assess the genes most tightly regulated in specific cell types and that these patterns are highly tissue-specific. The utility of this approach in an n-of-1 analysis was used to demonstrate that there are gene regulatory network perturbations common among individuals who suffer from specific pathologies in many different tissues. Lastly, the potential clinical value of this approach was shown in sperm.

**Methods**

*Data Collection*

Several publicly available datasets were used in this study. Infinium HumanMethylation450 Bead Chip data was obtained for tumor and healthy tissue samples from The Cancer Genome Atlas (TCGA) Program as compiled by the University of California Santa Cruz Xena Functional Genomics Explorer (13). Infinium HumanMethylation450 Bead Chip data for CD4+ T cell, CD8+ T cell, neuron and glia, lung, liver, and skin methylation data from healthy and diseased individuals were accessed from the NIH Gene Expression Omnibus (GSE130029, GSE130030, GSE66351, GSE51077, GSE61258, GSE115797, respectively).

Sperm Infinium HumanMethylation450 Bead Chip data from fertile sperm donors as well as patients undergoing *in vitro* fertilization (IVF) was used from a previously published single-site study by Aston, et al (14) as well the sperm Infinium MethylationEPIC Array data from a clinical multi-site study of patients being seen by physicians for fertility care as published by Jenkins et al (15).

*Sample Collection*

Semen samples were procured from University of Utah Andrology department from consented patients undergoing intrauterine insemination (IUI), as well as two independent fertile sperm donor cohorts. Semen samples from consented patients seeking fertility care were also procured from the Urology Department at Baylor College of Medicine.

*Sample Preparation*

For all semen samples, somatic cell lysis, sperm isolation, DNA extraction, and bisulfite conversion were performed as described by Aston, et al (14). The bisulfite converted sperm DNA was hybridized to Illumina Infinium HumanMethylationEPIC microarrays and ran as recommended by the manufacturer (Illumina) at Infinity BiologiX.

*Data Preprocessing*

**FIG. 12** contains a flow chart of data processing and statistical analysis. The raw methylation array data from the sperm, neuron, glia, skin, CD4+ T cell, and CD8+ T cell samples were preprocessed using the minfi R package (16) using SWAN normalization to produce beta and m-values for each cytosine-guanine dinucleotide (CpG). Density plots of the beta values of each sample were examined to ensure the distribution of beta values followed a bimodal distribution with prominent peaks between 0.0-0.2 and 0.8-1.0 and flat valleys from 0.2-0.8. Any samples not following this distribution were removed and the remaining samples were renormalized. Beta values are described as (methylated probe intensity / [methylated + unmethylated probe intensity + 100]) and range from 0-1 with values around 0 being unmethylated and values around 1 being methylated. M-values are described as (log(methylated probe intensity / unmethylated probe intensity) and are useful measures of methylation to prevent bias arising from heteroscedasticity seen when analyzing beta values (17) . These analysis are shown in **FIG. 13A-B**.

For data processing of sperm samples, sperm samples were removed from the analysis that did not have a mean methylation value less than 0.20 of all the CpG beta values in the differentially methylated region of DLK1 as described by Jenkins, et al (18) (chr14:101,191,893-101,192,913, GRCh37). According to Jenkins et al, the methylation states of the probes in this region are a good discriminator between sperm and somatic cells and this procedure ensured analyses were only performed on samples containing sperm DNA methylation and not contaminating somatic cell DNA methylation.

Raw data for the TCGA datasets as assembled on the UCSC Xena platform and the lung and liver datasets (GSE51077 and GSE61258, respectively) were not available, so the available beta values were used. These beta values were logit-transformed to obtain the m-values for these samples.

*Statistical analysis*

A gene promoter is described as the genomic region one kilobase upstream and one kilobase downstream from the transcription start site of a given gene. In this example, a gene promoter needed to contain five or more methylation array probes to be used in any downstream analysis. It his hypothesized a promoter could be included with less than 5 methylation probes. Gene methylation promoter variability (or "promoter variability") is defined as the standard deviation of the m-values of the methylation array probes present in a defined promoter region see **FIGS. 13A-B**). **FIG. 14A** shows the equation for calculating the variability value (or standard deviation) of a given promoter in a sample; $\sigma$ = gene promoter variability value, $x_1$ = m-value of a given methylation array probe in a given promoter, $\mu$ = mean of probe m-values in given promoter. **FIGS. 14B and 14C** shows the equation to calculate the promoter variability threshold for a given tissue. $\Theta$ = promoter variability threshold for a given tissue, $\sigma_1$ = promoter methylation variability value of a sample in a given cohort at a given promoter, $\mu$ = mean of the methylation variability values of a given promoter and N = the number of samples. For this example, equation **FIG. 14B** was used. Further experiments have shown **FIG. 14C** to work as well.

Hierarchical clustering was performed on all promoter variability values of samples from various tissue types using the R software package 'pheatmap' (R version 4.0.3) with default parameters. In cases where more than 20 samples existed for a given tissue, 20 samples were randomly selected for inclusion in the clustering analysis to give a more uniform number of samples per tissue type. Principal component analyses were performed on all promoter variability values using the 'sklearn' library in Python (Python version 3.7.3).

The most epigenetically stable promoters of a given tissue type were found by identifying the promoters with the lowest levels of variability in healthy samples of that tissue type. This was done by first calculating a stability threshold for each promoter in a given tissue. A promoter stability threshold represents the highest level of variability expected to see in a given promoter of a healthy sample of a given tissue. Then, the promoters were rank ordered by the stability threshold values in ascending order. For the analyses comparing promoters across tissue types (FIGS. 15A, 15B, 15E, 15F), the most stable promoters were defined as the top first percentile of promoters with the lowest stability thresholds in healthy samples of the given tissue. The most stable promoters for the sperm n-of-1 analyses were defined as the top 10th percentile of promoters with the lowest stability thresholds in fertile sperm donors.

Sperm n-of-1 analyses were performed by finding the most stable promoters in a cohort of fertile sperm donors (n=46) and counting the number of dysregulated promoters in each sample. A dysregulated promoter was defined as a promoter that fell above the corresponding variability threshold. Samples with the lowest number of dysregulated promoters are most similar to healthy controls.

Statistical differences in the pregnancy and live birth rates of men undergoing intrauterine insemination (IUI) and *in vitro* fertilization (IVF) with the least and most dysregulated promoters were calculated with two-sided t-tests. The men undergoing IUI had been through an average of 2.5 IUI attempts.

Tissue-specific gene ontology enrichment analyses were performed by running the PANTHER Overrepresentation Test on the gene names of the first percentile of most stable promoters in a given tissue. Each test was run using a background gene set that consisted of all genes with promoters containing five or more methylation array probes.

**Results**

*Tissues have methylation variability signatures*

Using microarray DNA methylation data, the differences in gene promoter methylation variability of various healthy tissues were explored. Unsupervised clustering of all gene promoter variability values showed tissue specificity and also revealed similarities among related tissues. For example, gastrointestinal tissue samples such as those from the esophagus, stomach, colon and rectum, clustered closely together. Clustering of samples associated with the immune system (CD4+ T cells, CD8+ T cells, thymus), female reproduction (endometrium, cervix), and brain (glia, neurons) was identified.

*Tissues are regulated at tissue-specific biological pathways*

Promoter variability was analyzed among various tissue types. The most stable promoters were identified in sperm. The average methylation variability values were assessed for these promoters in many samples across several tissue types as seen in **FIG. 15A.** At promoters indicated as most stable in sperm, sperm samples have significantly lower mean values than other tissue samples. Gene ontology analysis of these sperm promoters show significant enrichment for sperm-related biological processes as shown in **FIG. 15E.** The mean of the promoter variability values of the known sperm-related genes protamine 1 (PRM1), protamine 2 (PRM2), and protamine 3 (PRM3) which are genes expressed exclusively in sperm and replace the majority of histones to achieve extreme nuclear compaction in this specialized cell were assessed. As expected, sperm samples displayed significantly less variability in these promoters than other tissues, as shown in **FIG. 15C.** These same analyses were performed for the most stable promoters in neurons, as shown in **FIG. 15B** and **FIG. 15F,** and a known neuron-specific gene, CASP8 (shown in **FIG. 15D)** with similar results. **FIG. 16A-C** contains the results of these same analyses performed for several other tissue types. It is important to note that while the most stable promoters in a given tissue are generally characterized by very low promoter variability in samples from the given tissue, these promoters all have varying degrees of absolute methylation (hypo, mid, or hyper-methylation). It is also conceivable that this method can help overcome technical biases to methylation microarrays such as batch effects.

*Methylation variability can differentiate between healthy and diseased tissue*

In addition to distinguishing between tissue types, analysis of promoter methylation variability can enable the differentiation of diseased and healthy tissue samples of the same tissue type. For example, a method herein can be used to determine or identify a diseased tissue. A diseased tissue can comprise any tissue or cell described herein, for example a neuron, a skin tissue, or cancer of a cell or tissue. One notable example is the ability to distinguish between tumor and healthy tissue based on promoter variability signatures. **FIG. 18A** depicts the first two principal components of promoter variability values for colon primary tumor tissue and healthy colon tissue. The healthy colon tissue samples appear to be tightly clustered together, whereas the tumor samples are widely distributed throughout the plot. **FIG. 18B** shows the difference in promoter variability between psoriatic skin lesions and adjacent healthy skin samples from the same individuals. **FIG. 18C** shows a principal component analysis of neurons, glial cells, as well as bulk cell samples from postmortem brain tissue of individuals with Alzheimer's disease and controls. The plot shows clear separation among the neurons, glial cells, and bulk cell samples indicating a difference in promoter variability among different cell types in the same tissue. There is also separation between control and Alzheimer's disease samples in neuron and glial cell samples but such separation is not apparent among the bulk cell samples which may suggest subtle differences in promoter variability might be more apparent when samples are sorted for individual cell types.

As for other disease types, **FIG. 21** shows a principal component analysis of liver samples from healthy individuals and those with nonalcoholic fatty liver disease (NAFLD) and nonalcoholic steatohepatitis (NASH). **FIG. 22A-C** shows hierarchical clustering of diseased and control tissue samples. **FIG. 22A** shows clustering of normal and primary colon tumor samples. **FIG. 22B** shows clustering of psoriatic skin samples and normal skin samples. **FIG. 22C** shows clustering of control, nonalcoholic fatty liver disease (NAFLD), and nonalcoholic steatohepatitis (NASH) liver samples.

*Methylation variability of sperm can identify a subset of men with male factor infertility*

A n-of-1 analyses was performed on over 1500 sperm samples from fertile sperm donors as well as men being treated for male factor infertility. **FIG. 19A** shows there was a significantly higher number of dysregulated promoters in men being treated for male factor infertility compared to fertile sperm donors. **FIG. 20A-B** shows the number of dysregulated promoters in multiple cohorts of sperm samples, including the sperm donor cohort used to find the most stable promoters in sperm as well as the stability thresholds. To give a visual explanation of promoter methylation, **FIG. 19B** depicts the promoter variability values (dots) of the most stable sperm promoters and the corresponding stability thresholds for these promoters (black line) in an individual fertile sperm donor sample as well as a patient being treated for male factor infertility as shown in **FIG. 19C.** It is clear that the patient being treated for male factor infertility has many more dysregulated promoters than the fertile sperm donor. This suggests that male factor infertility may be more related to a global shift in methylation variability at promoters important for sperm cells rather than single nucleotide changes or epimutations.

The relationship between dysregulated promoters and pregnancy was assessed and live pregnancy rates in 1428 individuals being seen by a physician for infertility care. **Table 4** shows that in men undergoing intrauterine insemination (IUI), those with lowest number of dysregulated promoters (lowest 10th percentile of IUI patients) had significantly higher pregnancy and live birth rates than IUI patients with the highest number of dysregulated promoters (top 10th percentile of IUI patients). However, we saw no difference in pregnancy and birth rates when comparing men undergoing *in vitro* fertilization (IVF) (see **Table 5**) with the lowest and highest levels of dysregulated promoters, suggesting IVF may be a fertility treatment option for men with high levels of methylation dysregulation. **Table 4** shows the pregnancy and live birth rates from male patients undergoing on average 2.5 intrauterine insemination (IUI) cycles (N=553). **Table 5** shows the pregnancy and live birth rates from male patients undergoing IVF (N=251). For each patient's sperm sample, the number of dysregulated promoters were counted. Pregnancy and live birth rates were compared between the patient cohort with the top 10th percentile bottom 10th percentile of dysregulated promoters

**Table 4: Pregnancy and live birth rates from IUI in patients with the least and most dysregulated promoters**

| Patient Cohort | Pregnancy Rate from IUI | Live Birth Rate from IUI |
|---|---|---|
| Patients with the least number of dysregulated promoters (bottom 10th percentile) | 47.8% | 41.8% |
| Patients with the most number of dysregulated promoters (top 10th percentile) | 28.6% | 21.4% |
| | p=0.029 | p=0.016 |

**Table 5: Pregnancy and live birth rates from IVF in patients with the least and most dysregulated promoters**

| Patient Cohort | Pregnancy Rate from IVF | Live Birth Rate from IVF |
|---|---|---|
| Patients with the least number of dysregulated promoters (bottom 10th percentile) | 72.0% | 62.1% |
| Patients with the most number of dysregulated promoters (top 10th percentile) | 75.9% | 64.0% |
| | p=0.89 | p=0.75 |

**FIGS. 23A-C** show a further assessments of sperm promoter dysregulation and sperm concentration within the data set. **FIG. 23A** shows the variance levels of patients with the most dysregulated promoters vs patients with the least dysregulated promoters and the pregnancy rate and live birth rate from IUI. **FIG. 23B** shows the statistics from men with the most dysregulated promoters vs men with the least dysregulated promoters. The pregnancy rate from IUI and the average/median sperm concentration were significantly different between the two groups. **FIG. 23C** shows the statistics of pregnancy rate from IUI and live birth rate from IUI from men with the highest sperm concentration vs men with the lowest sperm concentration.

A method which assesses gene promoter DNA methylation variability to identify highly regulated genes in multiple tissue types is described herein and how these promoters can be impacted in various disease states. Hierarchical clustering of gene promoter variability from many tissues demonstrates how these patterns are in different tissues and how these patterns remain largely consistent in related, but distinct tissues. For example, numerous tissues from the gastrointestinal tract cluster together as do tissues important to the function of the immune system.

The most stable promoters in a given tissue have significantly lower methylation variability than the same promoters in other tissues highlighting the importance of genes and gene networks to any given tissue's function. It is believed this has never before been assessed and as such warrants further attention. Importantly, when assessing DNA methylation variability within the same tissue type, differences between healthy and diseased tissues such as in cancer, psoriasis, and Alzheimer's disease were determined.

To highlight the potential clinical impact of the assessment of promoter level DNA methylation variability, the pattern's utility in an assessment of male factor infertility was examined and it was found that men being seen by a physician for infertility had much higher levels of dysregulated promoters. In addition, men undergoing IUI treatments with the highest levels of dysregulated promoters had significantly lower pregnancy and live birth rates compared to men undergoing IUI treatments with the lowest levels of dysregulated promoters. However, this stark difference in pregnancy and live birth rates was not seen between men with the highest and lowest levels of dysregulated promoters in men undergoing IVF. This finding has great clinical significance because it suggests that if a man is struggling with infertility and has a high level of promoter dysregulation, he has much better odds at having a child after undergoing IVF than if he simply went through multiple rounds of IUI.

Taken together, these data suggest that promoter DNA methylation variability is an excellent indicator of tissue type. Without being bound by theory, this is likely due to the fact that the analysis of variability is able to successfully detect genes that are the most tightly regulated (via DNA methylation in this case) in any given tissue. The assumption is that these genes may play a role in cell function unique to each tissue. This is supported by the data that demonstrated that promoter DNA methylation variability is increased on average in abnormal tissues when compared to normal tissue. This was particularly apparent in our assessment of sperm DNA methylation variability patterns.

Of additional value in the assessment of this approach and it's translational capacity is the ability to perform these analyses in an n-of-1 context. Specifically, because intra-promoter variability within a single individual is assessed, one can reliably assess variability in a single individual with limited concerns of batch effects which can require normalization.

This was one of the largest analyses to date in terms of tissue types and sample numbers. However, many questions still remain to be answered. A deep analysis of sperm was completed. In some instances, similar work may need to be done in other tissue types.

The findings provide a means to define which genes each cell and tissue type tightly regulate to ensure their phenotype and function. Because these signals have potential utility in both the a basic understanding of tissue specific epigenetic patterns and in the clinical assessment of diseased tissues, as well as the prediction of outcomes, this example provides findings upon which tissue and disease specific assessments can be constructed in the future. The results here are encouraging and may offer another tool with which the health of tissues can be assessed. Further the results can help predict the outcomes from various clinical interventions, for example IVF vs IUI treatment.

**References**

1. Zhao SG, Chen WS, Li H, et al. The DNA methylation landscape of advanced prostate cancer. Nat Genet 2020;52(8):778-89.

2. Nishiyama A, Nakanishi M. Navigating the DNA methylation landscape of cancer. Trends Genet 2021;37(11):1012-27.

3. Davegardh C, García-Calzón S, Bacos K, Ling C. DNA methylation in the pathogenesis of type 2 diabetes in humans. Mol Metab 2018;14:12-25.

4. Ahmed SAH, Ansari SA, Mensah-Brown EPK, Emerald BS. The role of DNA methylation in the pathogenesis of type 2 diabetes mellitus. Clin Epigenetics 2020;12(1):1-23.

5. Horvath S. DNA methylation age of human tissues and cell types. Genome Biol 2013;14(10):1-20.

6. Jenkins TG, Aston KI, Cairns B, Smith A, Carrell DT. Paternal germ line aging: DNA methylation age prediction from human sperm. BMC Genomics 2018;19(1):1-10.

7. Benton MC, Johnstone A, Eccles D, et al. An analysis of DNA methylation in human adipose tissue reveals differential modification of obesity genes before and after gastric bypass and weight loss. Genome Biol 2015;16(1):1-21.

8. Godfrey KM, Sheppard A, Gluckman PD, et al. Epigenetic Gene Promoter Methylation at Birth Is Associated With Child's Later Adiposity. Diabetes 2011;60(5):1528-34.

9. Barrès R, Yan J, Egan B, et al. Acute Exercise Remodels Promoter Methylation in Human Skeletal Muscle. Cell Metab 2012;15(3):405-11.

10. Rönn T, Volkov P, Davegårdh C, et al. A Six Months Exercise Intervention Influences the Genome-wide DNA Methylation Pattern in Human Adipose Tissue. PLOS Genet 2013;9(6):e1003572.

11. Tobi EW, Lumey LH, Talens RP, et al. DNA methylation differences after exposure to prenatal famine are common and timing- and sex-specific. Hum Mol Genet 2009;18(21):4046-53.

12. Bartlett TE, Jones A, Goode EL, et al. Intra-Gene DNA Methylation Variability Is a Clinically Independent Prognostic Marker in Women's Cancers. PLOS ONE 2015;10(12):e0143178.

13. Goldman MJ, Craft B, Hastie M, et al. Visualizing and interpreting cancer genomics data via the Xena platform. Nat Biotechnol 2020;38(6):675-8.

14. Aston KI, Uren PJ, Jenkins TG, et al. Aberrant sperm DNA methylation predicts male fertility status and embryo quality. Fertil Steril 2015;104(6):1388-1397.e5.

15. Jenkins T, Aston K, Carrell D, et al. The impact of zinc and folic acid supplementation on sperm DNA methylation: results from the folic acid and zinc supplementation randomized clinical trial (FAZST). Fertil Steril [Internet] 2021 [cited 2021 Dec 1];Available from: https://doi.org/10.1016/j.fertnstert.2021.09.009

16. Aryee MJ, Jaffe AE, Corrada-Bravo H, et al. Minfi: a flexible and comprehensive Bioconductor package for the analysis of Infinium DNA methylation microarrays. Bioinformatics 2014;30(10):1363-9.

17. Du P, Zhang X, Huang C-C, et al. Comparison of Beta-value and M-value methods for quantifying methylation levels by microarray analysis. BMC Bioinformatics 2010;11(1):1-9.

18. Jenkins TG, Liu L, Aston KI, Carrell DT. Pre-screening method for somatic cell contamination in human sperm epigenetic studies. Syst Biol Reprod Med 2018;64(2):146-55.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

## ENUMERATED EMBODIMENTS

1. A method comprising:

a) obtaining a biological sample from a male subject, wherein the biological sample comprises seminal fluid;
b) extracting DNA from a sperm in the biological sample, extracting cell free DNA from the biological sample, or both;
c) detecting in an *in vitro* analytical assay, methylation present in at least 5 regions of an individual promoter comprised in the extracted DNA, the extracted cell free DNA, or both from the biological sample, wherein the promoter is selected from **Table 1;**
d) determining independently a standard deviation for methylation in each of the at least 5 regions of the individual promoter;
e) calculating an average standard deviation for methylation of the individual promoter from the standard deviation of methylation in each of the at least 5 regions of the individual promoter; and
f) determining if the average standard deviation for methylation of the individual promoter is greater than or equal to three standard deviations from a reference standard deviation of methylation for the individual promoter.

2. The method of embodiment 1, comprising determining that the average standard deviation of the individual promoter is greater than three standard deviations is independently determined in 22 or more different promoters.
3. The method of embodiment 1 or 2, which is a method of detecting diminished fertility of a male subject.
4. The method of embodiment 1, comprising determining that the average standard deviation of the individual promoter is greater than or equal to three standard deviations is independently determined in less than 22 different promoters, and wherein the average standard deviations for methylation of the individual promoters are determined in 1233 different promoters.
5. The method of embodiment 1 or 4, which is a method of detecting fertility of a male subject.
6. The method of any one of embodiments 1-5, wherein calculating the average standard deviation for methylation of the individual promoter from the standard deviation of methylation in each of the at least 5 regions of the individual promoter is calculated by:

$$\sigma = \sqrt{\frac{\sum |x_1 - \mu|^2}{N}}$$

,

wherein $\sigma$ = the average standard deviation for methylation, $x_1$ = an m-value of a given methylation array probe in the individual promoter, N = a number corresponding to the number of regions of the individual promoter, and $\mu$ = a mean of probe m-values in the individual promoter.

7. The method of any one of embodiments 1-5, wherein the reference standard deviation of methylation for the promoter is derived from a fertile subject.

8. The method of any one of embodiments 1-5, wherein the method further comprises determining:

    a) a morphological characteristic,
    b) a motility characteristic,
    c) a concentration, or
    d) any combination thereof of the sperm.

9. The method of any one of embodiments 1-8, wherein the detecting employs a computer processor.

10. The method of any one of embodiments 1-9, wherein the determining independently the standard deviation for methylation in each of the at least 5 regions of the individual promoter employs a computer processor.

11. The method of any one of embodiments 1-10, wherein the calculating the average standard deviation for methylation of the individual promoter from the standard deviation of methylation in each of the at least 5 regions of the individual promoter employs a computer processor.

12. The method of any one of embodiments 1-11, wherein the determining if the average standard deviation for methylation of the individual promoter is greater than or equal to three standard deviations from a reference standard deviation of methylation for the promoter employs a computer processor.

13. The method of any one of embodiments 1-12, wherein the method further comprises performing a treatment on the subject, wherein the treatment comprises *in vitro* fertilization (IVF) or intrauterine insemination (IUI).

14. The method of any one of embodiments 1-13, wherein the detecting comprises a sodium bisulfite conversion, a sequencing, a differential enzymatic cleavage of DNA, an affinity capture of methylated DNA, an array, or any combination thereof.

15. A method comprising:

    a) obtaining a biological sample from a male subject, wherein the biological sample comprises seminal fluid;
    b) extracting DNA from a sperm in the biological sample, extracting cell free DNA from the biological sample, or both;
    c) detecting in an *in vitro* analytical assay, methylation present in at least 5 regions of an individual promoter comprised in the extracted DNA, the extracted cell free DNA, or both from the biological sample, wherein the promoter is selected from Table 1;
    d) determining, with a computer program executed on a computer, a standard deviation for methylation in each of the at least 5 regions of the individual promoter;
    e) calculating, with the computer program executed on the computer, an average standard deviation for methylation of the individual promoter from the standard deviation of methylation in each of the at least 5 regions of the individual promoter; and
    f) determining, with the computer program executed on the computer, if the average standard deviation for methylation of the individual promoter is greater than or equal to three standard deviations from a reference standard deviation of methylation for the individual promoter.

16. A method comprising:

    a) obtaining a biological sample from a male subject, wherein the biological sample comprises seminal fluid;
    b) extracting DNA from a sperm in the biological sample, extracting cell free DNA from the biological sample, or both;
    c) detecting in an *in vitro* analytical assay, methylation present in at least 5 regions of an individual promoter comprised in the extracted DNA, the extracted cell free DNA, or both from the biological sample, wherein the promoter is selected from Table 1; and
    d) determining if an average standard deviation of the at least 5 regions of the individual promoter is greater than or equal to three standard deviations from a reference average standard deviation of the at least 5 regions of the individual promoter.

17. The method of embodiment 16, wherein the method comprises determining independently a standard deviation for methylation in each of the at least 5 regions of the individual promoter.

18. The method of any one of embodiments 16[250] or 17, wherein the method comprises calculating an average standard deviation for methylation of the individual promoter from the standard deviation of methylation in each of the at least 5 regions of the individual promoter.

19. A computer system for analyzing a DNA from a sperm cell, a cell free DNA from a seminal sample, or both obtained from a male subject, the computer system comprising:

a) a device for receiving sequenced data, wherein the sequenced data comprises methylation of at least 5 regions of an individual promoter comprised in the DNA from the sperm cell, the cell free DNA from the seminal sample, or both, and wherein the individual promoter is a promoter of Table 1;

b) a device for determining independently a standard deviation for methylation in each of the at least 5 regions of the individual promoter and calculating an average standard deviation from the standard deviation from methylation in each of the at least 5 regions of the individual promoter; and

c) a device for comparing the average standard deviation of the at least 5 regions of the individual promoter to a reference average standard deviation of at least 5 regions of the individual promoter and determining if the average standard deviation is greater than or equal to three standard deviations from the reference standard deviation of the individual promoter.

20. Use of an array used in detecting DNA methylation in at least 22 promoters selected from Table 1 from DNA obtained from a sperm cell, cell free DNA in a seminal sample, or both, wherein the DNA methylation is determined independently in at least 5 regions of an individual promoter for the manufacture of a diagnostic kit for determining male infertility of a human male subject.

21. The use of the array of embodiment 20, wherein the use further comprises:

a) determining independently a standard deviation for methylation in each of the at least 5 regions of the individual promoter;

b) calculating an average standard deviation for methylation of the individual promoter from the standard deviation of methylation in each of the at least 5 regions of the individual promoter; and

c) determining if the average standard deviation for methylation of the individual promoter is greater than or equal to three standard deviations from a reference standard deviation of methylation for the individual promoter.

**Claims**

1. A method comprising:

a) obtaining a biological sample from a male subject, wherein the biological sample comprises seminal fluid;

b) extracting DNA from a sperm in the biological sample, extracting cell free DNA from the biological sample, or both;

c) detecting in an *in vitro* analytical assay, methylation present in at least 5 regions of an individual promoter comprised in the extracted DNA, the extracted cell free DNA, or both from the biological sample; and

d) comparing a variability level for methylation of the at least 5 regions of the individual promoter to a reference variability threshold.

2. The method of claim 1, wherein the method comprises determining independently a variability level for methylation in each of the at least 5 regions of the individual promoter.

3. The method of any one of claims 1 or 2, wherein the method comprises determining a promoter dysregulation based on a change in the variability level for methylation of the individual promoter from the reference variability threshold .

4. The method of any one of claims 1 to 3, wherein the promoter is selected from **Table 1.**

5. The method of any one of claims 1 to 4, wherein the reference variability level is from a fertile control

6. The method of any one of claims 1 to 5, wherein the methylation of the promoter is determined for 22 or more different promoters.

7. The method of any one of claims 1-6, wherein the detecting employs a computer processor.

8. The method of any one of claims 1-7, wherein the comparing a variability level for methylation in each of the at least 5 regions of the individual promoter employs a computer processor.

9. The method of any one of claims 1-8, wherein the determining a promoter dysregulation based on a change in the variability level for methylation of the individual promoter from the reference variability threshold employs a computer processor.

10. The method of any one of claims 1-9, wherein the detecting comprises a sodium bisulfite conversion, a sequencing, a differential enzymatic cleavage of DNA, an affinity capture of methylated DNA, an array, or any combination thereof.

11. The method of any one of claims 1-10, wherein the method further comprises directing a subject to *in vitro* fertilization (IVF).

**FIG. 1**

IUI: Live birth rates between men with and without biomarker undergoing IUI

FIG. 2A

IVF: Live birth rates between men with and without biomarker undergoing IVF

FIG. 2B

**FIG. 3**

**FIG. 4**

Number of Dysregulated Promoters across the 1344 Samples

FIG. 5A

Male Age vs. Number of Dysregulated Promoters

N = 1343
R² = 0.000183

**FIG. 5B**

## Total Motile Count vs. Number of Dysregulated Promoters

N = 1322
R² = 0.001174

FIG. 5C

Male BMI vs. Number of Dysregulated Promoters

N = 1341
$R^2 = 4.1e^{-5}$

FIG. 6

Sperm Morphology vs. Number of Dysregulated Promoters

**FIG. 6 cont.**

**FIG. 6 cont.**

Sperm Concentration vs. Number of Dysregulated Promoters

## IUI: % Pregnancy and Live Birth, N=544

| Sperm methylation analysis | % Pregnancy | | % Live birth | |
|---|---|---|---|---|
| Excellent (N=44) | 50.0% | p=0.013* | 45.5% | p=0.003** |
| Average (N=449) | 46.1% | | 33.0% | |
| Poor (N=51) | 25.5% | | 17.6% | |

## FIG. 7A

## IUI, Removal of Female Factors: % Pregnancy and Live Birth, N=344

| Sperm methylation analysis | % Pregnancy | | % Live birth | |
|---|---|---|---|---|
| Excellent (N=29) | 51.7% | p=0.008** | 44.8% | p=0.034* |
| Average (N=284) | 46.8% | | 34.2% | |
| Poor (N=31) | 19.4% | | 19.4% | |

## FIG. 7B

## IVF: % Pregnancy and Live Birth, N=248

| Sperm methylation analysis | % Pregnancy | | % Live birth | |
|---|---|---|---|---|
| Excellent (N=22) | 72.7% | | 63.6% | |
| Average (N=199) | 79.9% | p=0.918 | 60.8% | p=0.761 |
| Poor (N=27) | 74.1% | | 59.3% | |

### FIG. 7C

## IVF, Removal of Female Factors: % Pregnancy and Live Birth, N=125

| Sperm methylation analysis | % Pregnancy | | % Live birth | |
|---|---|---|---|---|
| Excellent (N=13) | 69.2% | | 61.5% | |
| Average (N=104) | 89.4% | p=0.789 | 67.3% | p=0.967 |
| Poor (N=8) | 75.0% | | 62.5% | |

### FIG. 7D

# Permutations of birth rate differences of couples receiving IUI with men with poor and excellent sperm quality

## IUI and Total Motile Count: % Pregnancy and Live Birth, N=536

| Total Motile Count (M) | Sperm methylation analysis | % Pregnancy | % Live birth |
| --- | --- | --- | --- |
| ≥20 | Excellent (N=41) | 51.2% | 46.3% |
| <20 | Excellent (N=3) | Insufficient Data | Insufficient Data |
| ≥20 | Average (N=391) | 48.3% | 34.0% |
| <20 | Average (N=51) | 35.3% | 27.5% |
| ≥20 | Poor (N=40) | 27.5% | 17.5% |
| <20 | Poor (N=10) | 10.0% | 10.0% |

**FIG. 9**

Incidence of promoter dysregulation in men
with a Poor SpermQT result

FIG. 10

Incidence of promoter dysregulation in men
with a Poor SpermQT result and failed IUI

FIG. 11

Raw .idat files from Illumina
methylation microarrays

Preprocess raw data
with minfi (R package)

Remove samples without
distinct peaks from 0.0-0.2 and 0.8-1.0
and flat valleys in between

For sperm samples,
remove any samples where mean
of DLK1 beta value probes >= 0.20

Illumina methylation microarray
data sets with no publicly
available raw data

Calculate promoter variability
of each promoter with 5+ probes

Hierarchical clustering and
principal component analyses

Calculate promoter stability thresholds
for each promoter and sort promoters
based on thresholds

Use subset of most stable promoters
for gene ontology analyses

For n-of-1 analyses, count number of
dysregulated promoters in each sample

**FIG. 12**

Mean Promoter Values of 100 Most Stable Promoters
Promoters Found Using Beta Values

FIG. 13A

## Mean Promoter Values of 100 Most Stable Promoters
## Promoters Found Using M-values

FIG. 13B

$$\sigma = \sqrt{\frac{\sum |x_1 - \mu|^2}{N}}$$

**FIG. 14A**

$$\theta = \frac{\sum \sigma_1}{N} + 3\sqrt{\frac{\sum |\sigma_1 - \mu|^2}{N}}$$

**FIG. 14B**

$$\theta = 1.1\left(\frac{\sum \sigma_1}{N} + 3\sqrt{\frac{\sum |\sigma_1 - \mu|^2}{N}}\right)$$

**FIG. 14C**

FIG. 15A

FIG. 15B

FIG. 15C

FIG. 15D

## Sperm: Most Stable Promoters

| GO Term | Fold Enrichment | P-Value |
|---|---|---|
| Spermine Biosynthetic Process | 74.25 | 2.25E-04 |
| Serotonin Transport | 61.55 | 3.85E-04 |
| Quaternary Ammonium Group Transport | 41.03 | 6.26E-03 |

**FIG. 15E**

## Neuron: Most Stable Promoters

| GO Term | Fold Enrichment | P-Value |
|---|---|---|
| Cerebellar Molecular Layer Formation | 52.27 | 3.72E-02 |
| Cerebellar Molecular Layer Morphogenesis | 26.14 | 5.53E-02 |
| Cerebellar Molecular Layer Development | 26.14 | 5.53E-02 |

**FIG. 15F**

EP 4 782 553 A2

FIG. 16A

FIG. 16B

Most Stable Promoters in Liver

FIG. 16C

Promoters with Low Variability but Differing Methylation Values
Sperm Donor Samples (n=31)

FIG. 17A

FIG. 17B

FIG. 18A

Skin Principal Component Analysis

FIG. 18B

**FIG. 18C**

FIG. 19A

EP 4 782 553 A2

FIG. 19B

FIG. 19C

Most Stable Promoters in Sperm

**FIG. 20A**

FIG. 20B

FIG. 21

Category
Solid Tissue Normal
Primary Tumor

Category

FIG. 22A

**FIG. 22B**

**FIG. 22C**

Pregnancy / birth rates in men with normal sperm concentration
(>= 15 million sperm / mL)

| Variance Level | Pregnancy Rate from IUI | Live Birth Rate from IUI |
|---|---|---|
| Patients with least number of dysregulated promoters (bottom 10th percentile) | 47.1% | 39.2% |
| | p=0.082 | p=0.048* |
| Patients with most number of dysregulated promoters (top 10th percentile) | 29.5% | 20.5% |

**FIG. 23A**

# Statistics from men with low and high number of dysregulated promoters

| Variance Level | Pregnancy Rate from IUI | Live Birth Rate from IUI | Average / Median Age (Years) | Average / Median Sperm Conc (M/mL) | Average / Median BMI |
|---|---|---|---|---|---|
| Patients with least number of dysregulated promoters (bottom 10th percentile) | 47.8% | 41.8% | 33.7 / 33.0 | 108.8 / 87.0 | 29.3 / 28.2 |
| | p=0.029* | p=0.016* | p=0.72 | p=0.001** | p=0.88 |
| Patients with most number of dysregulated promoters (top 10th percentile) | 28.6% | 21.4% | 32.7 / 32.0 | 62.2 / 45.1 | 29.5 / 27.8 |

**FIG. 23B**

# Ranking by Sperm Concentration

| Concentration Level | Pregnancy Rate from IUI | Live Birth Rate from IUI |
|---|---|---|
| Patients with the highest sperm concentration (top 10th percentile) | 32.1% | 24.5% |
| | p=0.42 | p=0.68 |
| Patients with the lowest sperm concentration (bottom 10th percentile) | 25.0% | 21.2% |

**FIG. 23C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63252732
- US 63291536

**Non-patent literature cited in the description**

- **BRUTLAG et al.** *Comp. App. Biosci.*, 1990, vol. 6, 237-245
- **SCHLEGEL PN et al.** Diagnosis and Treatment of Infertility in Men: AUA/ASRM Guideline Part I. *J Urol.*, January 2021, vol. 205 (1), 36-43
- **BARRATT CL et al.** Diagnostic tools in male infertility-the question of sperm dysfunction. *Asian journal of andrology*, 2011, vol. 13 (1), 53-8
- **BONDE JP et al.** Relation between semen quality and fertility: a population-based study of 430 first-pregnancy planners. *Lancet*, 1998, vol. 352 (9135)
- **BENCHAIB M et al.** Quantitation by image analysis of global DNA methylation in human spermatozoa and its prognostic value in in vitro fertilization: a preliminary study. *Fertil Steril.*, 2003, vol. 80 (4), 947-53
- **HOUSHDARAN S. et al.** Widespread epigenetic abnormalities suggest a broad DNA methylation erasure defect in abnormal human sperm. *PloS one*, 2007, vol. 2 (12)
- **SCHISTERMAN EF et al.** A Randomized Trial to Evaluate the Effects of Folic Acid and Zinc Supplementation on Male Fertility and Live birth: Design and Baseline Characteristics. *Am J Epidemiol.*, 03 January 2020, vol. 189 (1), 8-26
- **ZHAO SG** ; **CHEN WS** ; **LI H et al.** The DNA methylation landscape of advanced prostate cancer. *Nat Genet*, 2020, vol. 52 (8), 778-89
- **NISHIYAMA A** ; **NAKANISHI M.** Navigating the DNA methylation landscape of cancer. *Trends Genet*, 2021, vol. 37 (11), 1012-27
- **DAVEGARDH C** ; **GARCÍA-CALZÓN S** ; **BACOS K** ; **LING C.** DNA methylation in the pathogenesis of type 2 diabetes in humans. *Mol Metab*, 2018, vol. 14, 12-25
- **AHMED SAH** ; **ANSARI SA** ; **MENSAH-BROWN EPK** ; **EMERALD BS**. The role of DNA methylation in the pathogenesis of type 2 diabetes mellitus. *Clin Epigenetics*, 2020, vol. 12 (1), 1-23
- **HORVATH S**. DNA methylation age of human tissues and cell types. *Genome Biol*, 2013, vol. 14 (10), 1-20
- **JENKINS TG** ; **ASTON KI** ; **CAIRNS B** ; **SMITH A** ; **CARRELL DT**. Paternal germ line aging: DNA methylation age prediction from human sperm. *BMC Genomics*, 2018, vol. 19 (1), 1-10
- **BENTON MC** ; **JOHNSTONE A** ; **ECCLES D et al.** An analysis of DNA methylation in human adipose tissue reveals differential modification of obesity genes before and after gastric bypass and weight loss. *Genome Biol*, 2015, vol. 16 (1), 1-21
- **GODFREY KM** ; **SHEPPARD A** ; **GLUCKMAN PD et al.** Epigenetic Gene Promoter Methylation at Birth Is Associated With Child's Later Adiposity. *Diabetes*, 2011, vol. 60 (5), 1528-34
- **BARRÈS R** ; **YAN J** ; **EGAN B et al.** Acute Exercise Remodels Promoter Methylation in Human Skeletal Muscle. *Cell Metab*, 2012, vol. 15 (3), 405-11
- **RÖNN T** ; **VOLKOV P** ; **DAVEGÅRDH C et al.** A Six Months Exercise Intervention Influences the Genome-wide DNA Methylation Pattern in Human Adipose Tissue. *PLOS Genet*, 2013, vol. 9 (6), e1003572
- **TOBI EW** ; **LUMEY LH** ; **TALENS RP et al.** DNA methylation differences after exposure to prenatal famine are common and timing- and sex-specific. *Hum Mol Genet*, 2009, vol. 18 (21), 4046-53
- **BARTLETT TE** ; **JONES A** ; **GOODE EL et al.** Intra-Gene DNA Methylation Variability Is a Clinically Independent Prognostic Marker in Women's Cancers. *PLOS ONE*, 2015, vol. 10 (12), e0143178
- **GOLDMAN MJ** ; **CRAFT B** ; **HASTIE M et al.** Visualizing and interpreting cancer genomics data via the Xena platform. *Nat Biotechnol*, 2020, vol. 38 (6), 675-8
- **ASTON KI** ; **UREN PJ** ; **JENKINS TG et al.** Aberrant sperm DNA methylation predicts male fertility status and embryo quality. *Fertil Steril*, 2015, vol. 104 (6), 1388-1397.e5

- **JENKINS T** ; **ASTON K** ; **CARRELL D et al.** The impact of zinc and folic acid supplementation on sperm DNA methylation: results from the folic acid and zinc supplementation randomized clinical trial (FAZST). *Fertil Steril [Internet*, 01 December 2021, https://doi.org/10.1016/j.fertnstert.2021.09.009
- **ARYEE MJ** ; **JAFFE AE** ; **CORRADA-BRAVO H et al.** Minfi: a flexible and comprehensive Bioconductor package for the analysis of Infinium DNA methylation microarrays. *Bioinformatics*, 2014, vol. 30 (10), 1363-9
- **DU P** ; **ZHANG X** ; **HUANG C-C et al.** Comparison of Beta-value and M-value methods for quantifying methylation levels by microarray analysis. *BMC Bioinformatics*, 2010, vol. 11 (1), 1-9
- **JENKINS TG** ; **LIU L** ; **ASTON KI** ; **CARRELL DT**. Pre-screening method for somatic cell contamination in human sperm epigenetic studies. *Syst Biol Reprod Med*, 2018, vol. 64 (2), 146-55